(19) Europäisches Patentamt
European Patent Office
Office européen des brevets

(11) **EP 1 344 771 A1**

(12) **EUROPÄISCHE PATENTANMELDUNG**

(43) Veröffentlichungstag:
**17.09.2003 Patentblatt 2003/38**

(21) Anmeldenummer: **03011479.7**

(22) Anmeldetag: **28.06.1996**

(51) Int Cl.[7]: **C07C 309/87**, C07C 311/29, C07C 311/65, C07C 331/32 // C07D249/12, C07D271/06, C07D271/10, C07D263/34, A01N47/38, A01N43/82, A01N43/74

(84) Benannte Vertragsstaaten:
**BE CH DE ES FR GB IT LI NL**

(30) Priorität: **11.07.1995 DE 19525162**

(62) Dokumentnummer(n) der früheren Anmeldung(en) nach Art. 76 EPÜ:
**96924805.3 / 0 842 157**

(71) Anmelder: **Bayer CropScience AG**
**40789 Monheim (DE)**

(72) Erfinder:
- **Müller, Klaus-Helmut, Dr.**
  **40593 Düsseldorf (DE)**
- **Kirsten, Rolf, Dr.**
  **40789 Monheim (DE)**
- **Gesing, Ernst Rudolf, Dr.**
  **40699 Erkrath (DE)**
- **Kluth, Joachim, Dr.**
  **40764 Langenfeld (DE)**
- **Drewes, Mark Wilhelm, Dr.**
  **40764 Langenfeld (DE)**
- **Findeisen, Kurt, Prof. Dr.**
  **51375 Leverkursen (DE)**
- **Jansen, Johannes Rudolf, Dr.**
  **40789 Monheim (DE)**
- **König, Klaus, Dr.**
  **51519 Odenthal (DE)**
- **Riebel, Hans-Jochem, Dr.**
  **56242 Selters (DE)**
- **Schallner, Otto, Dr.**
  **40789 Monheim (DE)**
- **Dollinger, Markus, Dr.**
  **51381 Leverkursen (DE)**
- **Santel, Hans-Joachim, Dr.**
  **51371 Leverkusen (DE)**

Bemerkungen:
Diese Anmeldung ist am 21 - 05 - 2003 als Teilanmeldung zu der unter INID-Kode 62 erwähnten Anmeldung eingereicht worden.

(54) **2-Alkoxy-phenylsulfonsäure-derivate als Zwischenprodukte für herbizide Sulfonylamino(thio)carbonylverbindungen**

(57) Die Erfindung betrifft Zwischenprodukte der allgemeinen Formel (XIII)

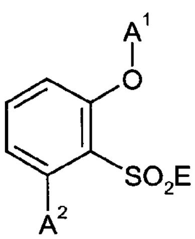

(XIII)

worin
E, $A^1$ und $A^2$ die in den Ansprüchen angegebene Bedeutung haben. Erfindungsgemäß können diese Zwischenprodukte zur Herstellung von neuen herbizid wirksamen Sulfonylamino(thio)carbonylverbindungen verwendet werden.

EP 1 344 771 A1

## Beschreibung

Die Erfindung betrifft neue Sulfonylamino(thio)carbonylverbindungen, mehrere Verfahren und neue Zwischenprodukte zu ihrer Herstellung sowie ihre Verwendung als Herbizide.

Es ist bereits bekannt, daß bestimmte Sulfonylaminocarbonylverbindungen, wie z.B. die Verbindungen 4,5-Dimethoxy-2-(2-methoxy-phenylsulfonylaminocarbonyl)-2,4-dihydro-3H-1,2,4-triazol-3-on, 4,5-Dimethoxy-2-(2,5-dimethoxy-phenylsulfonylaminocarbonyl)-2,4-dihydro-3H-1,2,4-triazol-3-on, 4,5-Diethoxy-2-(2,5-dimethoxy-phenylsulfonylaminocarbonyl)-2,4-dihydro-3H-1,2,4-triazol-3-on und N-(2,5-Dimethoxy-phenylsulfonyl)-1,5-dimethyl-1H-pyrazol-3-carboxamid, herbizide Eigenschaften aufweisen (vgl. EP 341489, EP 422469, EP 425948, EP 431291, EP 507171, EP 534266, DE 4029753). Die Wirkung dieser Verbindungen ist jedoch nicht in allen Belangen zufriedenstellend.

Es wurden nun die neuen Sulfonylamino(thio)carbonylverbindungen der allgemeinen Formel (I),

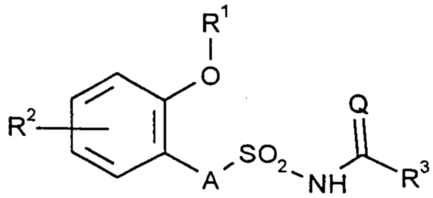

(I)

in welcher

A für eine Einfachbindung, für Sauerstoff, Schwefel oder die Gruppierung N-R steht, worin R für Wasserstoff, Alkyl, Alkenyl, Alkinyl oder Cycloalkyl steht,

Q für Sauerstoff oder Schwefel steht,

$R^1$ für Wasserstoff, Formyl oder für jeweils gegebenenfalls substituiertes Alkyl, Alkenyl, Alkinyl, Alkylcarbonyl, Alkoxycarbonyl, Alkylsulfonyl, Cycloalkyl, Cycloalkylcarbonyl oder Cycloalkylsulfonyl steht,

$R^2$ für Cyano, Halogen oder für jeweils gegebenenfalls substituiertes Alkyl, Alkenyl, Alkinyl, Alkoxy, Alkenyloxy oder Alkinyloxy steht und

$R^3$ für jeweils gegebenenfalls substituiertes Heterocyclyl mit 5 Ringgliedern steht, von denen mindestens eines Sauerstoff, Schwefel oder Stickstoff ist und ein bis drei weitere Ringglieder Stickstoff sein können,

sowie Salze von Verbindungen der Formel (I) gefunden,
wobei die vorbekannten Verbindungen 4,5-Dimethoxy-2-(2,5-dimethoxy-phenylsulfonylaminocarbonyl)-2,4-dihydro-3H-1,2,4-triazol-3-on, 4,5-Diethoxy-2-(2,5-dimethoxy-phenylsulfonylaminocarbonyl)-2,4-dihydro-3H-1,2,4-triazol-3-on und N-(2,5-Dimethoxy-phenylsulfonyl)-1,5-dimethyl-1H-pyrazol-3-carboxamid durch Disclaimer ausgenommen sind.

Man erhält die neuen Sulfonylamino(thio)carbonylverbindungen der allgemeinen Formel (I), wenn man

(a) Aminosulfonylverbindungen der allgemeinen Formel (II)

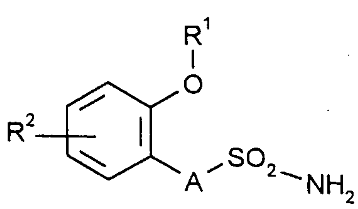

(II)

in welcher
A, $R^1$ und $R^2$ die oben angegebenen Bedeutungen haben,
mit (Thio)Carbonsäurederivaten der allgemeinen Formel (III)

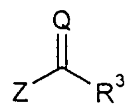

(III)

in welcher
Q und $R^3$ die oben angegebenen Bedeutungen haben und

Z für Halogen, Alkoxy, Aryloxy oder Arylalkoxy steht,

gegebenenfalls in Gegenwart eines Säureakzeptors und gegebenenfalls in Gegenwart eines Verdünnungsmittels umsetzt,
oder wenn man

(b) Sulfonyliso(thio)cyanate der allgemeinen Formel (IV)

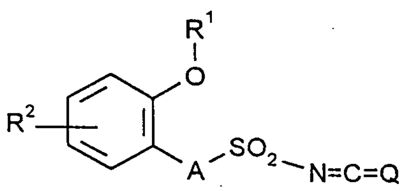

(IV)

in welcher
A, Q, $R^1$ und $R^2$ die oben angegebenen Bedeutungen haben,
mit Heterocyclen der allgemeinen Formel (V)

$$H\text{-}R^3 \quad (V)$$

in welcher

$R^3$ die oben angegebene Bedeutung hat,

gegebenenfalls in Gegenwart eines Reaktionshilfsmittels und gegebenenfalls in Gegenwart eines Verdünnungsmittels umsetzt,
oder wenn man

(c) Chlorsulfonylverbindungen der allgemeinen Formel (VI)

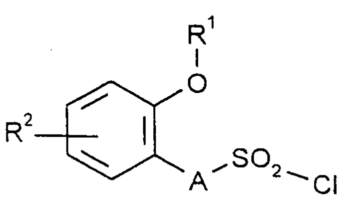

(VI)

in welcher
A, $R^1$ und $R^2$ die oben angegebenen Bedeutungen haben,
mit Heterocyclen der allgemeinen Formel (V)

$$H\text{-}R^3 \qquad (V)$$

in welcher

$R^3$ die oben angegebene Bedeutung hat,

und Metall(thio)cyanaten der allgemeinen Formel (VII)

$$MQCN \qquad (VII)$$

in welcher

Q die oben angegebene Bedeutung hat und
M für ein Alkali- oder Erdalkalimetall-Äquivalent steht,

gegebenenfalls in Gegenwart eines Reaktionshilfsmittels und gegebenenfalls in Gegenwart eines Verdünnungsmittels umsetzt,
oder wenn man

(d) Chlorsulfonylverbindungen der allgemeinen Formel (VI)

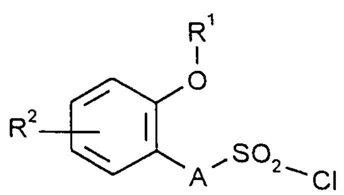
(VI)

in welcher
A, $R^1$ und $R^2$ die oben angegebenen Bedeutungen haben,
mit (Thio)Carbonsäureamiden der allgemeinen Formel (VIII)

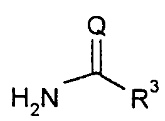
(VIII)

in welcher
Q und $R^3$ die oben angegebenen Bedeutungen haben,
gegebenenfalls in Gegenwart eines Säureakzeptors und gegebenenfalls in Gegenwart eines Verdünnungsmittels umsetzt,
oder wenn man

(e) Sulfonylamino(thio)carbonylverbindungen der allgemeinen Formel (IX)

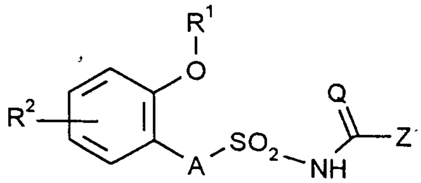

(IX)

in welcher
A, Q, $R^1$ und $R^2$ die oben angegebenen Bedeutungen haben und

Z für Halogen, Alkoxy, Aryloxy oder Arylalkoxy steht,

mit Heterocyclen der allgemeinen Formel (V)

$$H\text{-}R^3 \quad (V)$$

in welcher

$R^3$ die oben angegebene Bedeutung hat,

gegebenenfalls in Gegenwart eines Säureakzeptors und gegebenenfalls in Gegenwart eines Verdünnungsmittels umsetzt,
oder wenn man

(f) Heterocyclen der allgemeinen Formel (V)

$$H\text{-}R^3 \quad (V)$$

in welcher

$R^3$ die oben angegebene Bedeutung hat,

mit Chlorsulfonyliso(thio)cyanat gegebenenfalls in Gegenwart eines Verdünnungsmittels umsetzt und die hierbei gebildeten Addukte in situ mit Benzolderivaten der allgemeinen Formel (X)

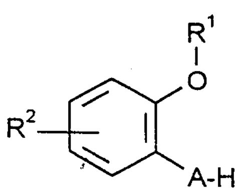

(X)

in welcher
A, $R^1$ und $R^2$ die oben angegebenen Bedeutungen haben,
gegebenenfalls in Gegenwart eines Säureakzeptors und gegebenenfalls in Gegenwart eines Verdünnungsmittels umsetzt,
und gegebenenfalls die nach den Verfahren (a), (b), (c), (d), (e) oder (f) erhaltenen Verbindungen der Formel (I) nach üblichen Methoden in Salze überführt.

Die neuen Sulfonylamino(thio)carbonylverbindungen der allgemeinen Formel (I) zeichnen sich durch starke herbizide Wirksamkeit aus.
Gegenstand der Erfindung sind vorzugsweise Verbindungen der Formel (I), in welcher

A für eine Einfachbindung, für Sauerstoff, Schwefel oder die Gruppierung N-R steht, worin R für Wasserstoff, $C_1$-$C_6$-Alkyl, $C_2$-$C_6$-Alkenyl, $C_2$-$C_6$-Alkinyl oder $C_3$-$C_6$-Cycloalkyl steht,

Q für Sauerstoff oder Schwefel steht,

$R^1$ für Wasserstoff, Formyl, für jeweils gegebenenfalls durch Cyano, Fluor, Chlor, Brom, Phenyl oder $C_1$-$C_4$-Alkoxy substituiertes Alkyl, Alkenyl, Alkinyl, Alkylcarbonyl, Alkoxycarbonyl oder Alkylsulfonyl mit jeweils bis zu 6 Kohlenstoffatomen, oder für jeweils gegebenenfalls durch Cyano, Fluor, Chlor, Brom oder $C_1$-$C_4$-Alkyl substituiertes $C_3$-$C_6$-Cycloalkyl, $C_3$-$C_6$-Cycloalkyl-carbonyl oder $C_3$-$C_6$-Cycloalkyl-sulfonyl steht,

$R^2$ für Cyano, Fluor, Chlor, Brom oder für jeweils gegebenenfalls durch Cyano, Fluor, Chlor, Brom oder $C_1$-$C_4$-Alkoxy substituiertes Alkyl, Alkenyl, Alkinyl, Alkoxy, Alkenyloxy oder Alkinyloxy mit jeweils bis zu 6 Kohlenstoffatomen steht und

$R^3$ für jeweils gegebenenfalls substituiertes Heterocyclyl der nachstehenden Formeln steht,

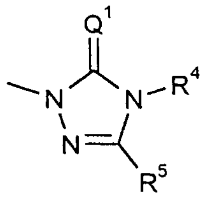

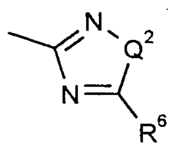

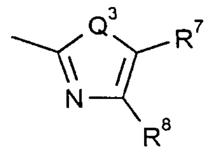

worin

$Q^1$, $Q^2$ und $Q^3$ jeweils für Sauerstoff oder Schwefel stehen sowie

$R^4$ für Wasserstoff, Hydroxy, Amino, Cyano, für $C_2$-$C_{10}$-Alkylidenamino, für gegebenenfalls durch Fluor, Chlor, Brom, Cyano, $C_1$-$C_4$-Alkoxy, $C_1$-$C_4$-Alkyl-carbonyl oder $C_1$-$C_4$-Alkoxy-carbonyl substituiertes $C_1$-$C_6$-Alkyl, für jeweils gegebenenfalls durch Fluor, Chlor und/oder Brom substituiertes $C_2$-$C_6$-Alkenyl oder $C_2$-$C_6$-Alkinyl, für jeweils gegebenenfalls durch Fluor, Chlor, Brom, Cyano, $C_1$-$C_4$-Alkoxy oder $C_1$-$C_4$-Alkoxy-carbonyl substituiertes $C_1$-$C_6$-Alkoxy, $C_1$-$C_6$-Alkylamino oder $C_1$-$C_6$-Alkyl-carbonylamino, für $C_3$-$C_6$-Alkenyloxy, für Di-($C_1$-$C_4$-alkyl)-amino, für jeweils gegebenenfalls durch Fluor, Chlor, Brom, Cyano und/oder $C_1$-$C_4$-Alkyl substituiertes $C_3$-$C_6$-Cycloalkyl, $C_3$-$C_6$-Cycloalkylamino oder $C_3$-$C_6$-Cycloalkyl-$C_1$-$C_4$alkyl, oder für jeweils gegebenenfalls durch Fluor, Chlor, Brom, Cyano, Nitro, $C_1$-$C_4$-Alkyl, Trifluormethyl und/oder $C_1$-$C_4$-Alkoxy substituiertes Phenyl, Phenylamino oder Phenyl-$C_1$-$C_4$-alkyl steht,

$R^5$ für Wasserstoff, Hydroxy, Mercapto, Amino, Cyano, Fluor, Chlor, Brom, Iod, für gegebenenfalls durch Fluor, Chlor, Brom, Cyano, $C_1$-$C_4$-Alkoxy, $C_1$-$C_4$-Alkyl-carbonyl oder $C_1$-$C_4$-Alkoxy-carbonyl substituiertes $C_1$-$C_6$-Alkyl, für jeweils gegebenenfalls durch Fluor, Chlor und/oder Brom substituiertes $C_2$-$C_6$-Alkenyl oder $C_2$-$C_6$-Alkinyl, für jeweils gegebenenfalls durch Fluor, Chlor, Cyano, $C_1$-$C_4$-Alkoxy oder $C_1$-$C_4$-Alkoxy-carbonyl substituiertes $C_1$-$C_6$-Alkoxy, $C_1$-$C_6$-Alkylthio, $C_1$-$C_6$-Alkylamino oder $C_1$-$C_6$-Alkyl-carbonylamino, für $C_3$-$C_6$-Alkenyloxy, $C_3$-$C_6$-Alkinyloxy, $C_3$-$C_6$-Alkenylthio, $C_3$-$C_6$-Alkinylthio, $C_3$-$C_6$-Alkenylamino oder $C_3$-$C_6$-Alkinylamino, für Di-($C_1$-$C_4$-alkyl)-amino, für jeweils gegebenenfalls durch Methyl und/oder Ethyl substituiertes Aziridino, Pyrrolidino, Piperidino oder Morpholino, für jeweils gegebenenfalls durch Fluor, Chlor, Brom, Cyano und/oder $C_1$-$C_4$-Alkyl substituiertes $C_3$-$C_6$-Cycloalkyl, $C_5$-$C_6$-Cycloalkenyl, $C_3$-$C_6$-Cycloalkyloxy, $C_3$-$C_6$-Cycloalkylthio, $C_3$-$C_6$-Cycloalkylamino, $C_3$-$C_6$-Cycloalkyl-$C_1$-$C_4$-alkyl, $C_3$-$C_6$-Cycloalkyl-$C_1$-$C_4$-alkoxy, $C_3$-$C_6$-Cycloalkyl-$C_1$-$C_4$-alkylthio oder $C_3$-$C_6$-Cycloalkyl-$C_1$-$C_4$-alkylamino, oder für jeweils gegebenenfalls durch Fluor, Chlor, Brom, Cyano, Nitro, $C_1$-$C_4$-Alkyl, Trifluormethyl, $C_1$-$C_4$-Alkoxy und/oder $C_1$-$C_4$-Alkoxy-carbonyl substituiertes Phenyl, Phenyl-$C_1$-$C_4$-alkyl, Phenoxy, Phenyl-$C_1$-$C_4$-alkoxy, Phenylthio, Phenyl-$C_1$-$C_4$-alkylthio, Phenylamino oder Phenyl-$C_1$-$C_4$-alkylamino steht, oder

$R^4$ und $R^5$ zusammen für gegebenenfalls verzweigtes Alkandiyl mit 3 bis 11 Kohlenstoffatomen stehen,

ferner

$R^6$, $R^7$ und $R^8$ gleich oder verschieden sind und für Wasserstoff, Cyano, Fluor, Chlor, Brom, oder für jeweils gegebenenfalls durch Fluor, Chlor, Brom oder $C_1$-$C_4$-Alkoxy substituiertes Alkyl, Alkenyl, Alkinyl, Alkoxy, Alkenyloxy, Alkinyloxy, Alkylthio, Alkenylthio, Alkinylthio, Alkylsulfinyl oder Alkylsulfonyl mit jeweils bis zu 6 Kohlenstoffatomen oder für gegebenenfalls durch Cyano, Fluor, Chlor, Brom oder $C_1$-$C_4$-Alkyl substituiertes Cycloalkyl mit 3 bis 6 Kohlenstoffatomen stehen,

wobei die vorbekannten Verbindungen 4,5-Dimethoxy-2-(2,5-dimethoxy-phenylsulfonylaminocarbonyl)-2,4-dihydro-3H-1,2,4-triazol-3-on, 4,5-Diethoxy-2-(2,5-dimethoxy-phenylsulfonylaminocarbonyl)-2,4-dihydro-3H-1,2,4-triazol-3-on und N-(2,5-Dimethoxy-phenylsulfonyl)-1,5-dimethyl-1H-pyrazol-3-carboxamid durch Disclaimer ausgenommen sind.

Gegenstand der Erfindung sind weiter vorzugsweise Natrium-, Kalium-, Magnesium-, Calcium-, Ammonium-, $C_1$-$C_4$-Alkyl-ammonium-, Di-($C_1$-$C_4$-alkyl)-ammonium-, Tri-($C_1$-$C_4$-alkyl)-ammonium-, Tetra-($C_1$-$C_4$-alkyl)-ammonium-, Tri-($C_1$-$C_4$-alkyl)-sulfonium-, $C_5$- oder $C_6$-Cycloalkyl-ammonium- und Di-($C_1$-$C_2$alkyl)-benzyl-ammonium-Salze von Verbindungen der Formel (I), in welcher A, Q, $R^1$, $R^2$ und $R^3$ die oben vorzugsweise angegebenen Bedeutungen haben.

Gegenstand der Erfindung sind insbesondere Verbindungen der Formel (I), in welcher

A für eine Einfachbindung, für Sauerstoff oder für die Gruppierung N-R steht, worin R für Wasserstoff, Methyl, Ethyl, n- oder i-Propyl, n-, i- oder s-Butyl, Propenyl, Butenyl, Propinyl, Butinyl, Cyclopropyl, Cyclobutyl, Cyclopentyl oder Cyclohexyl steht,

Q für Sauerstoff oder Schwefel steht,

$R^1$ für Wasserstoff, Formyl, für jeweils gegebenenfalls durch Fluor, Chlor, Brom, Methoxy oder Ethoxy substituiertes Methyl, Ethyl, n- oder i-Propyl, n-, i- oder s-Butyl, Propenyl, Butenyl, Propinyl, Butinyl, Acetyl, Propionyl, Butyroyl, Methoxycarbonyl, Ethoxycarbonyl, n- oder i-Propoxycarbonyl, Methylsulfonyl, Ethylsulfonyl, n- oder i-Propylsulfonyl, n-, i-, s- oder t-Butylsulfonyl, oder für jeweils gegebenenfalls durch Fluor, Chlor oder Methyl substituiertes Cyclopropyl, Cyclopropylcarbonyl oder Cyclopropylsulfonyl steht,

$R^2$ für Cyano, Fluor, Chlor, Brom, oder für jeweils gegebenenfalls durch Fluor, Chlor, Methoxy oder Ethoxy substituiertes Methyl, Ethyl, n- oder i-Propyl, n-, i- oder s-Butyl, Propenyl, Butenyl, Propinyl, Butinyl, Methoxy, Ethoxy, n- oder i-Propoxy, n-, i- oder s-Butoxy, Propenyloxy, Butenyloxy, Propinyloxy oder Butinyloxy steht und

$R^3$ für jeweils gegebenenfalls substituiertes Heterocyclyl der nachstehenden Formeln steht,

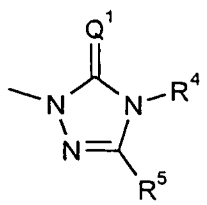

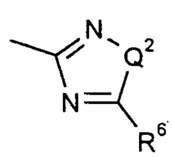

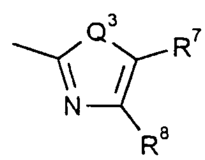

worin

$Q^1$, $Q^2$ und $Q^3$ jeweils für Sauerstoff oder Schwefel stehen sowie

$R^4$ für Wasserstoff, Hydroxy, Amino, für $C_3$-$C_8$-Alkylidenamino, für jeweils gegebenenfalls durch Fluor, Chlor, Cyano, Methoxy oder Ethoxy substituiertes Methyl, Ethyl, n- oder i-Propyl, n-, i-, s- oder t-Butyl, für jeweils gegebenenfalls durch Fluor, Chlor oder Brom substituiertes Propenyl, Butenyl, Propinyl oder Butinyl, für jeweils gegebenenfalls durch Fluor, Chlor, Cyano, Methoxy oder Ethoxy substituiertes Methoxy, Ethoxy, n-oder i-Propoxy, n-, i-, s- oder t-Butoxy, Methylamino, Ethylamino, n- oder i-Propylamino, n-, i-, s- oder t-Butylamino, für Propenyloxy oder Butenyloxy, für Dimethylamino oder Diethylamino, für jeweils gegebenenfalls durch Fluor,

Chlor, Methyl und/oder Ethyl substituiertes Cyclopropyl, Cyclobutyl, Cyclopentyl, Cyclohexyl, Cyclopropylamino, Cyclobutylamino, Cyclopentylamino, Cyclohexylamino, Cyclopropylmethyl, Cyclobutylmethyl, Cyclopentylmethyl oder Cyclohexylmethyl, oder für jeweils gegebenenfalls durch Fluor, Chlor, Methyl, Trifluormethyl und/oder Methoxy substituiertes Phenyl oder Benzyl steht,

$R^5$ für Wasserstoff, Hydroxy, Mercapto, Amino, Fluor, Chlor, Brom, für jeweils gegebenenfalls durch Fluor, Chlor, Cyano, Methoxy oder Ethoxy substituiertes Methyl, Ethyl, n- oder i-Propyl, n-, i-, s- oder t-Butyl, für jeweils gegebenenfalls durch Fluor, Chlor oder Brom substituiertes Ethenyl, Propenyl, Butenyl, Propinyl oder Butinyl, für jeweils gegebenenfalls durch Fluor, Chlor, Cyano, Methoxy oder Ethoxy substituiertes Methoxy, Ethoxy, n- oder i-Propoxy, n-, i-, s- oder t-Butoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, für Propenyloxy, Butenyloxy, Propinyloxy, Butinyloxy, Propenylthio, Propadienylthio, Butenylthio, Propinylthio, Butinylthio, Propenylamino, Butenylamino, Propinylamino oder Butinylamino, für Dimethylamino, Diethylamino oder Dipropylamino, für jeweils gegebenenfalls durch Fluor, Chlor, Methyl und/oder Ethyl substituiertes Cyclopropyl, Cyclobutyl, Cyclopentyl, Cyclohexyl, Cyclopentenyl, Cyclohexenyl, Cyclopropyloxy, Cyclobutyloxy, Cyclopentyloxy, Cyclohexyloxy, Cyclopropylthio, Cyclobutylthio, Cyclopentylthio, Cyclohexylthio, Cyclopropylamino, Cyclobutylamino, Cyclopentylamino, Cyclohexylamino, Cyclopropylmethyl, Cyclobutylmethyl, Cyclopentylmethyl, Cyclohexylmethyl, Cyclopropylmethoxy, Cyclobutylmethoxy, Cyclopentylmethoxy, Cyclohexylmethoxy, Cyclopropylmethylthio, Cyclobutylmethylthio, Cyclopentylmethylthio, Cyclohexylmethylthio, Cyclopropylmethylamino, Cyclobutylmethylamino, Cyclopentylmethylamino oder Cyclohexylmethylamino, oder für jeweils gegebenenfalls durch Fluor, Chlor, Methyl, Trifluormethyl, Methoxy und/oder Methoxycarbonyl substituiertes Phenyl, Benzyl, Phenoxy, Benzyloxy, Phenylthio, Benzylthio, Phenylamino oder Benzylamino steht, oder

$R^4$ und $R^5$ zusammen für gegebenenfalls verzweigtes Alkandiyl mit 3 bis 11 Kohlenstoffatomen stehen, ferner

$R^6$, $R^7$ und $R^8$ gleich oder verschieden sind und für Wasserstoff, Cyano, Fluor, Chlor, Brom, oder für jeweils gegebenenfalls durch Fluor, Chlor, Methoxy oder Ethoxy substituiertes Methyl, Ethyl, n- oder i-Propyl, n-, i-, s- oder t-Butyl, Propenyl, Butenyl, Propinyl, Butinyl, Methoxy, Ethoxy, n- oder i-Propoxy, n-, i-, s- oder t-Butoxy, Propenyloxy, Butenyloxy, Propinyloxy, Butinyloxy, Methylthio, Ethylthio, n- oder i-Propylthio, n-, i-, s- oder t-Butylthio, Propenylthio, Butenylthio, Propinylthio, Butinylthio, Methylsulfinyl, Ethylsulfinyl, Methylsulfonyl oder Ethylsulfonyl, oder für Cyclopropyl stehen,

wobei die vorbekannten Verbindungen 4,5-Dimethoxy-2-(2,5-dimethoxy-phenylsulfonylaminocarbonyl)-2,4-dihydro-3H-1,2,4-triazol-3-on, 4,5-Diethoxy-2-(2,5-dimethoxy-phenylsulfonylaminocarbonyl)-2,4-dihydro-3H-1,2,4-triazol-3-on und N-(2,5-Dimethoxy-phenylsulfonyl)-1,5-dimethyl-1H-pyrazol-3-carboxamid durch Disclaimer ausgenommen sind.

Eine ganz besonders bevorzugte Gruppe erfindungsgemäßer Verbindungen sind die Verbindungen der Formel (I), in welcher

A für eine Einfachbindung steht,

Q für Sauerstoff oder Schwefel steht,

$R^1$ für Methyl, Ethyl, n- oder i-Propyl steht,

$R^2$ für Chlor oder Methyl - jeweils in 5- oder 6-Position - steht und

$R^3$ für gegebenenfalls substituiertes Triazolinyl der nachstehenden Formel steht,

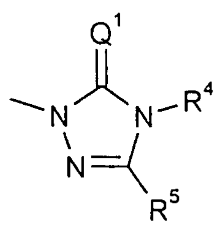

worin

- $Q^1$ für Sauerstoff oder Schwefel steht sowie
- $R^4$ für jeweils gegebenenfalls durch Fluor, Chlor, Cyano, Methoxy oder Ethoxy substituiertes Methyl, Ethyl, n- oder i-Propyl, für Propenyl oder Propinyl, für Methoxy, Ethoxy, n- oder i-Propoxy, oder für Cyclopropyl steht und
- $R^5$ für Wasserstoff, Chlor, Brom, für jeweils gegebenenfalls durch Fluor, Chlor, Cyano, Methoxy oder Ethoxy substituiertes Methyl, Ethyl, n- oder i-Propyl, für jeweils gegebenenfalls durch Fluor und/oder Chlor substituiertes Propenyl oder Propinyl, für jeweils gegebenenfalls durch Fluor, Chlor, Cyano, Methoxy oder Ethoxy substituiertes Methoxy, Ethoxy, n- oder i-Propoxy, Methylthio, Ethylthio, n- oder i-Propylthio, für Propenyloxy oder Cyclopropyl, steht.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangsstoffe bzw. Zwischenprodukte. Diese Restedefinitionen können untereinander, also auch zwischen den angegebenen bevorzugten Bereichen beliebig kombiniert werden.

Verwendet man beispielsweise 2-Fluor-6-methoxy-benzolsulfonamid und 5-Ethoxy-4-methyl-2-phenoxycarbonyl-2,4-dihydro-3H-1,2,4-triazol-3-thion als Ausgangsstoffe, so kann der Reaktionsablauf beim erfindungsgemäßen Verfahren (a) durch das folgende Formelschema skizziert werden:

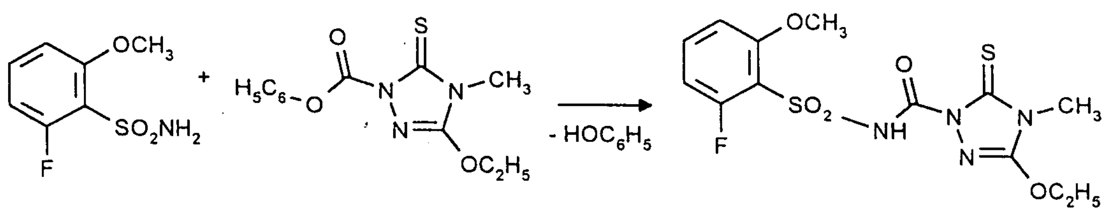


Verwendet man beispielsweise 2-Ethoxy-6-methyl-phenylsulfonyl-isothiocyanat und 5-Ethyl-4-methoxy-2,4-dihydro-3H-1,2,4-triazol-3-on als Ausgangsstoffe, so kann der Reaktionsablauf beim erfindungsgemäßen Verfahren (b) durch das folgende Formelschema skizziert werden:

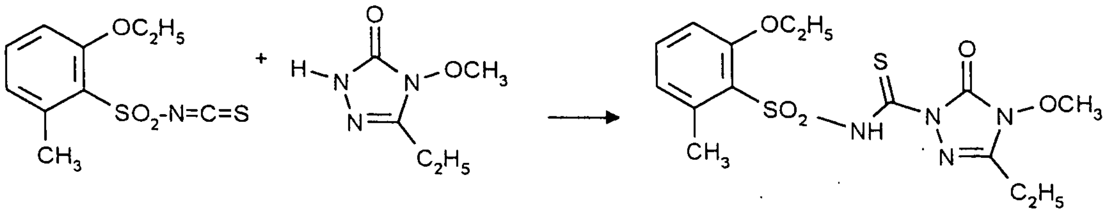

Verwendet man beispielsweise 2-Methoxy-3-methyl-benzolsulfochlorid, 5-Ethylthio-4-methoxy-2,4-dihydro-3H-1,2,4-triazol-3-on und Kaliumcyanat als Ausgangsstoffe, so kann der Reaktionsablauf beim erfindungsgemäßen Verfahren (c) durch das folgende Formelschema skizziert werden:

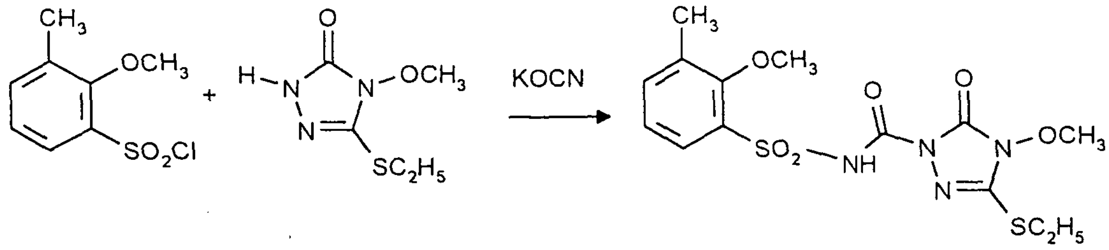


Verwendet man beispielsweise 2-Ethoxy-4-fluor-benzolsulfochlorid und 5-Methyl-1,2,4-oxadiazol-3-carboxamid als Ausgangsstoffe, so kann der Reaktionsablauf beim erfindungsgemäßen Verfahren (d) durch das folgende Formelschema skizziert werden:

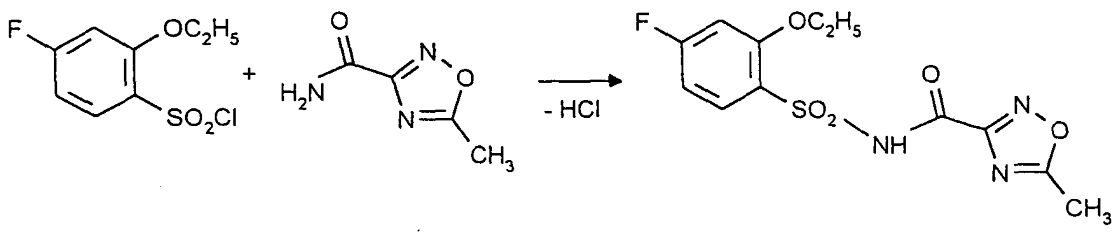


Verwendet man beispielsweise N-(2-Chlor-6-propoxy-phenylsulfonyl)-O-methylurethan und 4-Methyl-5-methylthio-2,4-dihydro-3H-1,2,4-triazol-3-on als Ausgangsstoffe, so kann der Reaktionsablauf beim erfindungsgemäßen Verfahren (e) durch das folgende Formelschema skizziert werden:

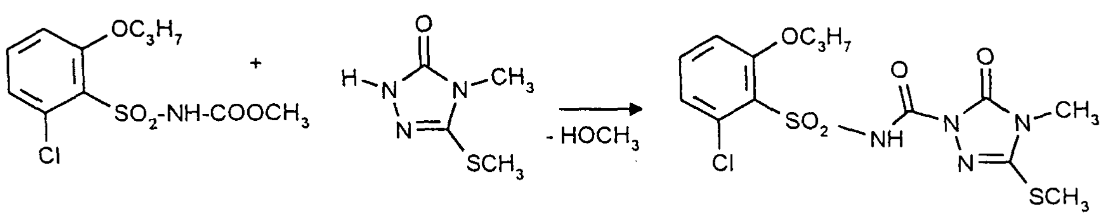


Verwendet man beispielsweise 5-Chlor-4-ethyl-2,4-dihydro-3H-1,2,4-triazol-3-on und Chlorsulfonylisocyanat sowie anschließend 2-Ethoxy-6-methyl-anilin als Ausgangsstoffe, so kann der Reaktionsablauf beim erfindungsgemäßen Verfahren (f) durch das folgende Formelschema skizziert werden:

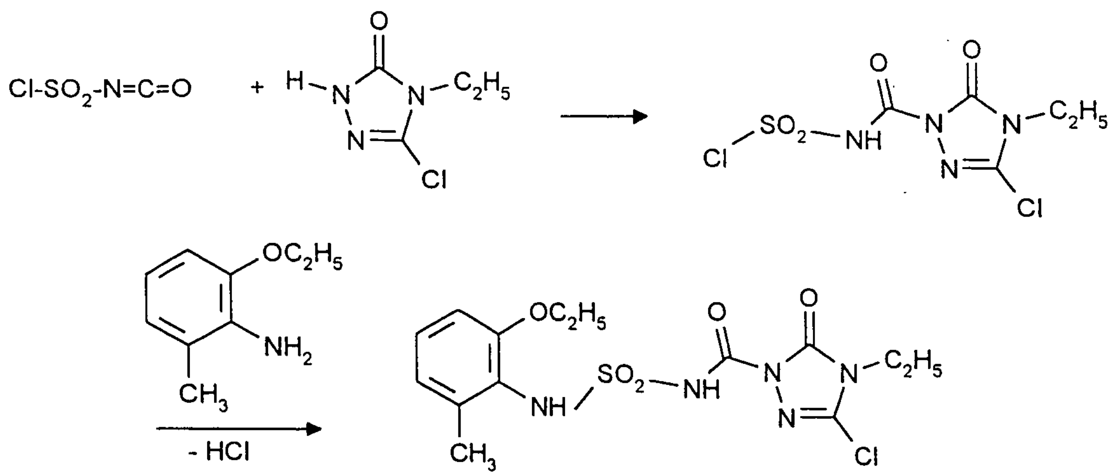


Die beim erfindungsgemäßen Verfahren (a) zur Herstellung der Verbindungen der Formel (I) als Ausgangsstoffe zu verwendenden Aminosulfonylverbindungen sind durch die Formel (II) allgemein definiert. In der Formel (II) haben A, $R^1$ und $R^2$ vorzugsweise bzw. insbesondere diejenige Bedeutung, die bereits oben bei der Beschreibung der erfindungsgemäß herzustellenden Verbindungen der Formel (I) vorzugsweise bzw. als insbesondere bevorzugt für A, $R^1$ und $R^2$ angegeben wurde.

Die Ausgangsstoffe der Formel (II) sind bekannt und/oder können nach an sich bekannten Verfahren hergestellt werden (vgl. EP 216504, DE 3208189, EP 44807, EP 23422).

Noch nicht aus der Literatur bekannt und als neue Stoffe ebenfalls Gegenstand der vorliegenden Anmeldung sind die Sulfonamide der allgemeinen Formel (IIa),

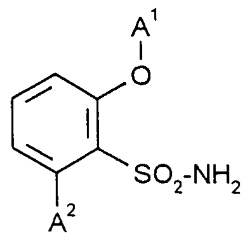

(IIa)

in welcher

$A^1$ für Ethyl, n- oder i-Propyl, n-, i-, s- oder t-Butyl, Trifluormethyl, Fluorethyl, Chlorethyl, Difluorethyl, Trifluorethyl, Chlortrifluorethyl, Methoxyethyl, Ethoxyethyl, Allyl, Propargyl oder Benzyl steht und

$A^2$ für Methyl, Ethyl, n- oder i-Propyl, n-, i-, s- oder t-Butyl steht.

Man erhält die neuen Sulfonamide der Formel (IIa), wenn man Sulfonsäurechloride der Formel (VIa)

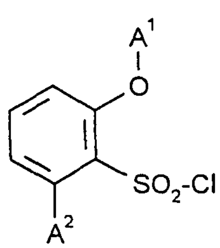

(VIa)

in welcher

$A^1$ und $A^2$ die oben angegebenen Bedeutungen haben,

mit Ammoniak gegebenenfalls in Gegenwart eines Verdünnungsmittels, wie z.B. Wasser, bei Temperaturen zwischen 0°C und 50°C umsetzt (vgl. die Herstellungsbeispiele).

Die Ausgangsstoffe der Formel (II) können im allgemeinen auch durch Umsetzung von Phenolderivaten der Formel (IIb)

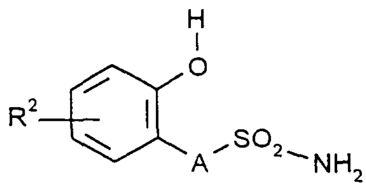

(IIb)

in welcher
A und $R^2$ die oben angegebenen Bedeutungen haben,
mit Alkylierungsmitteln der Formel (XI)

$$X\text{-}R^1 \qquad (XI)$$

in welcher

$R^1$ die oben angegebene Bedeutung hat und

X für Halogen oder die Gruppierung $R^1$-O-$SO_2$-O- steht,

gegebenenfalls in Gegenwart eines Säureakzeptors, wie z.B. Kaliumcarbonat, und gegebenenfalls in Gegenwart eines Verdünnungsmittels, wie z.B. Toluol, bei Temperaturen zwischen 10°C und 150°C erhalten werden (vgl. die Herstellungsbeispiele).

Die als Vorprodukte benötigten Phenolderivate der Formel (IIb) sind bekannt und/oder können nach an sich bekannten Verfahren hergestellt werden (vgl. EP 44807, Metalloberfläche - Angew. Elektrochemie 27 (1973), 217-227 - zitiert in Chem. Abstracts 79:86733; Herstellungsbeispiele).

Die weiter als Vorprodukte benötigten Alkylierungsmittel der Formel (XI) sind bekannte Synthesechemikalien.

Die beim erfindungsgemäßen Verfahren (a) zur Herstellung der Verbindungen der Formel (I) weiter als Ausgangsstoffe zu verwendenden (Thio)Carbonsäurederivate sind durch die Formel (III) allgemein definiert. In der Formel (III) haben Q und $R^3$ vorzugsweise bzw. insbesondere diejenige Bedeutung, die bereits oben bei der Beschreibung der erfindungsgemäß herzustellenden Verbindungen der Formel (I) vorzugsweise bzw. als insbesondere bevorzugt für Q und $R^3$ angegeben wurde; Z steht vorzugsweise für Fluor, Chlor, Brom, $C_1$-$C_4$-Alkoxy, Phenoxy oder Benzyloxy, insbesondere für Chlor, Methoxy, Ethoxy oder Phenoxy.

Die Ausgangsstoffe der Formel (III) sind bekannt und/oder können nach an sich bekannten Verfahren hergestellt werden (vgl. EP 459244, EP 341489, EP 422469, EP 425948, EP 431291, EP 507171, EP 534266).

Die beim erfindungsgemäßen Verfahren (b) zur Herstellung der Verbindungen der Formel (I) als Ausgangsstoffe zu verwendenden Sulfonyliso(thio)cyanate sind durch die Formel (IV) allgemein definiert. In der Formel (IV) haben A, Q, $R^1$ und $R^2$ vorzugsweise bzw. insbesondere diejenige Bedeutung, die bereits oben bei der Beschreibung der erfindungsgemäß herzustellenden Verbindungen der Formel (I) vorzugsweise bzw. als insbesondere bevorzugt für A, Q, $R^1$ und $R^2$ angegeben wurde.

Die Ausgangsstoffe der Formel (IV) sind bekannt und/oder können nach an sich bekannten Verfahren hergestellt werden (vgl. EP 23422, EP 216504).

Noch nicht aus der Literatur bekannt und als neue Stoffe auch Gegenstand der vorliegenden Anmeldung sind die Sulfonyliso(thio)cyanate der allgemeinen Formel (IVa)

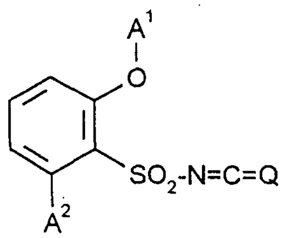

(IVa)

in welcher

Q für Sauerstoff oder Schwefel steht,

$A^1$ für Ethyl, n- oder i-Propyl, n-, i-, s- oder t-Butyl, Trifluormethyl, Fluorethyl, Chlorethyl, Difluorethyl, Trifluorethyl, Chlortrifluorethyl, Methoxyethyl, Ethoxyethyl, Allyl, Propargyl oder Benzyl steht und

$A^2$ für Methyl, Ethyl, n- oder i-Propyl, n-, i-, s- oder t-Butyl steht.

Man erhält die neuen Sulfonyliso(thio)cyanate der Formel (IVa), wenn man Sulfonamide der Formel (IIa) - oben - mit Phosgen bzw. Thiophosgen, gegebenenfalls in Gegenwart eines Alkylisocyanats, wie z.B. Butylisocyanat, gegebenenfalls in Gegenwart eines Reaktionshilfsmittels, wie z.B. Diazabicyclo[2.2.2]octan, und in Gegenwart eines Verdünnungsmittels, wie z.B. Toluol, Xylol oder Chlorbenzol, bei Temperaturen zwischen 80°C und 150°C umsetzt und nach Ende der Umsetzung die flüchtigen Komponenten unter vermindertem Druck abdestilliert.

Die bei den erfindungsgemäßen Verfahren (b), (c), (e) und (f) zur Herstellung der Verbindungen der Formel (I) weiter als Ausgangsstoffe zu verwendenden Heterocyclen sind durch die Formel (V) allgemein definiert. In der Formel (V) hat $R^3$ vorzugsweise bzw. insbesondere diejenige Bedeutung, die bereits oben bei der Beschreibung der erfindungsgemäß herzustellenden Verbindungen der Formel (I) vorzugsweise bzw. als insbesondere bevorzugt für $R^3$ angegeben wurde.

Die Ausgangsstoffe der Formel (V) sind bekannt und/oder können nach an sich bekannten Verfahren hergestellt werden (vgl. EP 341489, EP 422469, EP 425948, EP 431291, EP 507171, EP 534266).

Die bei den erfindungsgemäßen Verfahren (c) und (d) zur Herstellung der Verbindungen der Formel (I) als Ausgangsstoffe zu verwendenden Chlorsulfonylverbindungen sind durch die Formel (VI) allgemein definiert. In der Formel (VI) haben A, $R^1$ und $R^2$ vorzugsweise bzw. insbesondere diejenige Bedeutung, die bereits oben bei der Beschreibung der erfindungsgemäß herzustellenden Verbindungen der Formel (I) vorzugsweise bzw. als insbesondere bevorzugt für A, $R^1$ und $R^2$ angegeben wurde.

Die Ausgangsstoffe der Formel (VI) sind bekannt und/oder können nach an sich bekannten Verfahren hergestellt werden (vgl. EP 511826, DE 3208189, EP23422).

Noch nicht aus der Literatur bekannt und als neue Stoffe gleichfalls Gegenstand der vorliegenden Anmeldung sind die Sulfonsäurechloride der Formel (VIa)

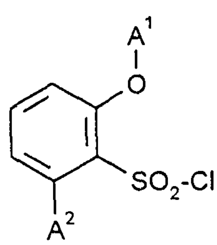

(VIa)

in welcher

$A^1$ für Ethyl, n- oder i-Propyl, n-, i-, s- oder t-Butyl, Trifluormethyl, Fluorethyl, Chlorethyl, Difluorethyl, Trifluorethyl, Chlortrifluorethyl, Methoxyethyl, Ethoxyethyl, Allyl, Propargyl oder Benzyl steht und

$A^2$ für Methyl, Ethyl, n- oder i-Propyl, n-, i-, s- oder t-Butyl steht.

Man erhält die neuen Sulfonsäurechloride der Formel (VIa), wenn man Anilinderivate der Formel (XII)

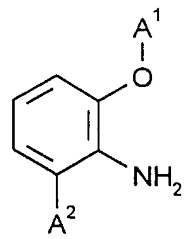

(XII)

in welcher
$A^1$ und $A^2$ die oben angegebenen Bedeutungen haben,
mit einem Alkalimetallnitrit, wie z.B. Natriumnitrit, in Gegenwart von Salzsäure bei Temperaturen zwischen -10°C und +10°C umsetzt und die so erhaltene Diazoniumsalzlösung mit Schwefeldioxid in Gegenwart eines Verdünnungsmittels, wie z.B. Dichlormethan oder 1,2-Dichlor-ethan, und in Gegenwart eines Katalysators, wie z.B. Kupfer(I)-chlorid, gegebenenfalls in Gegenwart eines weiteren Katalysators, wie z.B. Dodecyltrimethylammoniumbromid, bei Temperaturen zwischen -10°C und +50°C umsetzt (vgl. die Herstellungsbeispiele).

Die als Vorprodukte benötigten Anilinderivate der Formel (XII) sind bekannt und/oder können nach an sich bekannten Verfahren hergestellt werden (vgl. EP 511826, US 4992091, EP 185128, DE 2405479, Herstellungsbeispiele).

Die vorgenannten neuen Benzolsulfonsäurederivate der Formeln (IIa), (IVa) und (VIa) können zusammenfassend durch die folgende Formel (XIII) definiert werden:

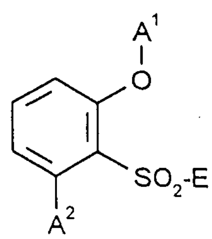

(XIII)

in welcher

E für $-NH_2$, -N=C=Q oder -Cl steht, wobei

Q für O oder S steht, und ferner

$A^1$ für Ethyl, n- oder i-Propyl, n-, i-, s- oder t-Butyl, Trifluormethyl, Fluorethyl, Chlorethyl, Difluorethyl, Trifluorethyl, Chlortrifluorethyl, Methoxyethyl, Ethoxyethyl, Allyl, Propargyl oder Benzyl steht und

$A^2$ für Methyl, Ethyl, n- oder i-Propyl, n-, i-, s- oder t-Butyl steht.

Die beim erfindungsgemäßen Verfahren (d) zur Herstellung der Verbindungen der Formel (I) als Ausgangsstoffe zu verwendenden (Thio)Carbonsäureamide sind durch die Formel (VIII) allgemein definiert. In der Formel (VIII) haben Q und $R^3$ vorzugsweise bzw. insbesondere diejenige Bedeutung, die bereits oben bei der Beschreibung der erfindungsgemäß herzustellenden Verbindungen der Formel (I) vorzugsweise bzw. als insbesondere bevorzugt für Q und $R^3$ angegeben wurde.

Die Ausgangsstoffe der Formel (VIII) sind bekannt und/oder können nach an sich bekannten Verfahren hergestellt werden (vgl. EP 459244).

Die beim erfindungsgemäßen Verfahren (e) zur Herstellung der Verbindungen der Formel (I) als Ausgangsstoffe zu verwendenden Sulfonylamino(thio)carbonylverbindungen sind durch die Formel (IX) allgemein definiert. In der Formel (IX) haben A, Q, $R^1$ und $R^2$ vorzugsweise bzw. insbesondere diejenige Bedeutung, die bereits oben bei der Beschreibung der erfindungsgemäß herzustellenden Verbindungen der Formel (I) vorzugsweise bzw. als insbesondere bevorzugt für A, Q, $R^1$ und $R^2$ angegeben wurde; Z steht vorzugsweise für Fluor, Chlor, Brom, $C_1$-$C_4$-Alkoxy,

Phenoxy oder Benzyloxy, insbesondere für Chlor, Methoxy, Ethoxy oder Phenoxy.

Die beim erfindungsgemäßen Verfahren (f) zur Herstellung der Verbindungen der Formel (I) als Ausgangsstoffe zu verwendenden Benzolderivate sind durch die Formel (X) allgemein definiert. In der Formel (X) haben A, $R^1$ und $R^2$ vorzugsweise bzw. insbesondere diejenige Bedeutung, die bereits oben bei der Beschreibung der erfindungsgemäß herzustellenden Verbindungen der Formel (I) vorzugsweise bzw. als insbesondere bevorzugt für A, $R^1$ und $R^2$ angegeben wurde.

Die Ausgangsstoffe der Formel (X) sind bekannt und/oder können nach an sich bekannten Verfahren hergestellt werden (vgl. EP 511826, US 4992091, EP 185128, DE 2405479, Herstellungsbeispiele).

Die erfindungsgemäßen Verfahren (a), (b), (c), (d), (e) und (f) zur Herstellung der neuen Verbindungen der Formel (I) werden vorzugsweise unter Verwendung von Verdünnungsmitteln durchgeführt. Als Verdünnungsmittel kommen dabei praktisch alle inerten organischen Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlormethan, Chlorbenzol und o-Dichlorbenzol; Ether wie Diethyl- und Dibutylether, Glykoldimethylether und Diglykoldimethylether, Tetrahydrofuran und Dioxan; Ketone wie Aceton, Methyl-ethyl-, Methyl-isopropyl- und Methyl-isobutyl-keton; Ester wie Essigssäuremethylester und -ethylester; Nitrile wie z.B. Acetonitril und Propionitril; Amide wie z.B. Dimethylformamid, Dimethylacetamid und N-Methylpyrrolidon, sowie Dimethylsulfoxid, Tetramethylensulfon und Hexamethylphosphorsäuretriamid.

Als Reaktionshilfsmittel bzw. als Säureakzeptoren können bei den erfindungsgemäßen Verfahren (a), (b), (c), (d), (e) und (f) alle üblicherweise für derartige Umsetzungen verwendbaren Säurebindemittel eingesetzt werden. Vorzugsweise in Frage kommen Alkalimetallhydroxide wie z.B. Natrium- und Kaliumhydroxid, Erdalkalihydroxide wie z.B. Calciumhydroxid, Alkalicarbonate und -alkoholate wie Natrium- und Kaliumcarbonat, Natrium- und Kalium-tert-butylat, ferner basische Stickstoffverbindungen, wie Trimethylamin, Triethylamin, Tripropylamin, Tributylamin, Diisobutylamin, Dicyclohexylamin, Ethyldiisopropylamin, Ethyldicyclohexylamin, N,N-Dimethylbenzylamin, N,N-Dimethyl-anilin, Pyridin, 2-Methyl-, 3-Methyl-, 4-Methyl-, 2,4-Dimethyl-, 2,6-Dimethyl-, 2-Ethyl-, 4-Ethyl- und 5-Ethyl-2-methyl-pyridin, 1,5-Diazabicyclo[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei den erfindungsgemäßen Verfahren (a), (b), (c), (d), (e) und (f) in einem größeren Bereich variiert werden. Im allgemeinen arbeitet man bei Temperaturen zwischen -20°C und +150°C, vorzugsweise bei Temperaturen zwischen 0°C und +100°C.

Die erfindungsgemäßen Verfahren (a), (b), (c), (d), (e) und (f) werden im allgemeinen unter Normaldruck durchgeführt. Es ist jedoch auch möglich, unter erhöhtem oder vermindertem Druck zu arbeiten.

Zur Durchführung der erfindungsgemäßen Verfahren (a), (b), (c), (d), (e) und (f) werden die jeweils benötigten Ausgangsstoffe im allgemeinen in angenähert äquimolaren Mengen eingesetzt. Es ist jedoch auch möglich, eine der jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei den erfindungsgemäßen Verfahren (a), (b), (c), (d), (e) und (f) jeweils nach üblichen Methoden (vgl. die Herstellungsbeispiele).

Aus den erfindungsgemäßen Verbindungen der allgemeinen Formel (I) können gegebenenfalls Salze hergestellt werden. Man erhält solche Salze in einfacher Weise nach üblichen Salzbildungsmethoden, beispielsweise durch Lösen oder Dispergieren einer Verbindung der Formel (I) in einem geeigneten Lösungsmittel, wie z.B. Methylenchlorid, Aceton, tert-Butyl-methylether oder Toluol, und Zugabe einer geeigneten Base. Die Salze können dann - gegebenenfalls nach längerem Rühren - durch Einengen oder Absaugen isoliert werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria,

Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfruchtund Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich vorzugsweise zur Bekämpfung von monokotylen und dikotylen Unkräutern sowohl im Vorauflaufals auch im Nachauflauf-Verfahren. Sie zeigen starke herbizide Aktivität und ein breites Wirkungsspektrum bei Anwendung auf den Boden und auf oberirdische Pflanzenteile.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolin-säure, Dicamba und Picloram; Aryloxyalkansäuren, wie z.B. 2,4-D, 2,4-DB, 2,4-DP, Fluroxypyr, MCPA, MCPP und Triclopyr; Aryloxy-phenoxyalkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z. B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

<u>**Herstellungsbeispiele:**</u>

<u>Beispiel 1</u>

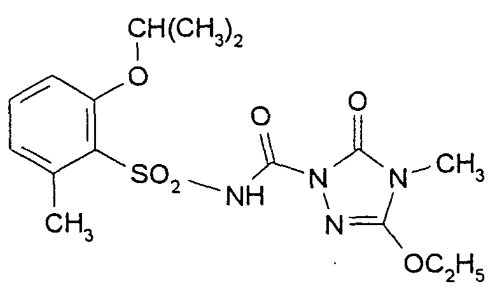

(Verfahren (a))

Eine Mischung aus 2,5 g (10 mMol) 5-Ethoxy-4-methyl-2-phenoxycarbonyl-2,4-dihydro-3H-1,2,4-triazol-3-on, 2,3 g (10 mMol) 2-Isopropoxy-6-methyl-benzolsulfonamid, 1,5 g (10 mMol) Diazabicyclo[5.4.0]undec-7-en (DBU) und 50 ml Acetonitril wird 5 Stunden bei 20°C gerührt. Dann wird im Wasserstrahlvakuum eingeengt und der Rückstand mit 50 ml IN-Salzsäure verrührt, abgesaugt, mit Diethylether verrührt und erneut abgesaugt.

Man erhält 2,4 g (60% der Theorie) 5-Ethoxy-4-methyl-2-(2-isopropoxy-6-methylphenylsulfonyl-aminocarbonyl)-2,4-dihydro-3H-1,2,4-triazol-3-on vom Schmelzpunkt 155°C.

Beispiel 2

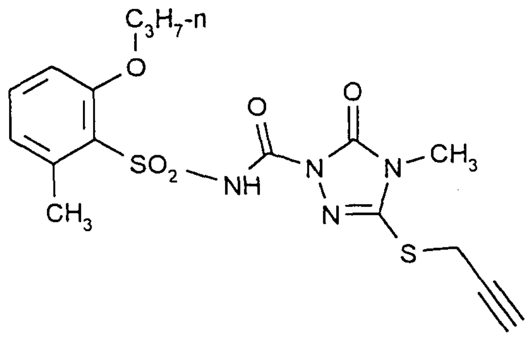

(Verfahren (c))

Eine Mischung aus 1,7 g (10 mMol) 4-Methyl-5-propargylthio-2,4-dihydro-3H-1,2,4-triazol-3-on, 1,3 g (20 mMol) Natriumcyanat, 2,5 g (10 mMol) 2-Methyl-6-n-propoxy-benzolsulfochlorid und 50 ml Acetonitril wird 3 Stunden unter Rückfluß erhitzt. Dann wird im Wasserstrahlvakuum eingeengt, der Rückstand mit 1N-Salzsäure verrührt und dreimal mit je 50 ml Methylenchlorid extrahiert. Die vereinigten organischen Extraktionslösungen werden eingeengt, der Rückstand mit Isopropanol digeriert und das kristallin angefallene Produkt durch Absaugen isoliert.
Man erhält 2,2 g (52% der Theorie) 4-Methyl-5-propargylthio-2-(2-methyl-6-npropoxy-phenylsulfonyl-aminocarbonyl)-2,4-dihydro-3H-1,2,4-triazol-3-on vom Schmelzpunkt 151°C.

Beispiel 3

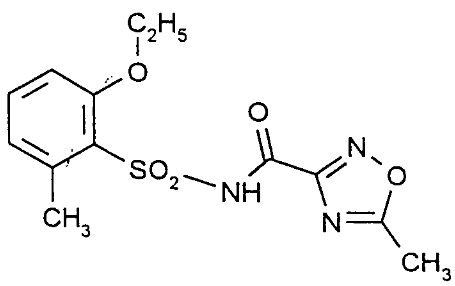

(Verfahren (d))

Eine Mischung aus 3,2 g (25 mMol) 5-Methyl-1,2,4-oxadiazol-3-carboxamid, 4,2 g (75 mMol) Kaliumhydroxid (Pulver) und 200 ml Dioxan wird 30 Minuten bei 60°C gerührt. Dann wird im Wasserstrahlvakuum auf etwa das halbe Volumen eingeengt und bei ca. 20°C wird eine Lösung von 7 g (30 mMol) 2-Ethoxy-6-methyl-benzolsulfochlorid in 10 ml Dioxan tropfenweise dazu gegeben. Die Reaktionsmischung wird dann noch ca. 15 Stunden bei 20°C gerührt. Anschließend wird im Wasserstrahlvakuum eingeengt, der Rückstand mit 50 ml IN-Salzsäure verrührt und das kristallin angefallene Produkt durch Absaugen isoliert.
Man erhält 4,0 g (49% der Theorie) N-(2-Ethoxy-6-methyl-phenylsulfonyl)-5-methyl-1,2,4-oxadiazol-3-carboxamid vom Schmelzpunkt 168°C.

Beispiel 4

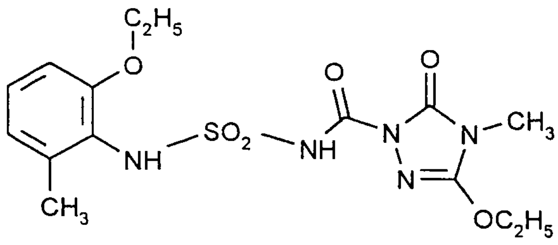

(Verfahren (f))

1,7 g (12 mMol) Chlorsulfonylisocyanat werden zu einer auf 5°C abgekühlten Lösung von 1,4 g (10 mMol) 5-Ethoxy-4-methyl-2,4-dihydro-3H-1,2,4-triazol-3-on in 50 ml Methylenchlorid hinzugegeben und dann wird eine Lösung von 1,5 g (10 mMol) 2-Ethoxy-6-methyl-anilin und 1,0 g (10 mMol) Triethylamin in 10 ml Methylenchlorid ebenfalls bei 5°C zugetropft. Die Reaktionsmischung wird dann 15 Stunden bei ca. 20°C gerührt. Anschließend werden 100 ml 1N-Salzsäure dazugegeben. Nach gutem Durchrühren wird die organische Phase abgetrennt, mit Natriumsulfat getrocknet und filtriert. Das Filtrat wird im Wasserstrahlvakuum eingeengt, der Rückstand mit Isopropanol digeriert und das kristallin angefallene Produkt durch Absaugen isoliert.

Man erhält 1,8 g (45% der Theorie) 5-Ethoxy-4-methyl-2-(2-ethoxy-6-methylphenyl-aminosulfonyl-aminocarbonyl)-2,4-dihydro-3H-1,2,4-triazol-3-onvom Schmelzpunkt 147°C.

Analog Beispiel 1 bis 4 sowie entsprechend der allgemeinen Beschreibung der erfindungsgemäßen Herstellungsverfahren können beispielsweise auch die in der nachstehenden Tabelle 1 aufgeführten Verbindungen der Formel (I) hergestellt werden.

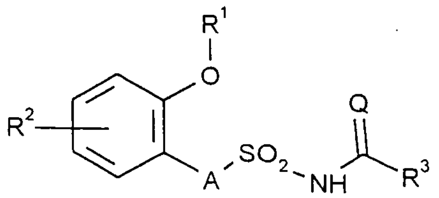

(I)

Tabelle 1: Beispiele für die Verbindungen der Formel (I)

| Bsp.-Nr. | A | Q | $R^1$ | (Position-) $R^2$ | $R^3$ | Schmelzpunkt (°C) |
|---|---|---|---|---|---|---|
| 5 | - | O | n-$C_3H_7$ | (6-) $CH_3$ | 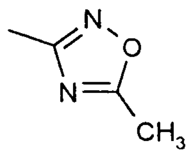 | 117 |
| 6 | - | O | $C_2H_5$ | (6-) Cl | 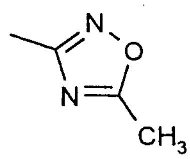 | 156 |
| 7 | - | O | $C_2H_5$ | (6-) $CH_3$ | 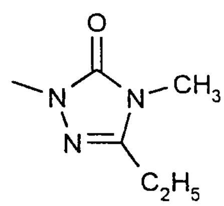 | 110 |
| 8 | - | O | $C_2H_5$ | (6-) $CH_3$ | 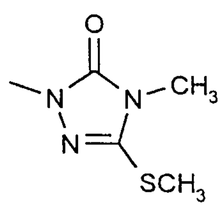 | 141 |
| 9 | - | O | $C_2H_5$ | (6-) $CH_3$ | 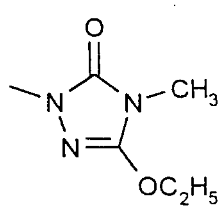 | 162 |

Tabelle 1 - Fortsetzung

| Bsp.-Nr. | A | Q | $R^1$ | (Position-) $R^2$ | $R^3$ | Schmelz-punkt (°C) |
|---|---|---|---|---|---|---|
| 10 | - | O | n-$C_3H_7$ | (6-) $CH_3$ | 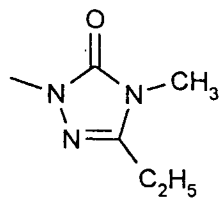 | 126 |
| 11 | - | O | n-$C_3H_7$ | (6-) $CH_3$ | 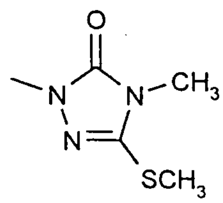 | 150 |
| 12 | - | O | n-$C_3H_7$ | (6-) $CH_3$ | 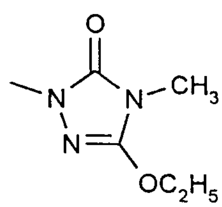 | 129 |
| 13 | - | O | $CH_3$ | (6-) $CH_3$ | 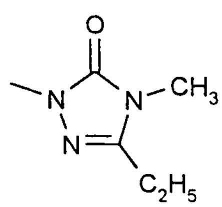 | 153 |
| 14 | - | O | $CH_3$ | (6-) $CH_3$ | 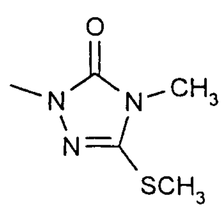 | 167 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 15 | - | O | $CH_3$ | (6-) $CH_3$ | 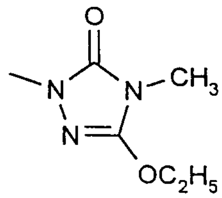 | 167 |
| 16 | - | O | i-$C_3H_7$ | (6-) $CH_3$ | 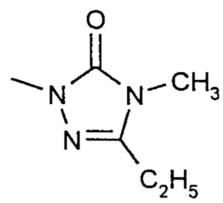 | 125 |
| 17 | - | O | i-$C_3H_7$ | (6-) $CH_3$ | 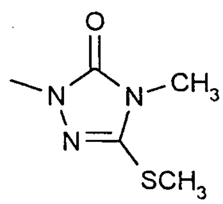 | 131 |
| 18 | - | O | $C_2H_5$ | (5-) $CH_3$ | 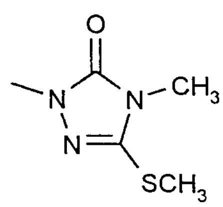 | 222 |
| 19 | - | O | $C_2H_5$ | (5-) $CH_3$ | 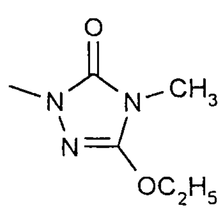 | 139 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 20 | - | O | $C_2H_5$ | (4-) $CH_3$ | 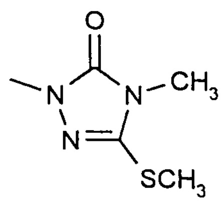 | 189 |
| 21 | - | O | $C_2H_5$ | (5-) $CH_3$ | 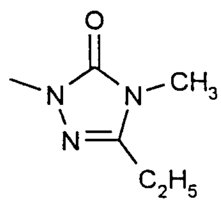 | 131 |
| 22 | - | O | $-C_2H_4-OC_2H_5$ | (6-) $CH_3$ | 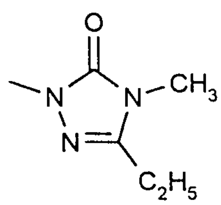 | 118 |
| 23 | - | O | $-CH_2CH_2Cl$ | (6-) $CH_3$ | 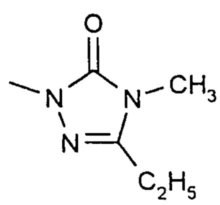 | 137 |
| 24 | - | O | $-CH_2CH_2Cl$ | (6-) $CH_3$ | 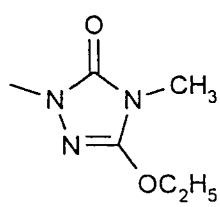 | 149 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 25 | - | O | i-$C_3H_7$ | (5-) $CH_3$ | 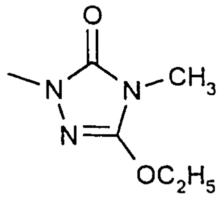 | 125 |
| 26 | - | O | i-$C_3H_7$ | (5-) $CH_3$ | 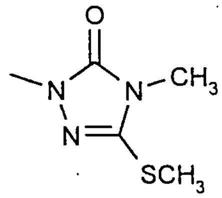 | 140 |
| 27 | - | O | n-$C_3H_7$ | (5-) $CH_3$ | 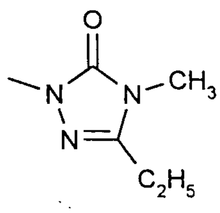 | 119 |
| 28 | - | O | n-$C_3H_7$ | (5-) $CH_3$ | 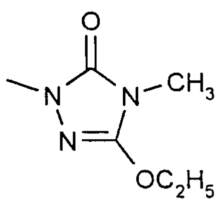 | 134 |
| 29 | - | O | n-$C_3H_7$ | (5-) $CH_3$ | 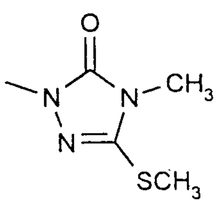 | 110 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 30 | - | O | $C_2H_5$ | (6-) $CH_3$ | 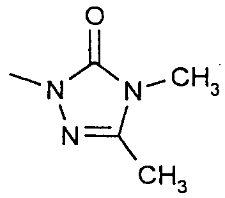 | 108 |
| 31 | - | O | $C_2H_5$ | (6-) $CH_3$ | 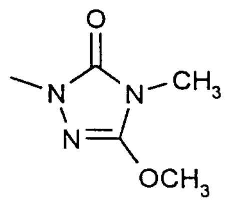 | 173 |
| 32 | - | O | $C_2H_5$ | (6-) $CH_3$ | 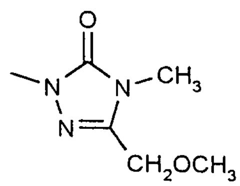 | 119 |
| 33 | - | O | $C_2H_5$ | (6-) $CH_3$ | 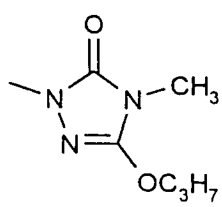 | 121 |
| 34 | - | O | $C_2H_5$ | (6-) $CH_3$ | 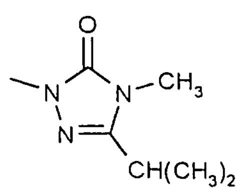 | 109 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 35 | - | O | $C_2H_5$ | (6-) $CH_3$ | 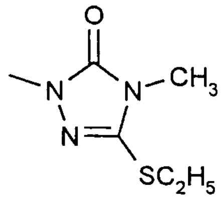 | 111 |
| 36 | - | O | n-$C_3H_7$ | (6-) $CH_3$ | 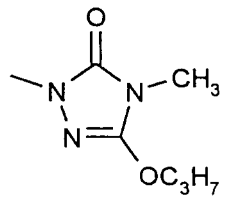 | 91 |
| 37 | - | O | n-$C_3H_7$ | (6-) $CH_3$ | 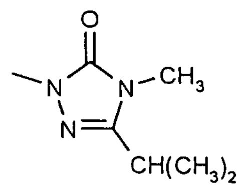 | 130 |
| 38 | - | O | n-$C_3H_7$ | (6-) $CH_3$ | 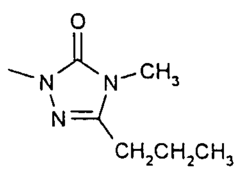 | 126 |
| 39 | - | O | n-$C_3H_7$ | (6-) $CH_3$ | 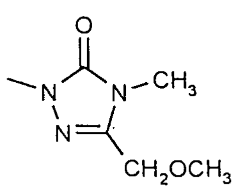 | 101 |

Tabelle 1 - Fortsetzung

| Bsp.-Nr. | A | Q | $R^1$ | (Position-) $R^2$ | $R^3$ | Schmelz-punkt (°C) |
|---|---|---|---|---|---|---|
| 40 | - | O | n-$C_3H_7$ | (6-) $CH_3$ | 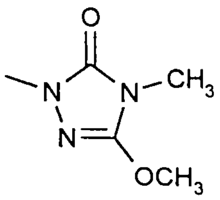 | 152 |
| 41 | - | O | n-$C_3H_7$ | (6-) $CH_3$ | 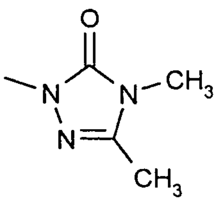 | 100 |
| 42 | - | O | n-$C_3H_7$ | (6-) $CH_3$ | 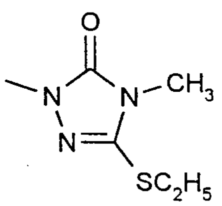 | 120 |
| 43 | - | O | n-$C_3H_7$ | (6-) $CH_3$ | 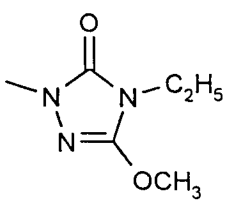 | 117 |
| 44 | - | O | n-$C_3H_7$ | (6-) $CH_3$ | 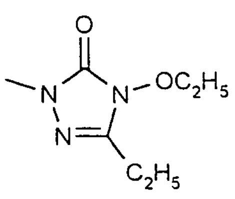 | 126 |

Tabelle 1 - Fortsetzung

| Bsp.-Nr. | A | Q | $R^1$ | (Position-) $R^2$ | $R^3$ | Schmelz-punkt (°C) |
|---|---|---|---|---|---|---|
| 45 | - | O | n-$C_3H_7$ | (6-) $CH_3$ | 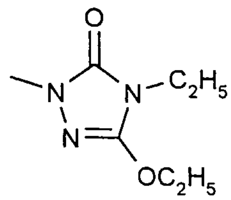 | 113 |
| 46 | - | O | i-$C_3H_7$ | (6-) $CH_3$ | 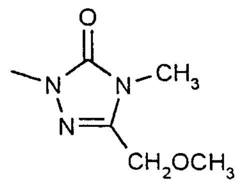 | 130 |
| 47 | - | O | i-$C_3H_7$ | (6-) $CH_3$ | 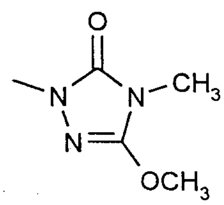 | 139 |
| 48 | - | O | i-$C_3H_7$ | (6-) $CH_3$ | 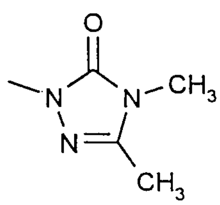 | 121 |
| 49 | - | O | i-$C_3H_7$ | (6-) $CH_3$ | 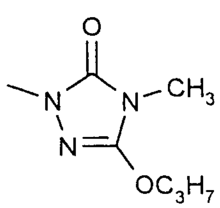 | 119 |

Tabelle 1 - Fortsetzung

| Bsp.-Nr. | A | Q | $R^1$ | (Position-) $R^2$ | $R^3$ | Schmelz-punkt (°C) |
|---|---|---|---|---|---|---|
| 50 | - | O | i-$C_3H_7$ | (6-) $CH_3$ | 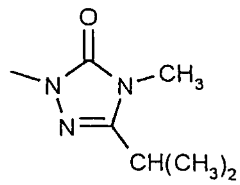 | 128 |
| 51 | - | O | i-$C_3H_7$ | (6-) $CH_3$ | 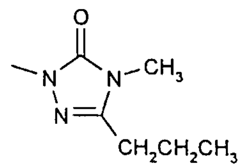 | 134 |
| 52 | - | O | i-$C_3H_7$ | (6-) $CH_3$ | 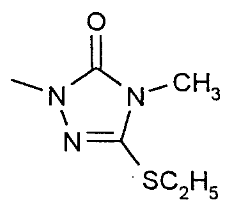 | 130 |
| 53 | - | O | i-$C_3H_7$ | (6-) $CH_3$ | 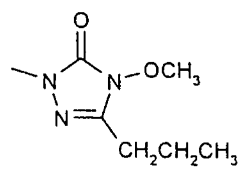 | 117 |
| 54 | - | O | i-$C_3H_7$ | (6-) $CH_3$ | 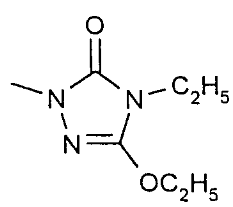 | 134 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 55 | - | O | $i-C_3H_7$ | (6-) $CH_3$ | 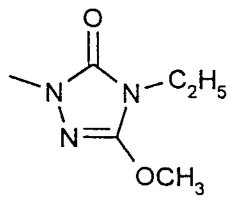 | 141 |
| 56 | - | O | $i-C_3H_7$ | (6-) $CH_3$ | 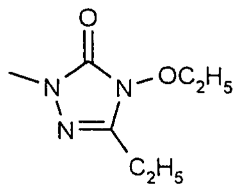 | 132 |
| 57 | - | O | $i-C_3H_7$ | (6-) $CH_3$ | 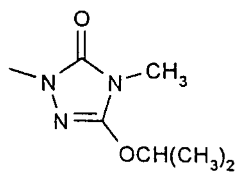 | 166 |
| 58 | - | O | $i-C_3H_7$ | (6-) $CH_3$ | 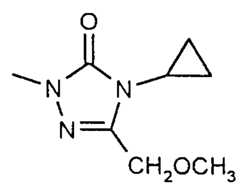 | 118 |
| 59 | - | O | $i-C_3H_7$ | (6-) $CH_3$ | 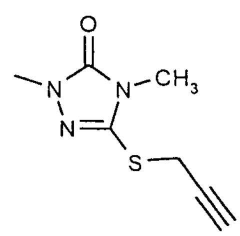 | 150 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 60 | - | O | i-$C_3H_7$ | (6-) $CH_3$ | 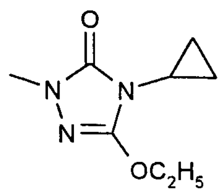 | 144 |
| 61 | - | O | i-$C_3H_7$ | (6-) $CH_3$ | 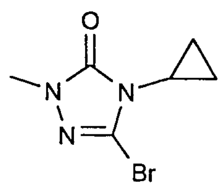 | 170 |
| 62 | - | O | i-$C_3H_7$ | (6-) $CH_3$ | 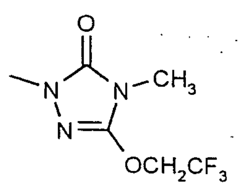 | 120 |
| 63 | - | O | n-$C_3H_7$ | (6-) $CH_3$ | 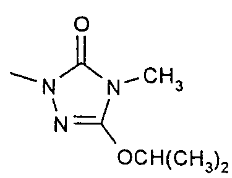 | 124 |
| 64 | - | O | n-$C_3H_7$ | (6-) $CH_3$ | 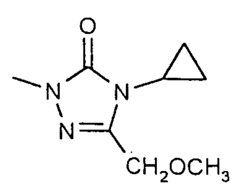 | 125 |
| 65 | - | O | n-$C_3H_7$ | (6-) $CH_3$ | 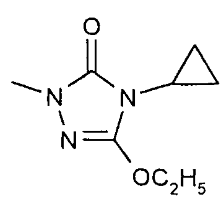 | 116 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 66 | - | O | n-$C_3H_7$ | (6-) $CH_3$ | 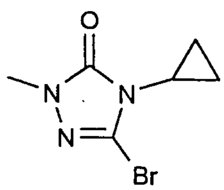 | 152 |
| 67 | - | O | n-$C_3H_7$ | (6-) $CH_3$ | 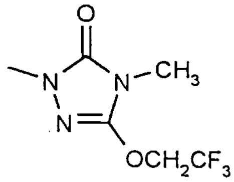 | 143 |
| 68 | - | O | $C_2H_5$ | (6-) $CH_3$ | 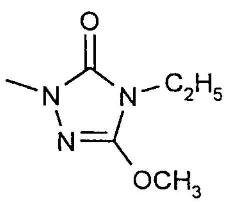 | 160 |
| 69 | - | O | $C_2H_5$ | (6-) $CH_3$ | 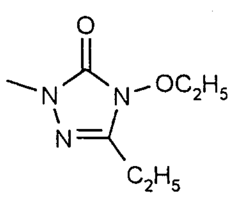 | 133 |
| 70 | - | O | $C_2H_5$ | (6-) $CH_3$ | 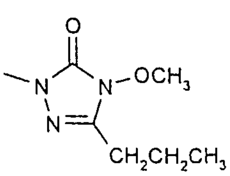 | 97 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 71 | - | O | $C_2H_5$ | (6-) $CH_3$ | 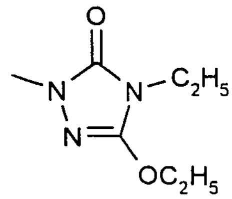 | 96 |
| 72 | - | O | $C_2H_5$ | (6-) $CH_3$ | 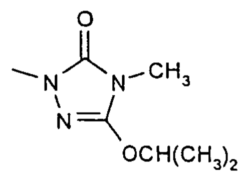 | 156 |
| 73 | - | O | $C_2H_5$ | (6-) $CH_3$ | 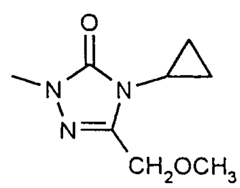 | 145 |
| 74 | - | O | $C_2H_5$ | (6-) $CH_3$ | 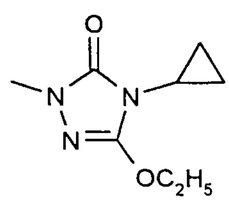 | 120 |
| 75 | - | O | $C_2H_5$ | (6-) $CH_3$ | 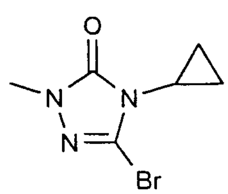 | 125 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 76 | - | O | $C_2H_5$ | (6-) $CH_3$ | 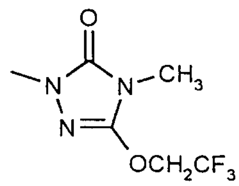 | 140 |
| 77 | - | O | H | (6-) $CH_3$ | 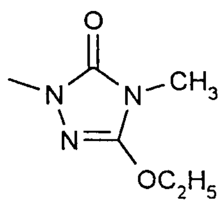 | 88 |
| 78 | - | O | $C_2H_5$ | (5-) $CH_3$ | 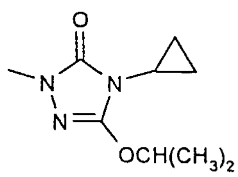 | 130 |
| 79 | - | O | n-$C_3H_7$ | (6-) $CH_3$ | 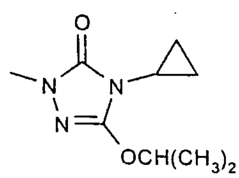 | 141 |
| 80 | - | O | n-$C_3H_7$ | (6-) $CH_3$ | 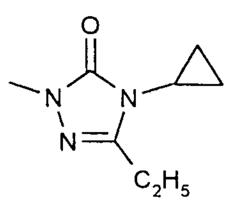 | 98 |

Tabelle 1 - Fortsetzung

| Bsp. Nr. | A | Q | $R^1$ | (Position-) $R^2$ | $R^3$ | Schmelz-punkt (°C) |
|---|---|---|---|---|---|---|
| 81 | - | O | n-$C_3H_7$ | (6-) $CH_3$ | 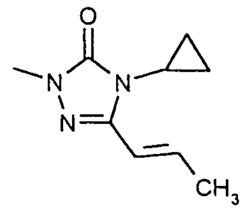 | 141 |
| 82 | - | O | n-$C_3H_7$ | (6-) $CH_3$ | 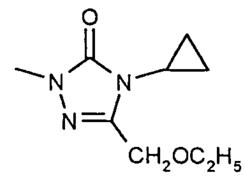 | 101 |
| 83 | - | O | n-$C_3H_7$ | (6-) $CH_3$ | 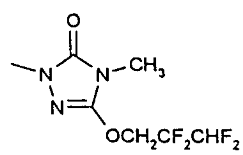 | 136 |
| 84 | - | O | n-$C_3H_7$ | (6-) $CH_3$ | 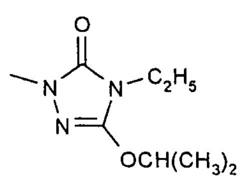 | 96 |
| 85 | - | O | n-$C_3H_7$ | (6-) $CH_3$ | 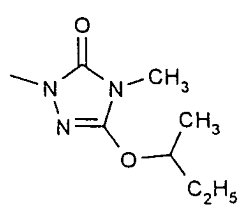 | 90 |
| 86 | - | O | n-$C_3H_7$ | (6-) $CH_3$ | 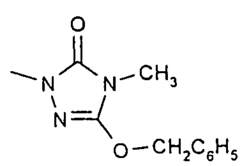 | 136 |

Tabelle 1 - Fortsetzung

| Bsp. Nr. | A | Q | $R^1$ | (Position-) $R^2$ | $R^3$ | Schmelzpunkt (°C) |
|---|---|---|---|---|---|---|
| 87 | - | O | $C_2H_5$ | (6-) $CH_3$ | 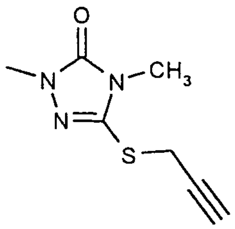 | 122 |
| 88 | - | O | $C_2H_5$ | (6-) $CH_3$ | 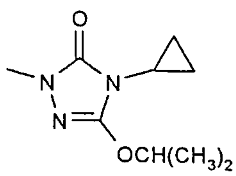 | 154 |
| 89 | - | O | i-$C_3H_7$ | (6-) $CH_3$ | 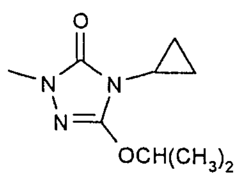 | 139 |
| 90 | - | O | $C_2H_5$ | (6-) $CH_3$ | 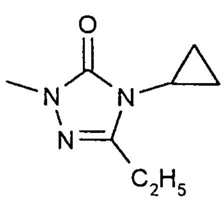 | 142 |
| 91 | - | O | $C_2H_5$ | (6-) $CH_3$ | 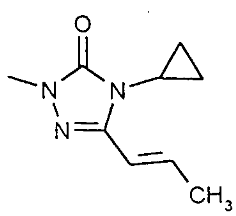 | 153 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 92 | - | O | $C_2H_5$ | (6-) $CH_3$ | 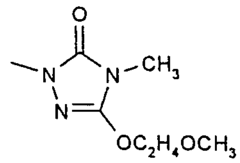 | 145 |
| 93 | - | O | $C_2H_5$ | (6-) $CH_3$ | 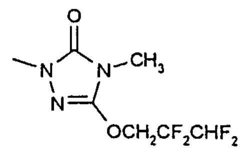 | 132 |
| 94 | - | O | $C_2H_5$ | (6-) $CH_3$ | 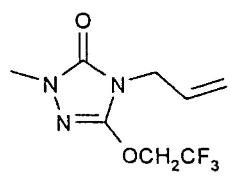 | 141 |
| 95 | - | O | $C_2H_5$ | (6-) $CH_3$ | 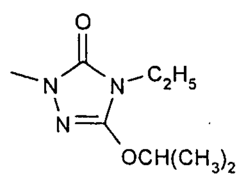 | 130 |
| 96 | - | O | $CH_3$ | (5-) $CH_3$ | 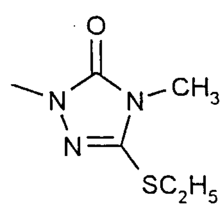 | 156 |
| 97 | - | O | $CH_3$ | (5-) $CH_3$ | 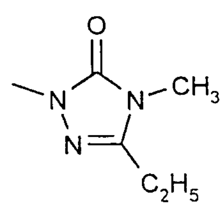 | 177 |

Tabelle 1 - Fortsetzung

| Bsp. Nr. | A | Q | $R^1$ | (Position-) $R^2$ | $R^3$ | Schmelzpunkt (°C) |
|---|---|---|---|---|---|---|
| 98 | - | O | $CH_3$ | (4-) $CH_3$ | 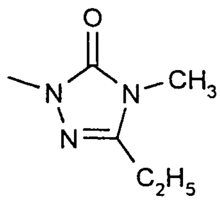 | 115 |
| 99 | - | O | $CH_3$ | (4-) $CH_3$ | 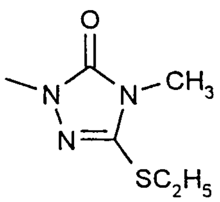 | 166 |
| 100 | - | O | $CH_3$ | (3-) $CH_3$ | 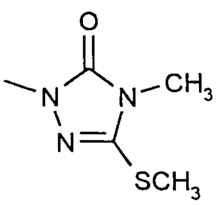 | 162 |
| 101 | - | O | $CH_3$ | (3-) $CH_3$ | 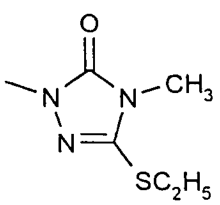 | 143 |
| 102 | - | O | $CH_3$ | (3-) $CH_3$ | 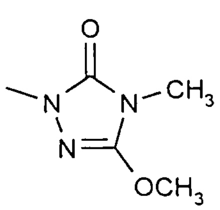 | 165 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 103 | - | O | $CHF_2$ | (5-) $CH_3$ | 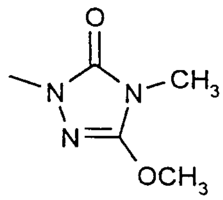 | 176 |
| 104 | - | O | $CHF_2$ | (5-) $CH_3$ | 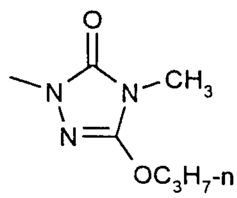 | 119 |
| 105 | - | O | $CHF_2$ | (5-) $CH_3$ | 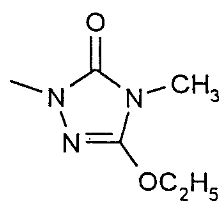 | 126 |
| 106 | - | O | $CHF_2$ | (5-) $CH_3$ | 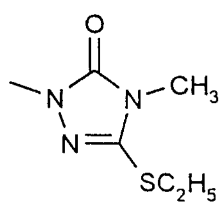 | 151 |
| 107 | - | O | $CHF_2$ | (5-) $CH_3$ | 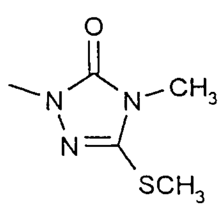 | 188 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 108 | - | O | $CHF_2$ | (5-) $CH_3$ | 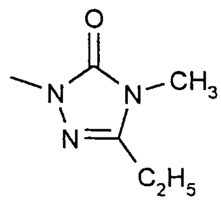 | 137 |
| 109 | - | O | $CHF_2$ | (5-) $CH_3$ | 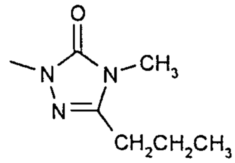 | 117 |
| 110 | - | O | $CHF_2$ | (5-) $CH_3$ | 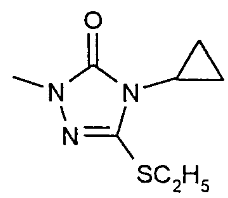 | 155 |
| 111 | - | O | $CHF_2$ | (4-) $CH_3$ | 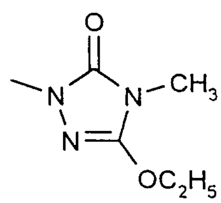 | 152 |
| 112 | - | O | $CHF_2$ | (4-) $CH_3$ | 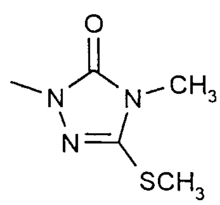 | 176 |
| 113 | - | O | $CHF_2$ | (4-) $CH_3$ | 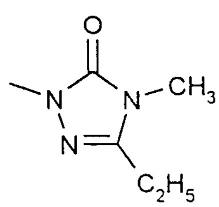 | 108 |

Tabelle 1 - Fortsetzung

| Bsp. Nr. | A | Q | $R^1$ | (Position-) $R^2$ | $R^3$ | Schmelzpunkt (°C) |
|---|---|---|---|---|---|---|
| 114 | - | O | $CHF_2$ | (6-) $CH_3$ | 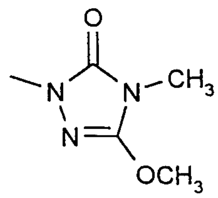 | 163 |
| 115 | - | O | $CHF_2$ | (6-) $CH_3$ | 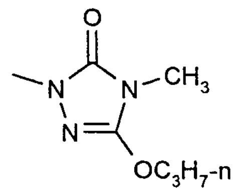 | 136 |
| 116 | - | O | $CHF_2$ | (6-) $CH_3$ | 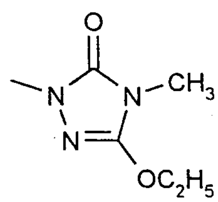 | 118 |
| 117 | - | O | $CHF_2$ | (6-) $CH_3$ | 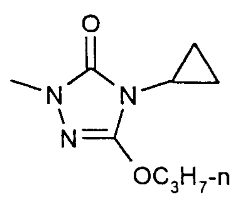 | 104 |
| 118 | - | O | $CHF_2$ | (5-) $CH_3$ | 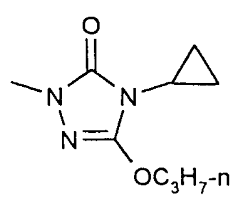 | 98 |
| 119 | - | O | $CHF_2$ | (6-) $CH_3$ | 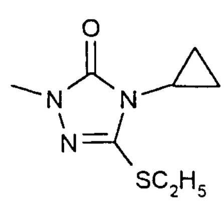 | 128 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 120 | - | O | $CHF_2$ | (6-) $CH_3$ | 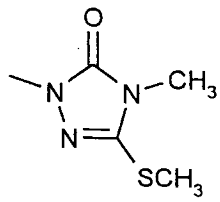 | 165 |
| 121 | - | O | $CHF_2$ | (6-) $CH_3$ | 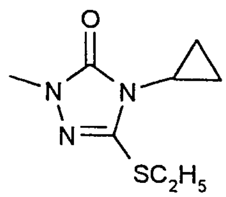 | 155 |
| 122 | - | O | $CHF_2$ | (6-) $CH_3$ | 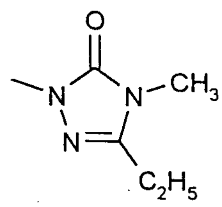 | 105 |
| 123 | - | O | $CHF_2$ | (6-) $CH_3$ | 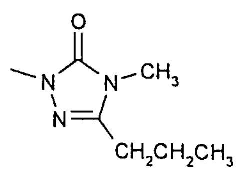 | 81 |
| 124 | - | O | $CHF_2$ | (6-) $CH_3$ | 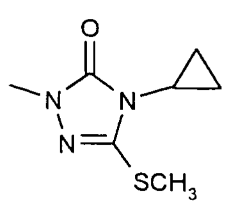 | 174 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 125 | - | O | $CHF_2$ | (5-) $CH_3$ | 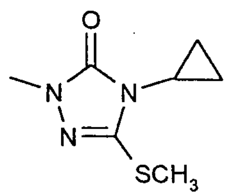 | 150 |
| 126 | - | O | $CHF_2$ | (6-) $CH_3$ | 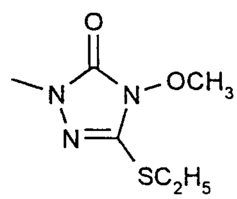 | 124 |
| 127 | - | O | $CHF_2$ | (6-) $CH_3$ | 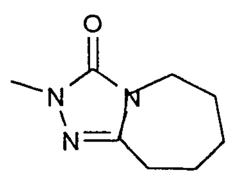 | 200 |
| 128 | - | S | n-$C_3H_7$ | (6-) $CH_3$ | 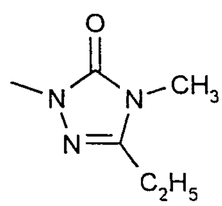 | 160 |
| 129 | - | S | n-$C_3H_7$ | (6-) $CH_3$ | 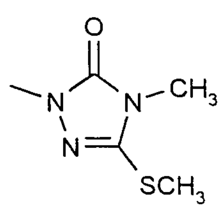 | 148 |
| 130 | - | S | $C_2H_5$ | (6-) $CH_3$ | 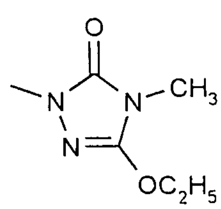 | 141 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 131 | - | S | $C_2H_5$ | (6-) $CH_3$ | 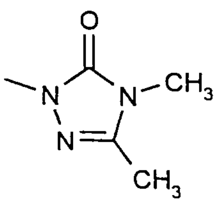 | 125 |
| 132 | - | S | $C_2H_5$ | (6-) $CH_3$ | 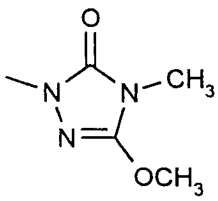 | 158 |
| 133 | - | S | i-$C_3H_7$ | (6-) $CH_3$ | 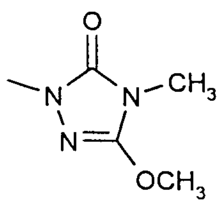 | 155 |
| 134 | - | S | n-$C_3H_7$ | (6-) $CH_3$ | 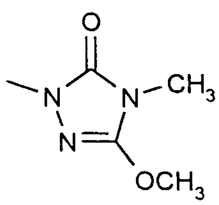 | 153 |
| 135 | - | S | n-$C_3H_7$ | (6-) $CH_3$ | 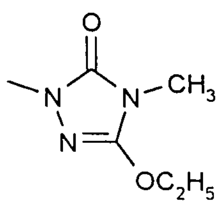 | 131 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 136 | - | S | n-$C_3H_7$ | (6-) $CH_3$ | 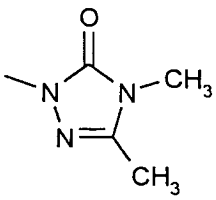 | 120 |
| 137 | - | O | $C_2H_5$ | (6-) Cl | 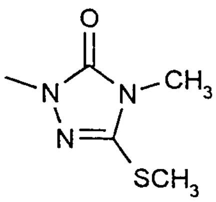 | 149 |
| 138 | - | O | $C_2H_5$ | (6-) Cl | 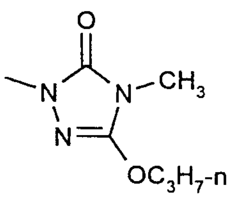 | 99 |
| 139 | - | O | $CH_3$ | (6-) Cl | 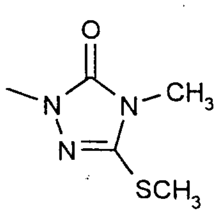 | 176 |
| 140 | - | O | $CH_3$ | (6-) Cl | 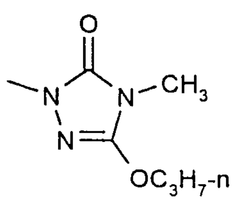 | 192 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 141 | - | O | $C_2H_5$ | (6-) Cl | 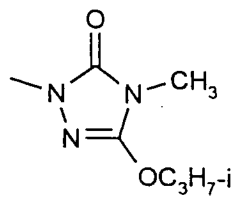 | 144 |
| 142 | - | O | $CH_3$ | (6-) Cl | 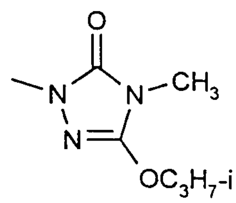 | 114 |
| 143 | - | O | $C_2H_5$ | (6-) Cl | 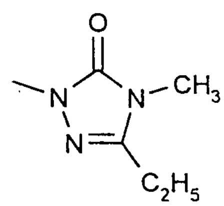 | 144 |
| 144 | - | O | $CH_3$ | (6-) Cl | 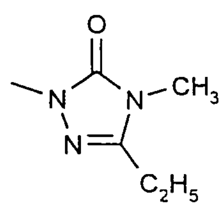 | 157 |
| 145 | - | O | $C_2H_5$ | (6-) Cl | 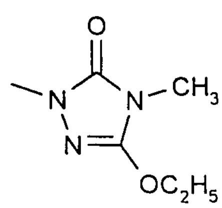 | 142 |

Tabelle 1 - Fortsetzung

| Bsp.-Nr. | A | Q | $R^1$ | (Position-) $R^2$ | $R^3$ | Schmelz-punkt (°C) |
|---|---|---|---|---|---|---|
| 146 | - | O | $CH_3$ | (6-) Cl | 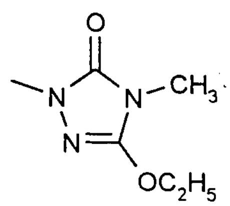 | 191 |
| 147 | - | O | $C_2H_5$ | (6-) Cl | 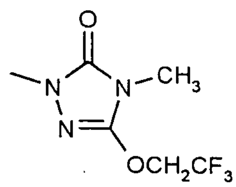 | 116 |
| 148 | - | O | $CH_3$ | (6-) Cl | 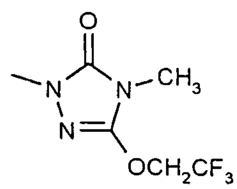 | 205 |
| 149 | - | O | $CH_3$ | (6-) Cl | 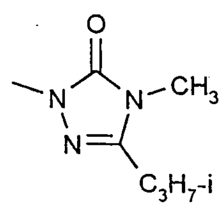 | 147 |
| 150 | - | O | $C_2H_5$ | (6-) Cl | 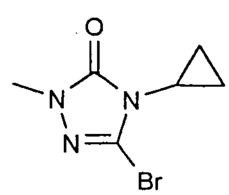 | 117 |

Tabelle 1 - Fortsetzung

| Bsp. Nr. | A | Q | $R^1$ | (Position-) $R^2$ | $R^3$ | Schmelz-punkt (°C) |
|---|---|---|---|---|---|---|
| 151 | - | O | $CH_3$ | (6-) Cl | 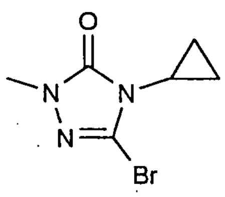 | 149 |
| 152 | - | O | $CH_3$ | (6-) Cl | 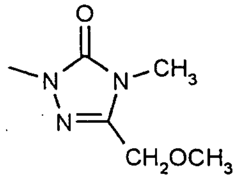 | 176 |
| 153 | - | O | $CH_3$ | (6-) Cl | 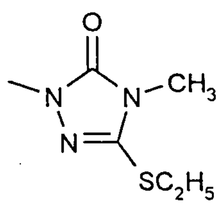 | 150 |
| 154 | - | O | $CH_3$ | (6-) Cl | 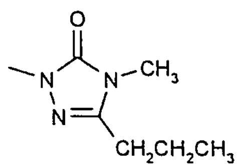 | 146 |
| 155 | - | O | $CH_3$ | (6-) Cl | 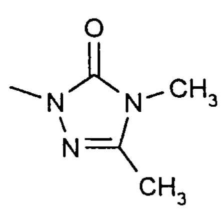 | 191 |
| 156 | - | O | $CH_3$ | (6-) Cl | 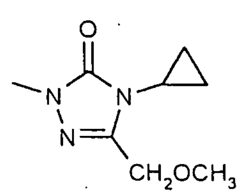 | 127 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 157 | - | O | $CH_3$ | (6-) Cl | 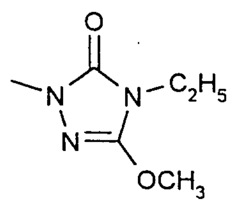 | 174 |
| 158 | - | O | $n-C_3H_7$ | (6-) Cl | 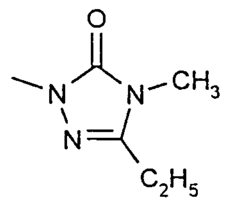 | 117 |
| 159 | - | O | $n-C_3H_7$ | (6-) Cl | 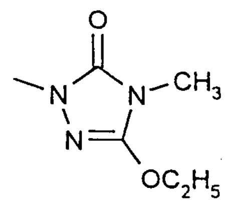 | 134 |
| 160 | - | O | $n-C_3H_7$ | (6-) Cl | 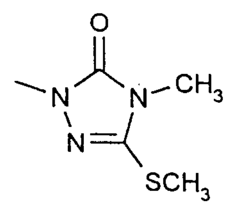 | 115 |
| 161 | - | O | $n-C_3H_7$ | (6-) Cl | 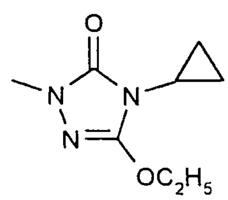 | 137 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 162 | - | O | n-$C_3H_7$ | (6-) Cl | 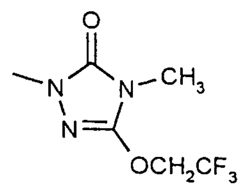 | 125 |
| 163 | - | O | n-$C_3H_7$ | (6-) Cl | 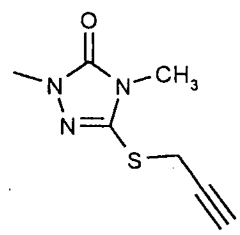 | 119 |
| 164 | - | O | H | (6-) Cl | 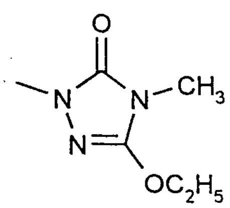 | 147 |
| 165 | - | O | n-$C_3H_7$ | (6-) Cl | 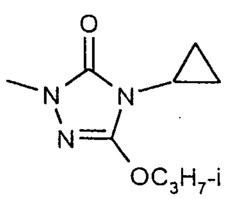 | 148 |
| 166 | - | O | i-$C_3H_7$ | (6-) Cl | 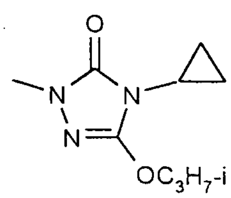 | 143 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 167 | - | O | i-$C_3H_7$ | (6-) Cl | 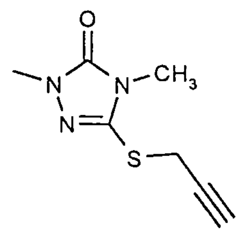 | 122 |
| 168 | - | O | $CH_3$ | (6-) Cl | 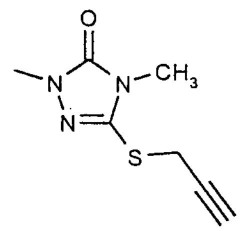 | 165 |
| 169 | - | O | n-$C_3H_7$ | (5-) Cl | 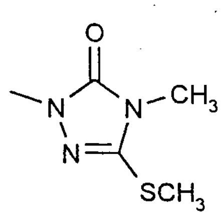 | 154 |
| 170 | - | O | n-$C_3H_7$ | (5-) Cl | 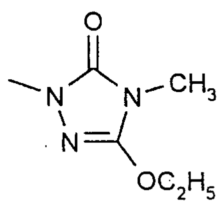 | 136 |
| 171 | - | O | n-$C_3H_7$ | (5-) Cl | 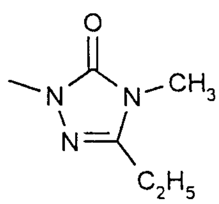 | 128 |

Tabelle 1 - Fortsetzung

| Bsp. Nr. | A | Q | $R^1$ | (Position-) $R^2$ | $R^3$ | Schmelz-punkt (°C) |
|---|---|---|---|---|---|---|
| 172 | - | O | $CH_3$ | (6-) Cl | 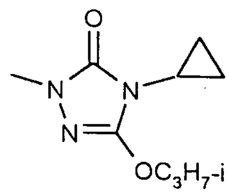 | 143 |
| 173 | - | O | $i-C_3H_7$ | (6-) Cl | 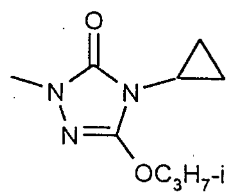 | 136 |
| 174 | - | O | $i-C_3H_7$ | (6-) Cl | 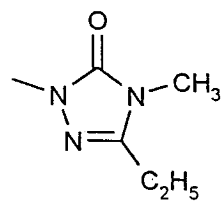 | 121 |
| 175 | - | O | $i-C_3H_7$ | (6-) Cl | 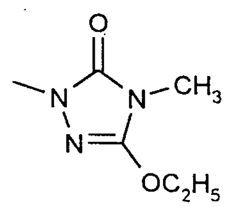 | 158 |
| 176 | - | O | $i-C_3H_7$ | (6-) Cl | 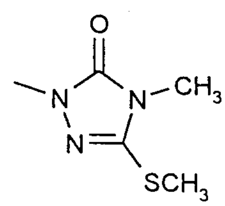 | 141 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 177 | - | O | i-$C_3H_7$ | (6-) Cl | 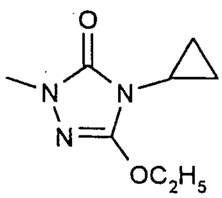 | 127 |
| 178 | - | O | i-$C_3H_7$ | (6-) Cl | 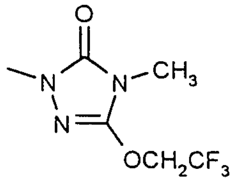 | 143 |
| 179 | - | O | i-$C_3H_7$ | (6-) Cl | 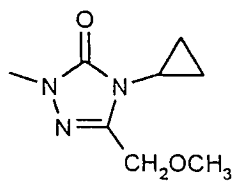 | 129 |
| 180 | - | O | i-$C_3H_7$ | (6-) Cl | 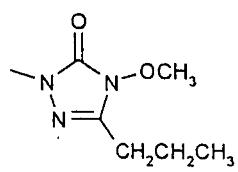 | 95 |
| 181 | - | O | n-$C_3H_7$ | (6-) $CH_3$ | 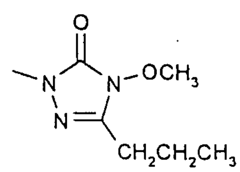 | 74 |
| 182 | - | O | n-$C_3H_7$ | (6-) $CH_3$ | 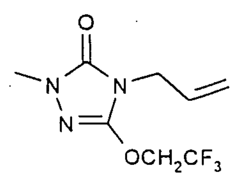 | 114 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 183 | - | O | n-$C_3H_7$ | (6-) $CH_3$ | 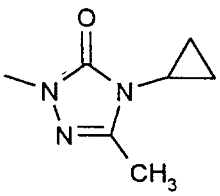 | 140 |
| 184 | - | O | n-$C_3H_7$ | (6-) $CH_3$ | 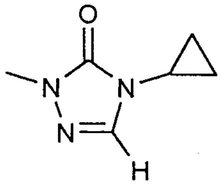 | 159 |
| 185 | - | O | n-$C_3H_7$ | (6-) $CH_3$ | 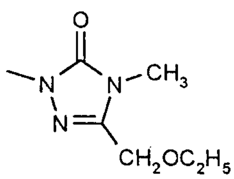 | 107 |
| 186 | - | O | n-$C_3H_7$ | (6-) $CH_3$ | 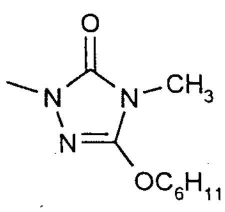 | 132 |
| 187 | - | O | n-$C_3H_7$ | (6-) $CH_3$ | 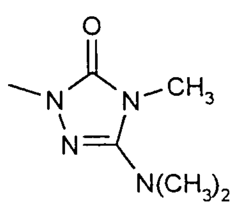 | 132 |

Tabelle 1 - Fortsetzung

| Bsp.-Nr. | A | Q | $R^1$ | (Position-) $R^2$ | $R^3$ | Schmelz-punkt (°C) |
|---|---|---|---|---|---|---|
| 188 | - | O | $n-C_3H_7$ | (6-) $CH_3$ | 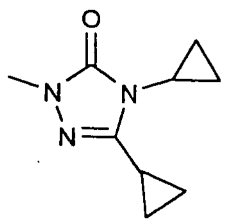 | 110 |
| 189 | - | O | $CH_3$ | (6-) $CH_3$ | 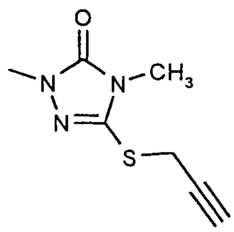 | 159 |
| 190 | - | O | $n-C_3H_7$ | (6-) $CH_3$ | 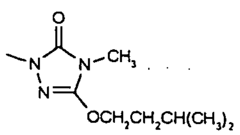 | 138 |
| 191 | - | O | $n-C_3H_7$ | (6-) $CH_3$ | 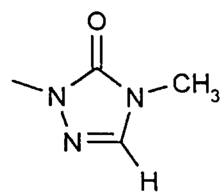 | 147 |
| 192 | - | O | $n-C_3H_7$ | (6-) $CH_3$ | 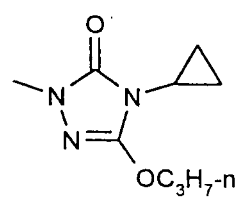 | 114 |
| 193 | - | O | $n-C_3H_7$ | (6-) $CH_3$ | 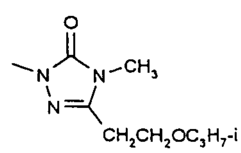 | 125 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 194 | - | O | n-$C_3H_7$ | (6-) $CH_3$ | 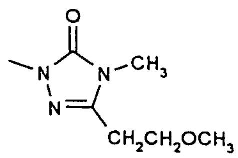 | 126 |
| 195 | - | O | n-$C_3H_7$ | (6-) $CH_3$ | 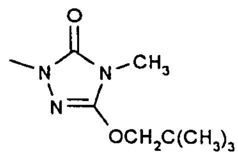 | 151 |
| 196 | - | O | n-$C_3H_7$ | (6-) $CH_3$ | 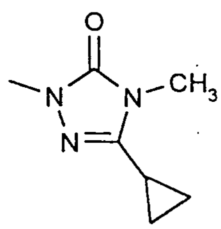 | 121 |
| 197 | - | O | n-$C_3H_7$ | (6-) $CH_3$ | 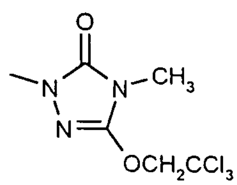 | 147 |
| 198 | NH | O | $C_2H_5$ | (6-) $CH_3$ | 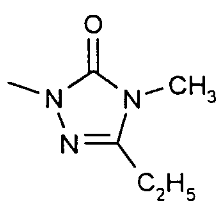 | 135 |
| 199 | - | O | i-$C_3H_7$ | (6-) $CH_3$ | 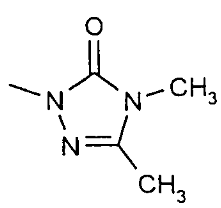 | 263 (Natrium-salz) |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 200 | - | O | $C_2H_5$ | (6-) $CH_3$ | 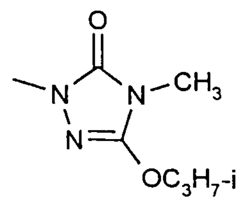 | 119 |
| 201 | - | O | $C_2H_5$ | (6-) $CH_3$ | 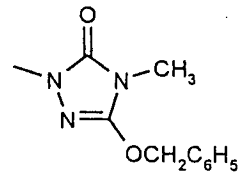 | 146 |
| 202 | - | O | $C_2H_5$ | (6-) $CH_3$ | 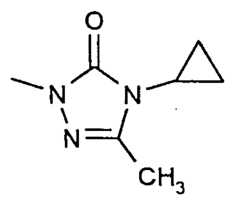 | 128 |
| 203 | - | O | $C_2H_5$ | (6-) $CH_3$ | 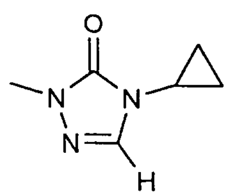 | 186 |
| 204 | - | O | $C_2H_5$ | (6-) $CH_3$ | 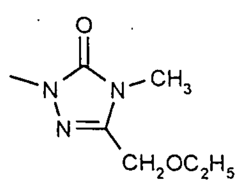 | 239 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 205 | - | O | $C_2H_5$ | (6-) $CH_3$ | 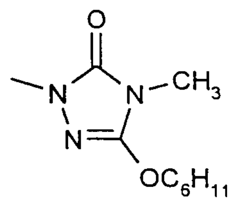 | 152 |
| 206 | - | O | $C_2H_5$ | (6-) $CH_3$ | 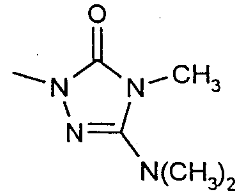 | 155 |
| 207 | - | O | $C_2H_5$ | (6-) $CH_3$ | 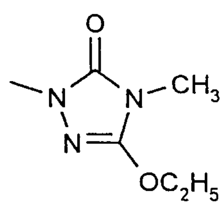 | 145 (Na-Salz) |
| 208 | - | O | i-$C_3H_7$ | (6-) $CH_3$ | 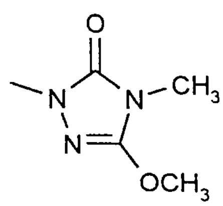 | 209 (Na-Salz) |
| 209 | - | O | $C_2H_5$ | (6-) $CH_3$ | 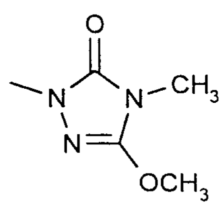 | 147 (Na-Salz) |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 210 | - | O | $C_2H_5$ | (6-) $CH_3$ | 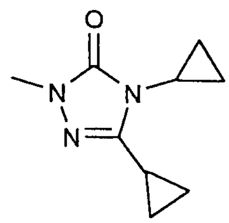 | 140 |
| 211 | - | O | $C_2H_5$ | (6-) $CH_3$ | 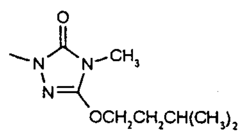 | 118 |
| 212 | - | O | $C_2H_5$ | (6-) $CH_3$ | 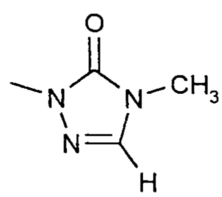 | 156 |
| 213 | - | O | $C_2H_5$ | (6-) $CH_3$ | 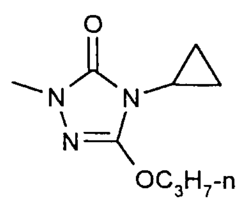 | 110 |
| 214 | - | O | $C_2H_5$ | (6-) $CH_3$ | 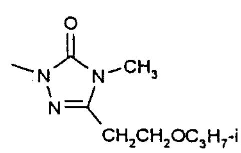 | 133 |
| 215 | - | O | $i$-$C_3H_7$ | (6-) $CH_3$ | 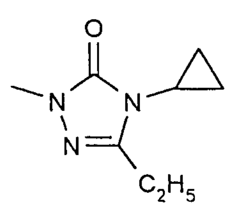 | 138 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 216 | - | O | i-$C_3H_7$ | (6-) $CH_3$ | 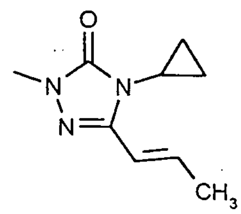 | 154 |
| 217 | - | O | i-$C_3H_7$ | (6-) $CH_3$ | 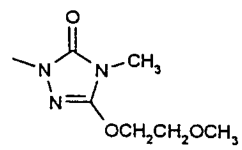 | 149 |
| 218 | - | O | i-$C_3H_7$ | (6-) $CH_3$ | 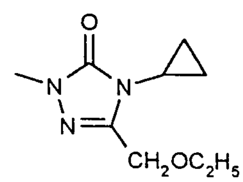 | 112 |
| 219 | - | O | i-$C_3H_7$ | (6-) $CH_3$ | 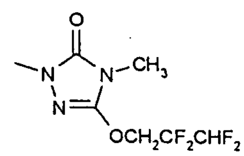 | 162 |
| 220 | - | O | i-$C_3H_7$ | (6-) $CH_3$ | 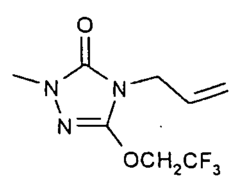 | 99 |
| 221 | - | O | i-$C_3H_7$ | (6-) $CH_3$ | 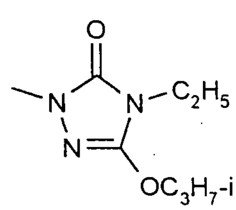 | 146 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 222 | - | O | $C_2H_5$ | (6-) $CH_3$ | 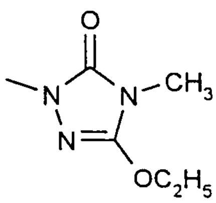 | 134 |
| 223 | - | O | $CH_3$ | (6-) $CH_3$ | 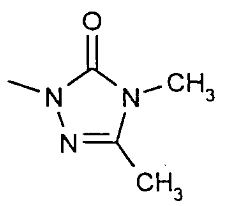 | 199 |
| 224 | - | O | $CH_3$ | (6-) $CH_3$ | 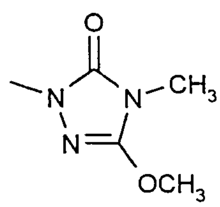 | 176 |
| 225 | - | O | $CH_3$ | (6-) $CH_3$ | 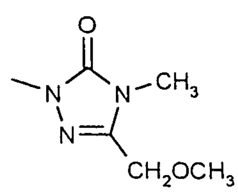 | 145 |
| 226 | - | O | i-$C_3H_7$ | (6-) $CH_3$ | 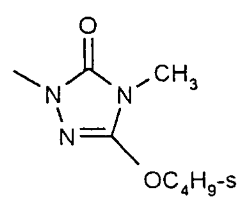 | 133 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 227 | NH | O | n-$C_3H_7$ | (5-) $CH_3$ | 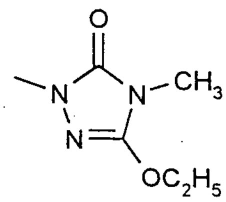 | 127 |
| 228 | - | O | i-$C_3H_7$ | (6-) $CH_3$ | 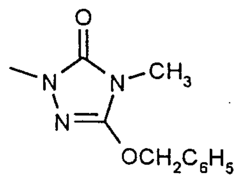 | 144 |
| 229 | - | O | i-$C_3H_7$ | (6-) $CH_3$ | 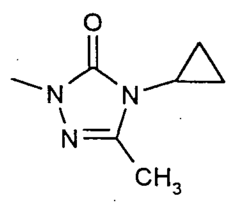 | 141 |
| 230 | - | O | i-$C_3H_7$ | (6-) $CH_3$ | 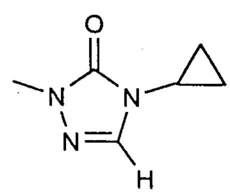 | 152 |
| 231 | - | O | i-$C_3H_7$ | (6-) $CH_3$ | 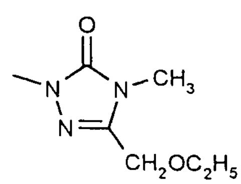 | 132 |
| 232 | - | O | i-$C_3H_7$ | (6-) $CH_3$ | 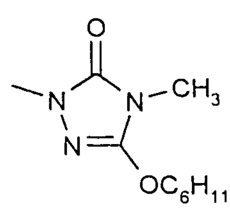 | 147 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 233 | - | O | i-$C_3H_7$ | (6-) $CH_3$ | 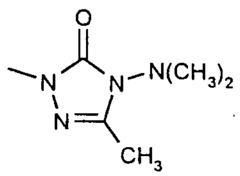 | 163 |
| 234 | - | O | i-$C_3H_7$ | (6-) $CH_3$ | 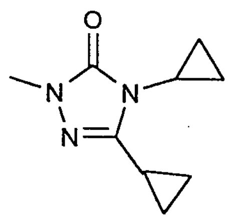 | 102 |
| 235 | - | O | $C_2H_5$ | (6-) $CH_3$ | 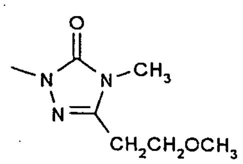 | 121 |
| 236 | - | O | $C_2H_5$ | (6-) $CH_3$ | 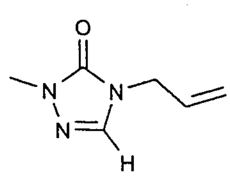 | 113 |
| 237 | - | O | $C_2H_5$ | (6-) $CH_3$ | 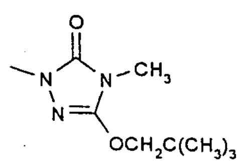 | 145 |
| 238 | - | O | $C_2H_5$ | (6-) $CH_3$ | 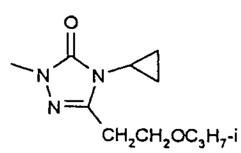 | 137 |

Tabelle 1 - Fortsetzung

| Bsp.-Nr. | A | Q | $R^1$ | (Position-) $R^2$ | $R^3$ | Schmelz-punkt (°C) |
|---|---|---|---|---|---|---|
| 239 | - | O | $C_2H_5$ | (6-) $CH_3$ | 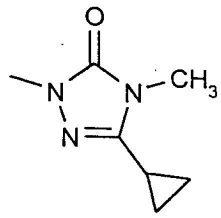 | 172 |
| 240 | - | O | $C_2H_5$ | (6-) $CH_3$ | 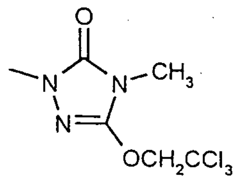 | 148 |
| 241 | - | O | $C_2H_5$ | (6-)$CH_3$ | 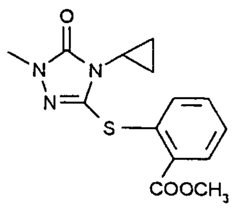 | 157 |
| 242 | - | O | $C_2H_5$ | (6-)$CH_3$ | 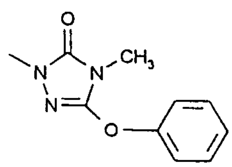 | 186 |
| 243 | NH | O | $CH_3$ | (6-)$OCH_3$ | 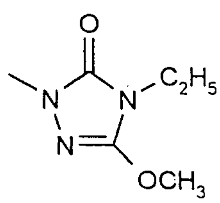 | 170 |
| 244 | - | O | $CHF_2$ | (5-)$CH_3$ | 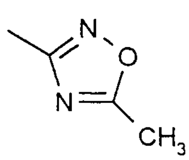 | |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 245 | - | S | $i-C_3H_7$ | (6-)$CH_3$ | 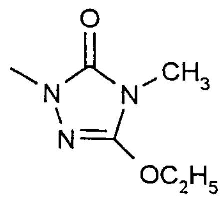 | 160 |
| 246 | - | O | $CH_3$ | (6-)$CF_3$ | 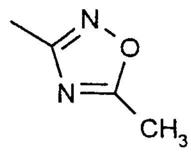 | 205 |
| 247 | - | S | $CH_3$ | (6-)$CF_3$ | 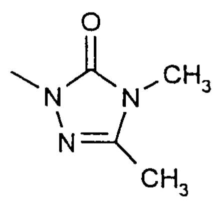 | 92 |
| 248 | - | S | $CH_3$ | (6-)$CF_3$ | 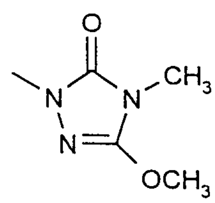 | 154 |
| 249 | - | S | $CH_3$ | (6-)$CF_3$ | 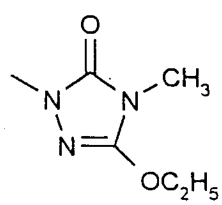 | 157 |
| 250 | - | O | $C_2H_5$ | (6-)$CF_3$ | 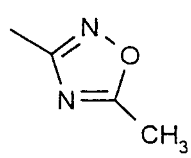 | 176 |

Tabelle 1 - Fortsetzung

| Bsp.-Nr. | A | Q | $R^1$ | (Position-) $R^2$ | $R^3$ | Schmelz-punkt (°C) |
|---|---|---|---|---|---|---|
| 251 | - | O | n-$C_3H_7$-n | (6-)$CF_3$ | 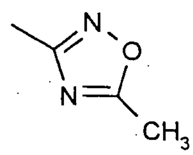 | 166 |
| 252 | - | O | i-$C_3H_7$ | (6-)$CF_3$ | 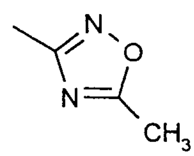 | 190 |
| 253 | - | O | $CH_3$ | (6-)$CF_3$ | 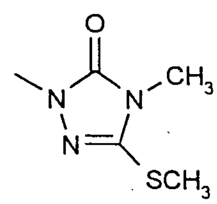 | 203 |
| 254 | - | O | $CH_3$ | (6-)$CF_3$ | 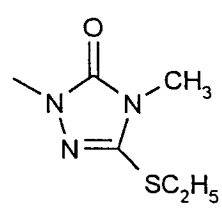 | 156 |
| 255 | - | O | $CH_3$ | (6-)$CF_3$ | 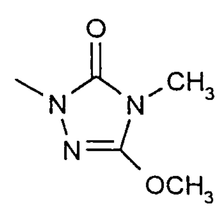 | 170 |
| 256 | - | O | $CH_3$ | (6-)$CF_3$ | 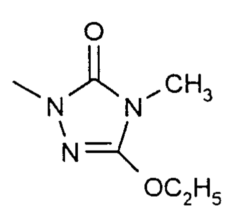 | 198 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 257 | - | O | $CH_3$ | (6-)$CF_3$ | 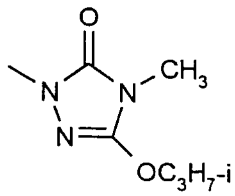 | 213 |
| 258 | - | O | $CH_3$ | (6-)$CF_3$ | 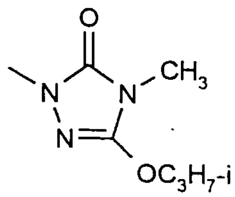 | 152 |
| 259 | - | O | $CH_3$ | (6-)$CF_3$ | 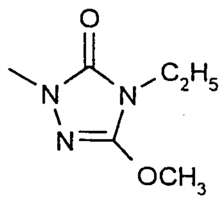 | 187 |
| 260 | - | O | $CH_3$ | (6-)$CF_3$ | 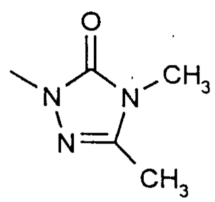 | 210 |
| 261 | - | O | $CH_3$ | (6-)$CF_3$ | 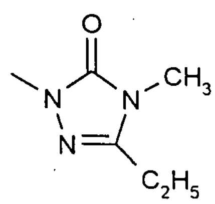 | 172 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 262 | - | O | $CH_3$ | (6-)$CF_3$ | 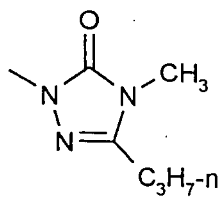 | 145 |
| 263 | - | O | $CH_3$ | (6-)$CF_3$ | 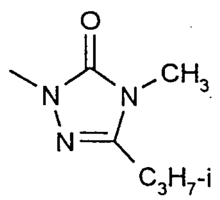 | 136 |
| 264 | - | O | $CH_3$ | (6-)$CF_3$ | 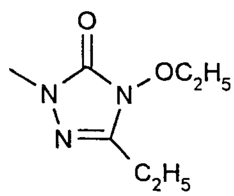 | 153 |
| 265 | - | O | $CH_3$ | (6-)$CF_3$ | 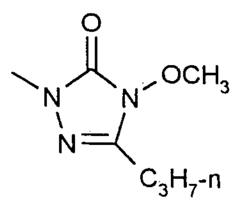 | 136 |
| 266 | - | O | $CH_3$ | (6-)$CF_3$ | 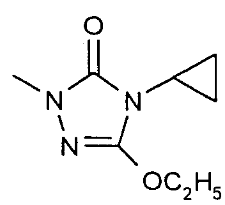 | 210 |

Tabelle 1 - Fortsetzung

| Bsp. Nr. | A | Q | $R^1$ | (Position-) $R^2$ | $R^3$ | Schmelz-punkt (°C) |
|---|---|---|---|---|---|---|
| 267 | - | O | $CH_3$ | (6-)$CF_3$ | 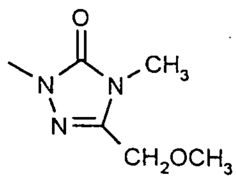 | 147 |
| 268 | - | O | $CH_3$ | (6-)$CF_3$ | 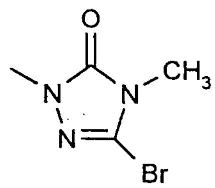 | 169 |
| 269 | - | O | $CH_3$ | (6-)$CF_3$ | 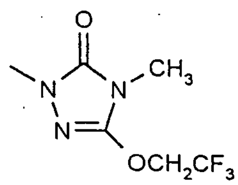 | 215 |
| 270 | - | O | $CH_3$ | (6-)$CF_3$ | 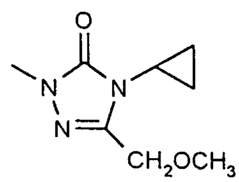 | 138 |
| 271 | - | O | $CH_3$ | (6-)$CF_3$ | 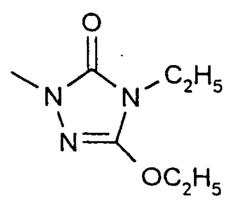 | 182 |
| 272 | - | S | $C_2H_5$ | (6-)$CF_3$ | 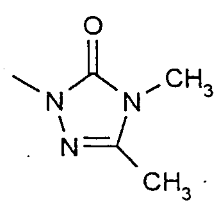 | 112 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 273 | - | S | $C_2H_5$ | (6-)$CF_3$ | 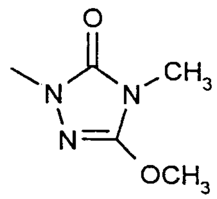 | 167 |
| 274 | - | S | $C_2H_5$ | (6-)$CF_3$ | 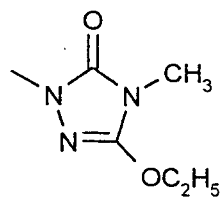 | 152 |
| 275 | - | S | n-$C_3H_7$ | (6-)$CF_3$ | 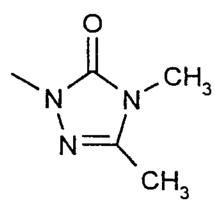 | 119 |
| 276 | - | S | n-$C_3H_7$ | (6-)$CF_3$ | 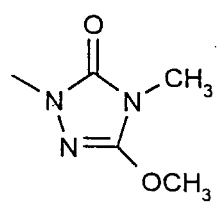 | 157 |
| 277 | - | S | n-$C_3H_7$ | (6-)$CF_3$ | 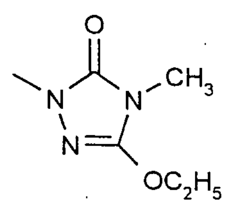 | 154 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 278 | - | S | i-$C_3H_7$ | (6-)$CF_3$ | 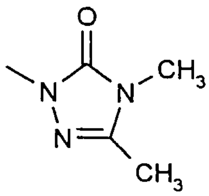 | 137 |
| 279 | - | S | i-$C_3H_7$ | (6-)$CF_3$ | 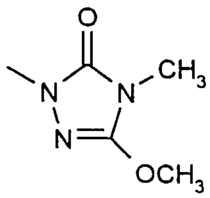 | 167 |
| 280 | - | S | i-$C_3H_7$ | (6-)$CF_3$ | 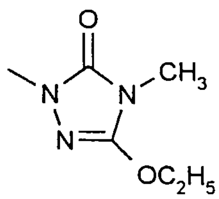 | 137 |
| 281 | - | O | $C_2H_5$ | (6-)$CF_3$ | 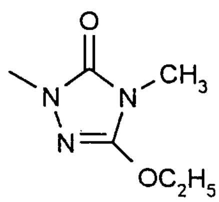 | 154 |
| 282 | - | O | $C_2H_5$ | (6-)$CF_3$ | 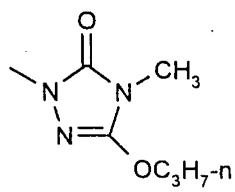 | 160 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 283 | - | O | $C_2H_5$ | (6-)$CF_3$ | 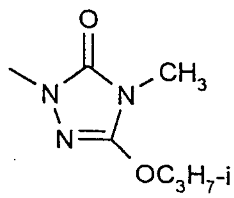 | 139 |
| 284 | - | O | $C_2H_5$ | (6-)$CF_3$ | 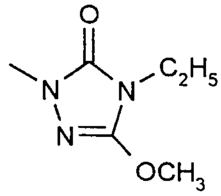 | 134 |
| 285 | - | O | $C_2H_5$ | (6-)$CF_3$ | 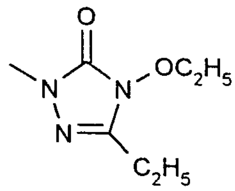 | 142 |
| 286 | - | O | $C_2H_5$ | (6-)$CF_3$ | 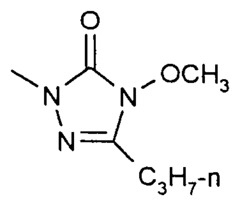 | 120 |
| 287 | - | O | n-$C_3H_7$ | (6-)$CF_3$ | 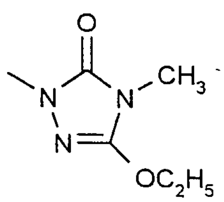 | 130 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 288 | - | O | n-$C_3H_7$ | (6-)$CF_3$ | 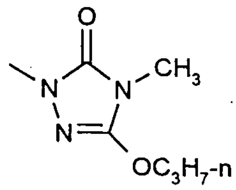 | 127 |
| 289 | - | O | n-$C_3H_7$ | (6-)$CF_3$ | 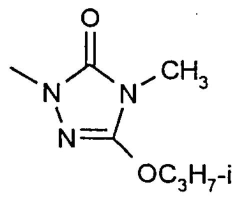 | 116 |
| 290 | - | O | n-$C_3H_7$ | (6-)$CF_3$ | 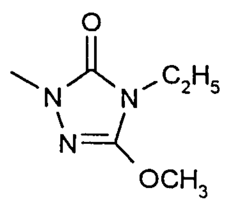 | 126 |
| 291 | - | O | n-$C_3H_7$ | (6-)$CF_3$ | 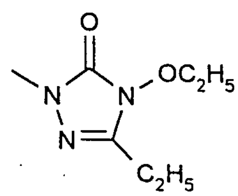 | 113 |
| 292 | - | O | i-$C_3H_7$ | (6-)$CF_3$ | 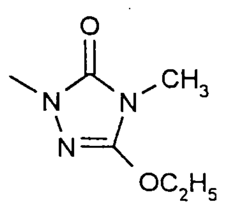 | 151 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 293 | - | O | i-$C_3H_7$ | (6-)$CF_3$ | 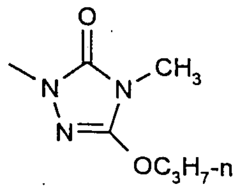 | 157 |
| 294 | - | O | i-$C_3H_7$ | (6-)$CF_3$ | 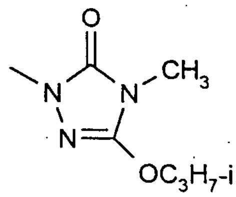 | 171 |
| 295 | - | O | i-$C_3H_7$ | (6-)$CF_3$ | 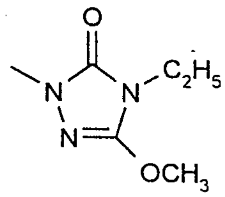 | 137 |
| 296 | - | O | i-$C_3H_7$ | (6-)$CF_3$ | 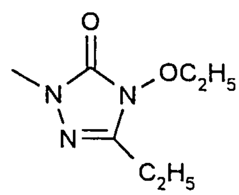 | 125 |
| 297 | - | O | n-$C_3H_7$ | (6-)$CF_3$ | 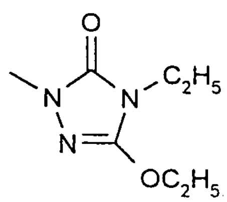 | 109 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 298 | - | O | n-$C_3H_7$ | (6-)$CF_3$ | 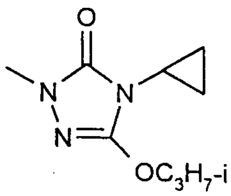 | 138 |
| 299 | - | O | i-$C_3H_7$ | (6-)$CF_3$ | 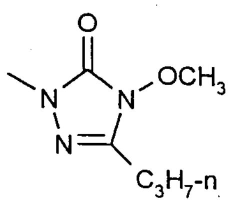 | 130 |
| 300 | - | O | $C_2H_5$ | (6-)$CF_3$ | 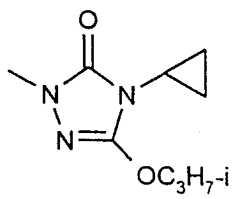 | 165 |
| 301 | - | O | i-$C_3H_7$ | (6-)$CF_3$ | 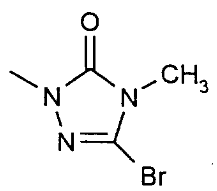 | 148 |
| 302 | - | O | i-$C_3H_7$ | (6-)$CF_3$ | 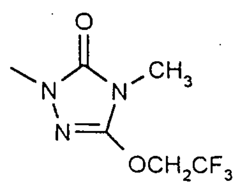 | 147 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 303 | - | O | i-$C_3H_7$ | (6-)$CF_3$ | 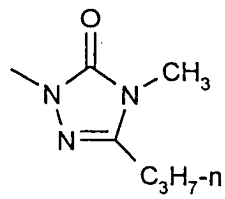 | 172 |
| 304 | - | O | i-$C_3H_7$ | (6-)$CF_3$ | 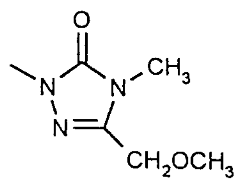 | 147 |
| 305 | - | O | i-$C_3H_7$ | (6-)$CF_3$ | 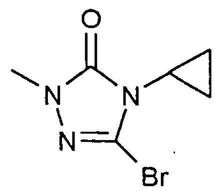 | 136 |
| 306 | - | O | $C_2H_5$ | (6-)$CF_3$ | 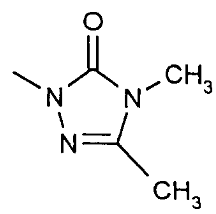 | 124 |
| 307 | - | O | $C_2H_5$ | (6-)$CF_3$ | 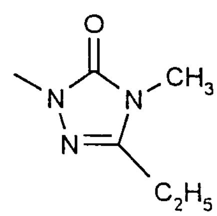 | 98 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 308 | - | O | $C_2H_5$ | (6-)$CF_3$ | 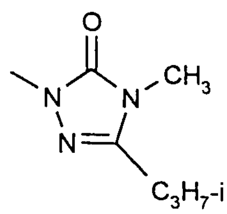 | 125 |
| 309 | - | O | $C_2H_5$ | (6-)$CF_3$ | 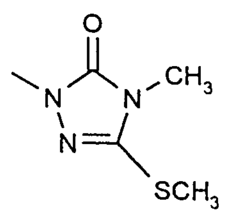 | 179 |
| 310 | - | O | $C_2H_5$ | (6-)$CF_3$ | 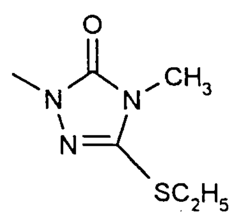 | 153 |
| 311 | - | O | $C_2H_5$ | (6-)$CF_3$ | 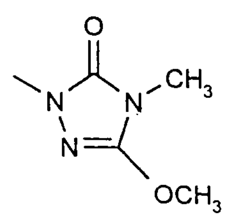 | 171 |
| 312 | - | O | n-$C_3H_7$ | (6-)$CF_3$ | 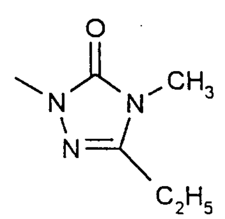 | 113 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 313 | - | O | n-$C_3H_7$ | (6-)$CF_3$ | 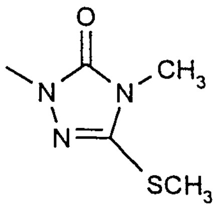 | 138 |
| 314 | - | O | n-$C_3H_7$ | (6-)$CF_3$ | 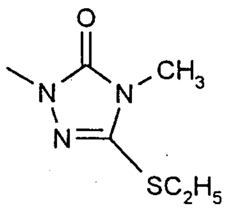 | 110 |
| 315 | - | O | n-$C_3H_7$ | (6-)$CF_3$ | 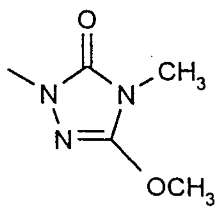 | 134 |
| 316 | - | O | i-$C_3H_7$ | (6-)$CF_3$ | 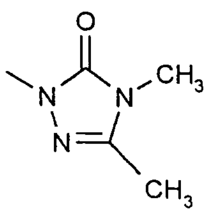 | 167 |
| 317 | - | O | i-$C_3H_7$ | (6-)$CF_3$ | 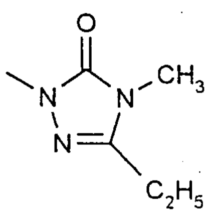 | 120 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 318 | - | O | i-$C_3H_7$ | (6-)$CF_3$ | 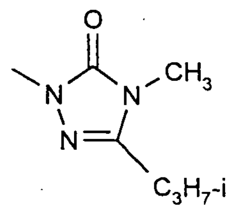 | 117 |
| 319 | - | O | i-$C_3H_7$ | (6-)$CF_3$ | 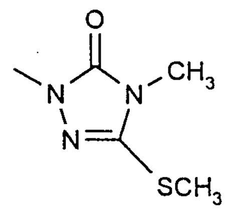 | 160 |
| 320 | - | O | i-$C_3H_7$ | (6-)$CF_3$ | 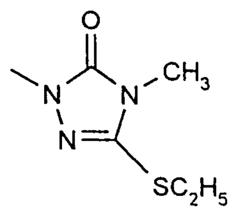 | 154 |
| 321 | - | O | i-$C_3H_7$ | (6-)$CF_3$ | 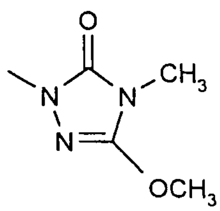 | 159 |
| 322 | - | O | n-$C_3H_7$ | (6-)$CF_3$ | 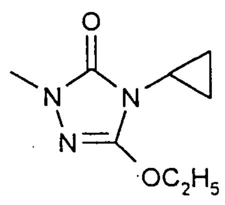 | 141 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 323 | - | O | $i-C_3H_7$ | (6-)$CF_3$ | 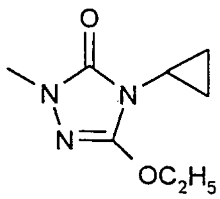 | 146 |
| 324 | - | O | $i-C_3H_7$ | (6-)$CF_3$ | 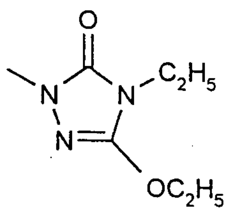 | 134 |
| 325 | - | O | $i-C_3H_7$ | (6-)$CF_3$ | 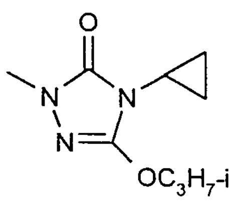 | 168 |
| 326 | - | O | $CH_3$ | (6-)$C_3H_7-n$ | 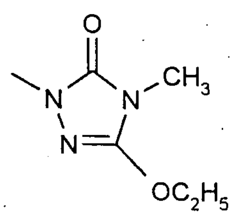 | 158 |
| 327 | - | O | $CH_3$ | (6-)$C_3H_7-n$ | 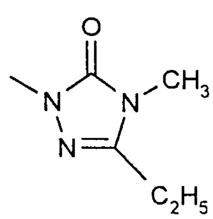 | 172 |

Tabelle 1 - Fortsetzung

| Bsp.-Nr. | A | Q | $R^1$ | (Position-) $R^2$ | $R^3$ | Schmelz-punkt (°C) |
|---|---|---|---|---|---|---|
| 328 | - | O | $CH_3$ | (6-)$C_3H_7$-n | 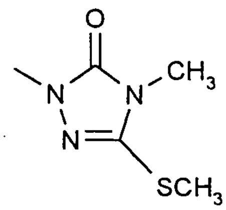 | 147 |
| 329 | - | O | $C_2H_5$ | (6-)$CF_3$ | 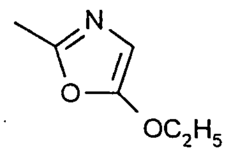 | 66 |
| 330 | - | O | $C_2H_5$ | (6-)$CH_3$ | 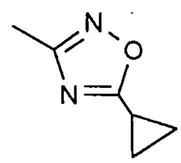 | 134 |
| 331 | - | O | $C_2H_5$ | (6-)$CH_3$ | 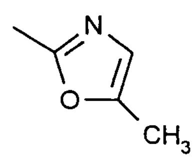 | 149 |
| 332 | - | O | n-$C_3H_7$ | (6-)$CH_3$ | 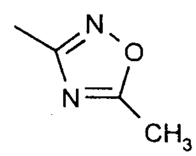 | 114 |
| 333 | - | O | H | (6-)Cl | 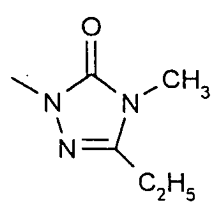 | 102 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 334 | - | O | H | (6-)Cl | 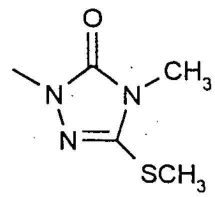 | 143 |
| 335 | - | O | 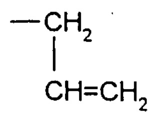 | (6-)Cl | 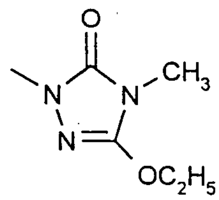 | 130 |
| 336 | - | O | $-CH_2C_6H_5$ | (6-)Cl | 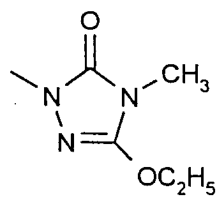 | 143 |
| 337 | - | O | $-CH_2C_6H_5$ | (6-)Cl | 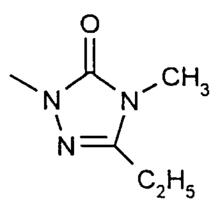 | 99 |
| 338 | - | O | $-CH_2C{\equiv}CH$ | (6-)Cl | 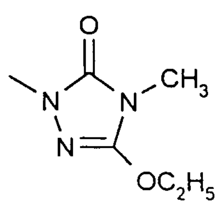 | 161 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 339 | - | O | n-$C_3H_7$ | (6-)$CH_3$ | 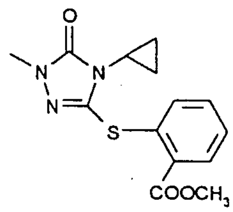 | 133 |
| 340 | - | O | H | (6-)$CH_3$ | 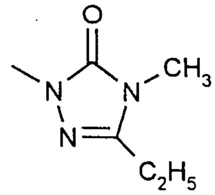 | 100 |
| 341 | - | O | H | (6-)$CH_3$ | 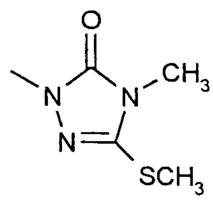 | 147 |
| 342 | - | O | -$CH_2C_6H_5$ | (6-)$CH_3$ | 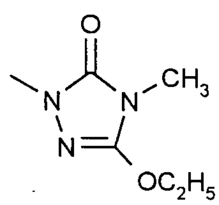 | 157 |
| 343 | - | O | 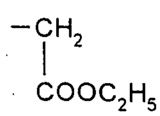 | (6-)$CH_3$ | 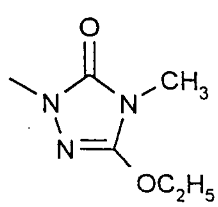 | 150 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 344 | - | O | $-CH_2C{\equiv}CH$ | (6-)$CH_3$ | 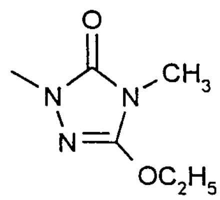 | 172 |
| 345 | - | O | i-$C_3H_7$ | (6-)$CH_3$ | 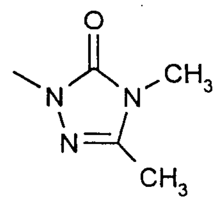 | 263 (Na-Salz) |
| 346 | - | O | $C_2H_5$ | (6-)$CH_3$ | 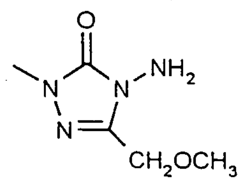 | 136 |
| 347 | - | O | i-$C_3H_7$ | (6-)$CH_3$ | 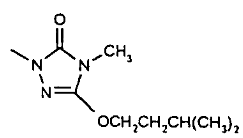 | 113 |
| 348 | - | O | i-$C_3H_7$ | (6-)$CH_3$ | 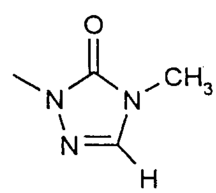 | 175 |
| 349 | - | O | i-$C_3H_7$ | (6-)$CH_3$ | 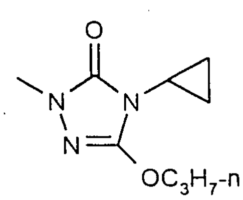 | 135 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 350 | - | O | $i\text{-}C_3H_7$ | $(6\text{-})CH_3$ | 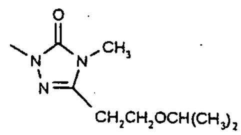 | 78 |
| 351 | - | O | $i\text{-}C_3H_7$ | $(6\text{-})CH_3$ | 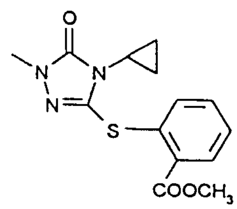 | 125 |
| 352 | - | O | $i\text{-}C_3H_7$ | $(6\text{-})CH_3$ | 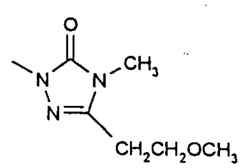 | 140 |
| 353 | - | O | $CH_3$ | $(6\text{-})CH_3$ | 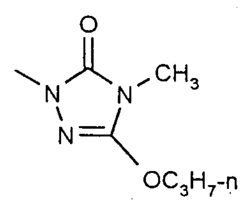 | 161 |
| 354 | - | O | $CH_3$ | $(6\text{-})CH_3$ | 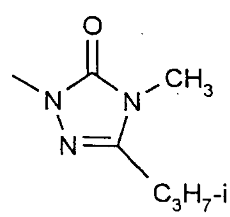 | 142 |
| 355 | - | O | $CH_3$ | $(6\text{-})CH_3$ | 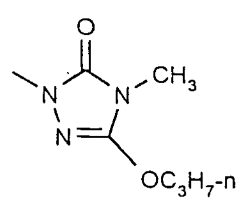 | 124 |

Tabelle 1 - Fortsetzung

| Bsp.-Nr. | A | Q | $R^1$ | (Position-) $R^2$ | $R^3$ | Schmelz-punkt (°C) |
|---|---|---|---|---|---|---|
| 356 | - | O | $CH_3$ | (6-)$CH_3$ | 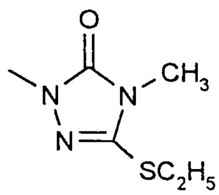 | 153 |
| 357 | - | O | $CH_3$ | (6-)$CH_3$ | 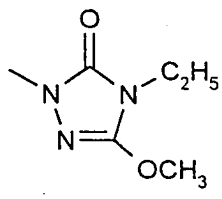 | 170 |
| 358 | - | O | $CH_3$ | (6-)$CH_3$ | 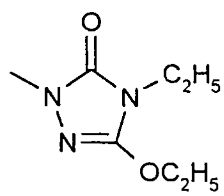 | 116 |
| 359 | - | O | $CH_3$ | (6-)$CH_3$ | 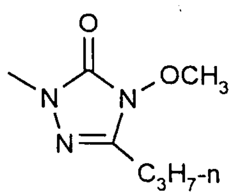 | 120 |
| 360 | - | O | $CH_3$ | (5-)Cl | 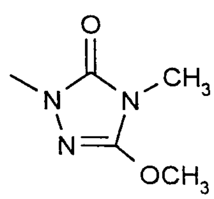 | 172 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 361 | - | O | $CH_3$ | (5-)Cl | 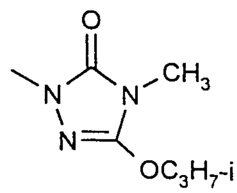 | 175 |
| 362 | - | O | $CH_3$ | (5-)Cl | 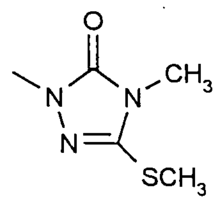 | 192 |
| 363 | - | O | $CH_3$ | (5-)Cl | 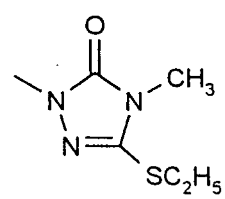 | 195 |
| 364 | - | O | $CH_3$ | (5-)Cl | 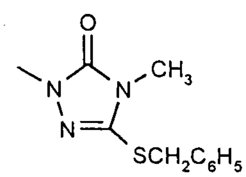 | 174 |
| 365 | - | O | $CH_3$ | (5-)Cl | 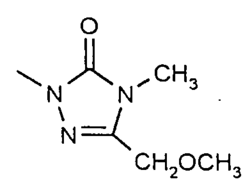 | 160 |
| 366 | - | O | $CH_3$ | (5-)Cl | 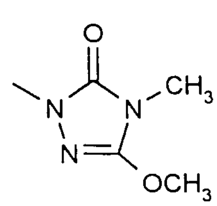 | 214 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 367 | - | O | $CH_3$ | (5-)Cl | 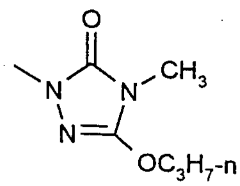 | 185 |
| 368 | - | O | $CH_3$ | (5-)Cl | 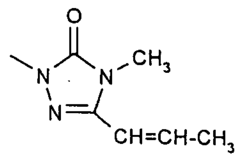 | 191 |
| 369 | NH | O | $CH_3$ | (6-)$CH_3$ | 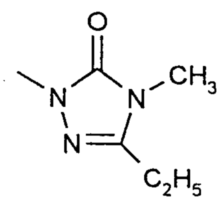 | 161 |
| 370 | NH | O | i-$C_3H_7$ | (6-)$CH_3$ | 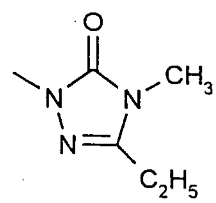 | 132 |
| 371 | NH | O | $CH_3$ | (6-)$OCH_3$ | 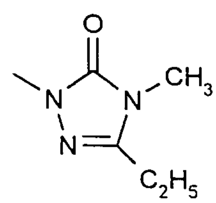 | 151 |
| 372 | NH | O | $CH_3$ | (6-)$CH_3$ | 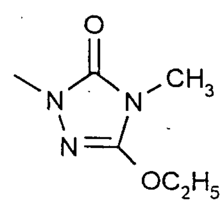 | 161 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 373 | NH | O | i-$C_3H_7$ | (6-)$CH_3$ | 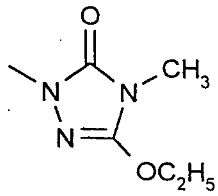 | 128 |
| 374 | NH | O | $CH_3$ | (6-)$CH_3$ | 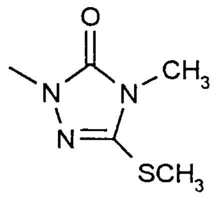 | 140 |
| 375 | - | S | i-$C_3H_7$ | (6-)$CH_3$ | 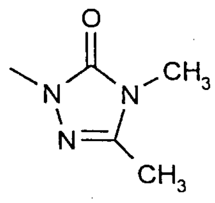 | 108 |
| 376 | - | O | $CHF_2$ | (4-)$CH_3$ | 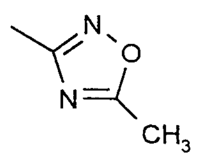 | 131 |
| 377 | - | O | $CH_3$ | (6-)$CF_3$ | 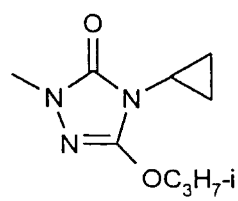 | 187 |
| 378 | - | O | $CH_3$ | (6-)$CF_3$ | 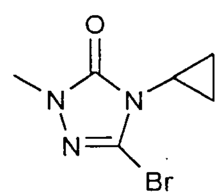 | 154 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 379 | - | O | $CH_3$ | (6-)$C_2H_5$ | 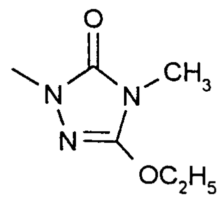 | 179 |
| 380 | - | O | $CH_3$ | (6-)$C_2H_5$ | 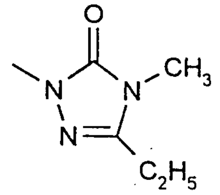 | 178 |
| 381 | - | O | $CH_3$ | (6-)$C_2H_5$ | 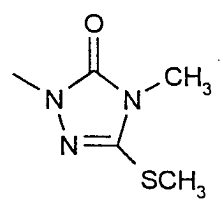 | 167 |
| 382 | - | O | $C_2H_5$ | (6-)$C_2H_5$ | 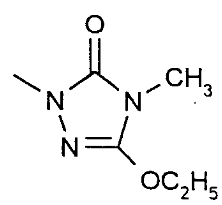 | 135 |
| 383 | - | O | $C_2H_5$ | (6-)$C_2H_5$ | 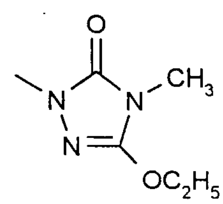 | 146 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 384 | - | O | $C_2H_5$ | (6-)$C_2H_5$ | 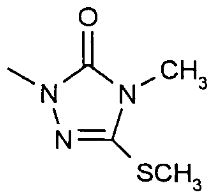 | 174 |
| 385 | - | O | $CH_3$ | (6-)$C_2H_5$ | 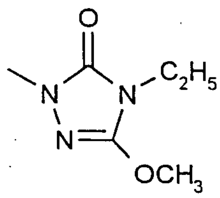 | 130 |
| 386 | - | O | $CH_3$ | (6-)$C_2H_5$ | 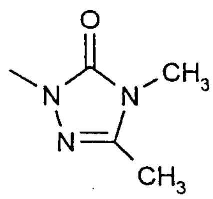 | 195 |
| 387 | - | O | $CH_3$ | (6-)$C_2H_5$ | 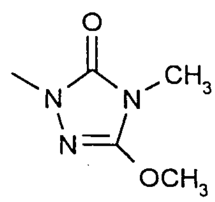 | 183 |
| 388 | - | O | $C_2H_5$ | (6-)$C_3H_7$-n | 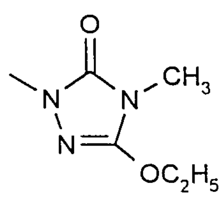 | 135 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 389 | - | O | $C_2H_5$ | (6-)$C_3H_7$-n | 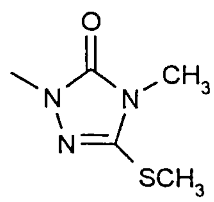 | 149 |
| 390 | - | O | $CH_3$ | (6-)$C_3H_7$-n | 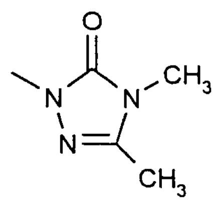 | 193 |
| 391 | - | O | $CH_3$ | (6-)$C_3H_7$-n | 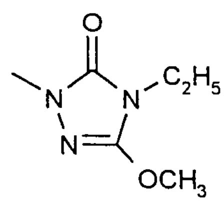 | 125 |
| 392 | - | O | $CH_3$ | (6-)$C_3H_7$-n | 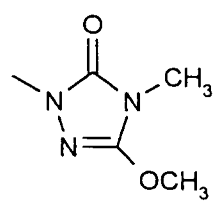 | 182 |
| 393 | - | O | $C_2H_5$ | (6-)$C_3H_7$-n | 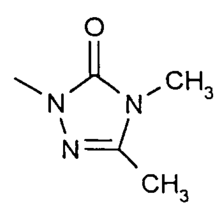 | 120 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 394 | - | O | $C_2H_5$ | (6-)$C_3H_7$-n | 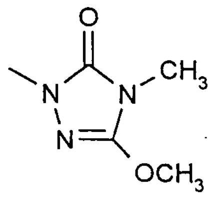 | 158 |
| 395 | - | O | i-$C_3H_7$ | (6-)$CH_3$ | 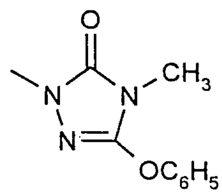 | 180 |
| 396 | - | O | n-$C_4H_9$ | (6-)$CH_3$ | 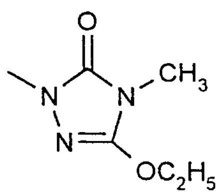 | 132 |
| 397 | - | O | n-$C_4H_9$ | (6-)$CH_3$ | 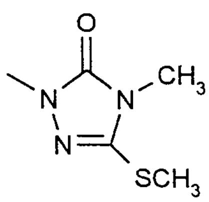 | 143 |
| 398 | - | O | n-$C_4H_9$ | (6-)$CH_3$ | 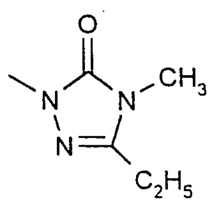 | 106 |

Tabelle 1 - Fortsetzung

| Bsp.-Nr. | A | Q | $R^1$ | (Position-) $R^2$ | $R^3$ | Schmelz-punkt (°C) |
|---|---|---|---|---|---|---|
| 399 | - | O | n-$C_4H_9$ | (6-)$CH_3$ | 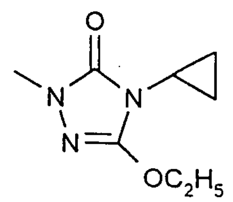 | 98 |
| 400 | - | O | n-$C_4H_9$ | (6-)$CH_3$ | 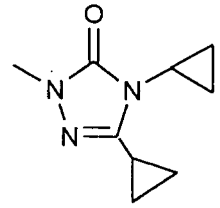 | 140 |
| 401 | - | O | $CH_3$ | (6-)$CH_3$ | 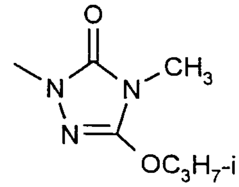 | 147 |
| 402 | - | O | $CH_3$ | (6-)$CH_3$ | 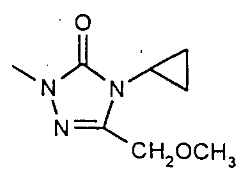 | 123 |
| 403 | - | O | $CH_3$ | (6-)$CH_3$ | 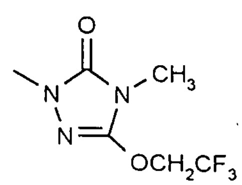 | 185 |
| 404 | - | O | $CH_3$ | (6-)$CH_3$ | 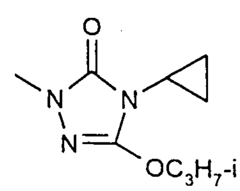 | 154 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 405 | NH | O | i-$C_3H_7$ | (6-)$CH_3$ | 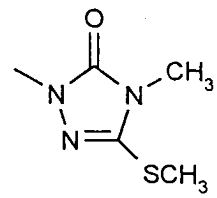 | 150 |
| 406 | - | O | $C_2H_5$ | (6-)$C_2H_5$ | 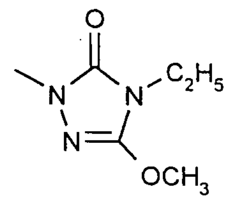 | 135 |
| 407 | - | O | $C_2H_5$ | (6-)$C_2H_5$ | 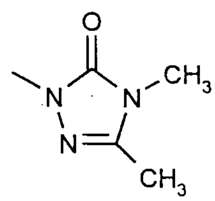 | 134 |
| 408 | - | O | $C_2H_5$ | (6-)$C_2H_5$ | 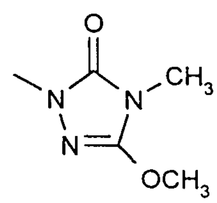 | 178 |
| 409 | - | O | $C_3H_7$-i | (6-)$C_2H_5$ | 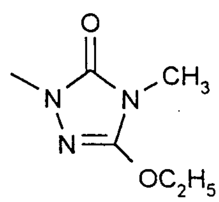 | 109 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 410 | - | O | $C_3H_7$-i | (6-)$C_2H_5$ | 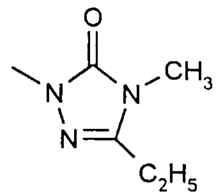 | 125 |
| 411 | - | O | $C_3H_7$-i | (6-)$C_2H_5$ | 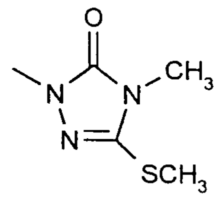 | 161 |
| 412 | - | O | $C_3H_7$-i | (6-)$C_2H_5$ | 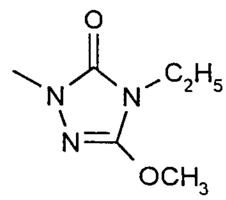 | 114 |
| 413 | - | O | $C_3H_7$-i | (6-)$C_2H_5$ | 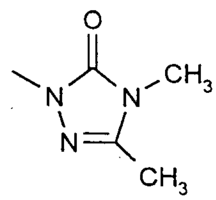 | 142 |
| 414 | - | O | $C_3H_7$-i | (6-)$C_2H_5$ | 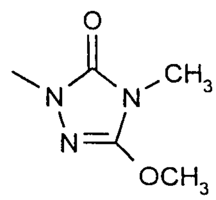 | 124 |
| 415 | - | O | $CH_3$ | (6-)$C_2H_5$ | 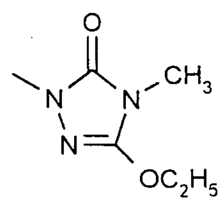 | 175 (Na-Salz) |

Tabelle 1 - Fortsetzung

| Bsp.-Nr. | A | Q | $R^1$ | (Position-) $R^2$ | $R^3$ | Schmelz-punkt (°C) |
|---|---|---|---|---|---|---|
| 416 | - | O | $C_2H_5$ | (6-)$C_3H_7$-n | 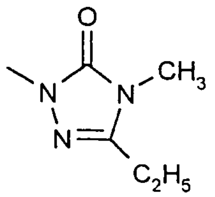 | 126 |
| 417 | - | O | $C_2H_5$ | (6-)$C_3H_7$-n | 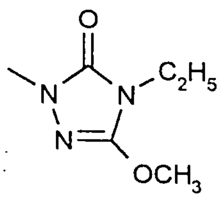 | 121 |
| 418 | - | O | $CH_3$ | (6-)$C_3H_7$-n | 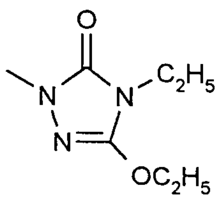 | 109 |
| 419 | - | O | $CH_3$ | (6-)$C_3H_7$-n | 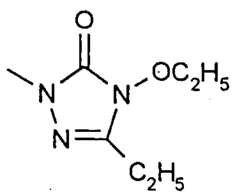 | 145 |
| 420 | - | O | $CH_3$ | (6-)$C_3H_7$-n | 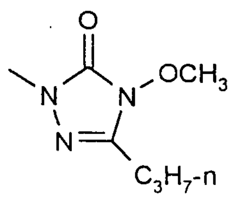 | 126 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 421 | - | O | $CH_3$ | (6-)$C_3H_7$-n | 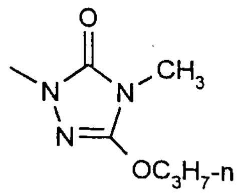 | 130 |
| 422 | - | O | $CH_3$ | (6-)$C_3H_7$-n | 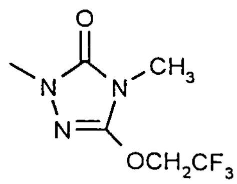 | 155 |
| 423 | - | O | $CH_3$ | (6-)$C_3H_7$-n | 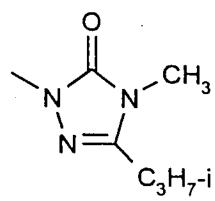 | 133 |
| 424 | - | O | $CH_3$ | (6-)$C_3H_7$-n | 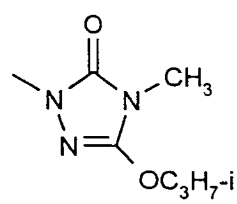 | 145 |
| 425 | - | O | $-SO_2CH_3$ | (6-)$CH_3$ | 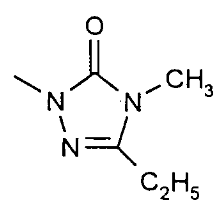 | 95 |
| 426 | - | O | $-SO_2CH_3$ | (6-)$CH_3$ | 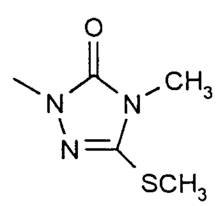 | 153 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 427 | - | O | $C_4H_9$-n | (6-)$CH_3$ | 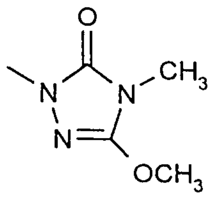 | 154 |
| 428 | - | O | $-CH_2C\equiv CH$ | (6-)$CH_3$ | 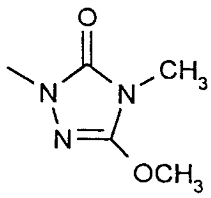 | 167 |
| 429 | - | O | $-CH_2C\equiv CH$ | (6-)$CH_3$ | 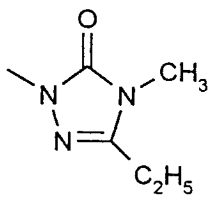 | 170 |
| 430 | - | O | $-CH_2C\equiv CH$ | (6-)$CH_3$ | 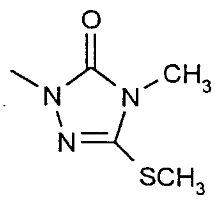 | 153 |
| 431 | - | O | $C_4H_9$-i | (6-)$CH_3$ | 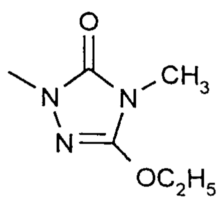 | 123 |
| 432 | - | O | $C_4H_9$-i | (6-)$CH_3$ | 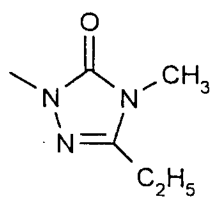 | 145 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 433 | - | O | $C_4H_9$-i | (6-)$CH_3$ | 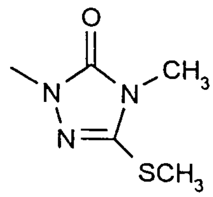 | 143 |
| 434 | - | O | $C_3H_7$-i | (6-)$CH_3$ | 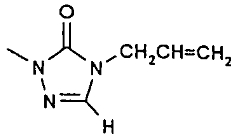 | 138 |
| 435 | - | O | $C_3H_7$-i | (6-)$CH_3$ | 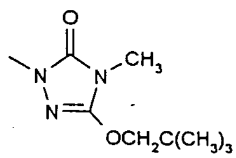 | 161 |
| 436 | - | O | $C_3H_7$-i | (6-)$CH_3$ | 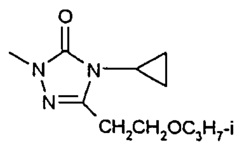 | 128 |
| 437 | - | O | $C_3H_7$-i | (6-)$CH_3$ | 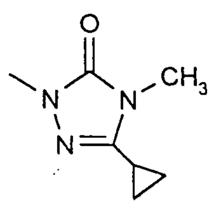 | 177 |
| 438 | - | O | $C_3H_7$-i | (6-)$CH_3$ | 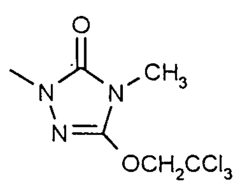 | 165 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 439 | - | O | $CH_3$ | (6-)$CH_3$ | 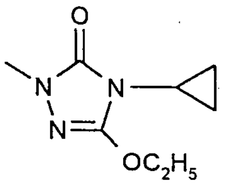 | 157 |
| 440 | - | O | $CH_3$ | (6-)$CH_3$ | 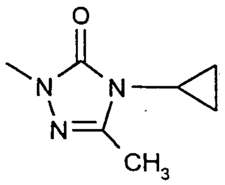 | 168 |
| 441 | - | O | $CH_3$ | (6-)$CH_3$ | 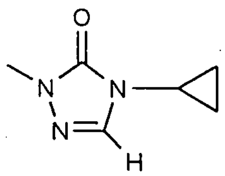 | 164 |
| 442 | - | O | $CH_3$ | (6-)$CH_3$ | 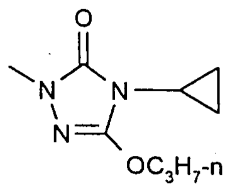 | 125 |
| 443 | - | O | $CH_3$ | (6-)$CH_3$ | 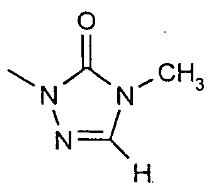 | 162 |
| 444 | - | O | $CH_3$ | (6-)$OCH_3$ | 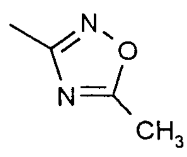 | 154 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 445 | - | S | $CH_3$ | (4-)$C_3H_7$-i | 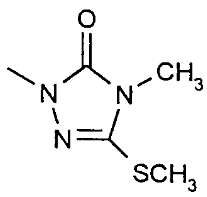 | 116 |
| 446 | - | S | $CH_3$ | (4-)$C_3H_7$-i | 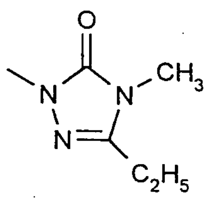 | 110 |
| 447 | - | S | $CH_3$ | (4-)$C_3H_7$-i | 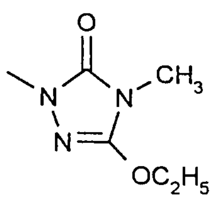 | 134 |
| 448 | - | O | $CH_3$ | (4-)$C_3H_7$-i | 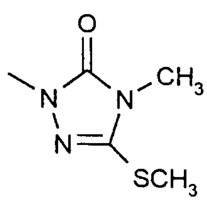 | 152 |
| 449 | - | O | $CH_3$ | (4-)$C_3H_7$-i | 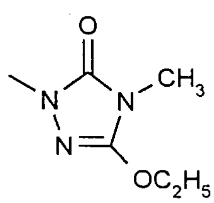 | 159 |
| 450 | - | O | $CH_3$ | (4-)$C_3H_7$-i | 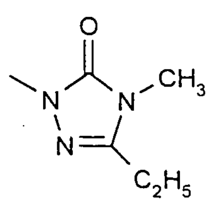 | 150 |

Tabelle 1 - Fortsetzung

| Bsp. Nr. | A | Q | $R^1$ | (Position-) $R^2$ | $R^3$ | Schmelz-punkt (°C) |
|---|---|---|---|---|---|---|
| 451 | - | O | $C_2H_5$ | (6-)$OCH_3$ | 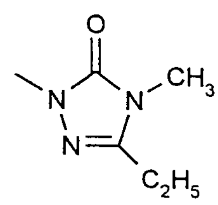 | 107 |
| 452 | - | O | $C_2H_5$ | (6-)$OCH_3$ | 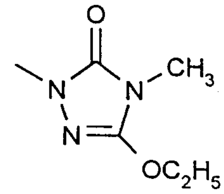 | 104 |
| 453 | - | O | $C_2H_5$ | (6-)$OCH_3$ | 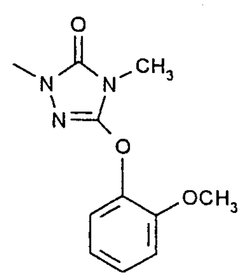 | 100 |
| 454 | - | S | $C_2H_5$ | (6-)$OCH_3$ | 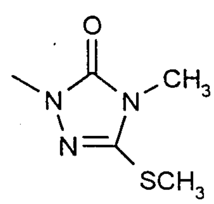 | 103 |
| 455 | - | S | $C_2H_5$ | (6-)$OCH_3$ | 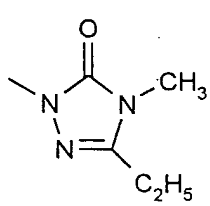 | 95 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 456 | - | S | $C_2H_5$ | (6-)$OCH_3$ | 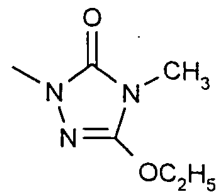 | 105 |
| 457 | - | O | $C_2H_5$ | (6-)$OC_2H_5$ | 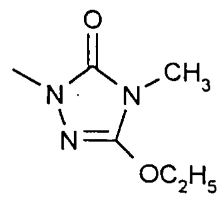 | 130 |
| 458 | - | O | $C_2H_5$ | (6-)$OC_2H_5$ | 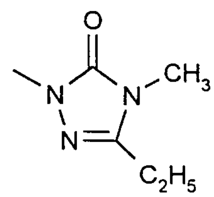 | 100 |
| 459 | - | O | $C_3H_7$-i | (6-)$OC_3H_7$-i | 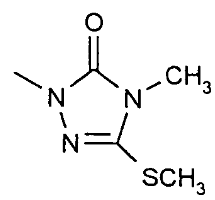 | 114 |
| 460 | - | O | $C_3H_7$-i | (6-)$OC_3H_7$-i | 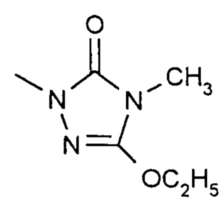 | 125 |
| 461 | - | S | $C_3H_7$-i | (6-)$OC_3H_7$-i | 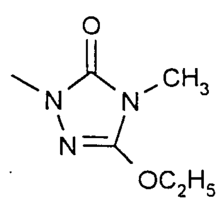 | 143 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 462 | - | O | $C_2H_5$ | (6-)$OC_2H_5$ | 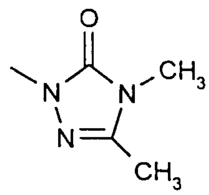 | 120 |
| 463 | - | O | $-CF_2CHFCl$ | (6-)$CH_3$ | 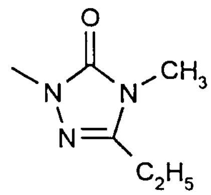 | 124 |
| 464 | - | O | $-CF_2CHFCl$ | (6-)$CH_3$ | 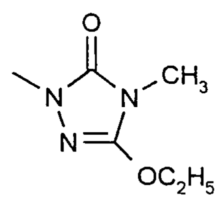 | 115 |
| 465 | - | O | $-CF_2CHFCl$ | (6-)$CH_3$ | 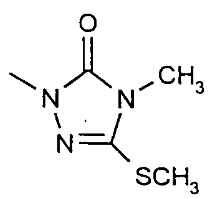 | 150 |
| 466 | - | O | $CH_3$ | (3-)$CH_3$ | 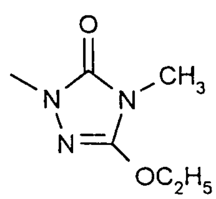 | 178 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 467 | - | O | $CH_3$ | (3-)Cl | 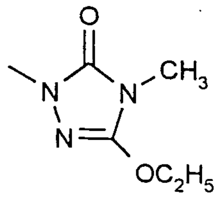 | 188 |
| 468 | - | O | $C_2H_5$ | (3-)Cl | 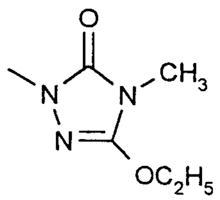 | 159 |
| 469 | - | O | $CH_3$ | (3-)F | 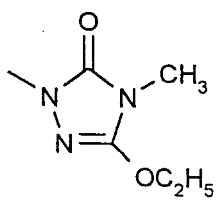 | 176 |
| 470 | - | O | $C_2H_5$ | (6-)$CF_3$ | 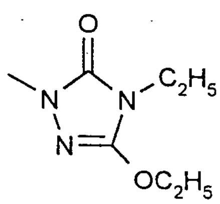 | 124 |
| 471 | - | O | $C_2H_5$ | (6-)$CF_3$ | 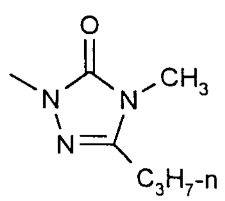 | 34 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 472 | - | O | $C_2H_5$ | (6-)$CF_3$ | 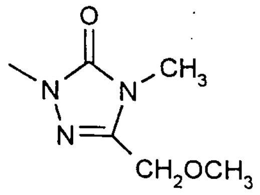 | 68 |
| 473 | - | O | $C_3H_7$-i | (6-)$CF_3$ | 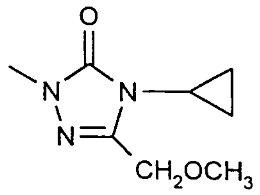 | 41 |
| 474 | - | O | $C_2H_5$ | (6-)$CF_3$ | 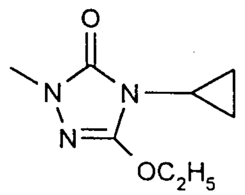 | 127 |
| 475 | - | O | H | (6-)$CF_3$ | 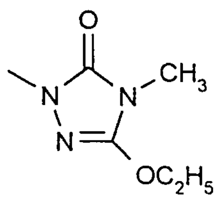 | 125 |
| 476 | - | S | $C_2H_5$ | (6-)$CH_3$ | 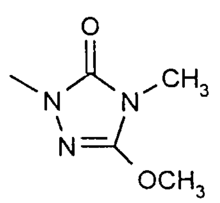 | 214 (Na-Salz) |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 477 | NH | O | $C_2H_5$ | (6-)$CH_3$ | 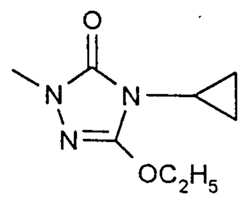 | 128 |
| 478 | NH | O | $C_2H_5$ | (6-)$CH_3$ | 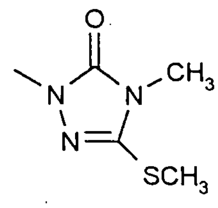 | 148 |
| 479 | NH | O | $C_3H_7$-n | (6-)$CH_3$ | 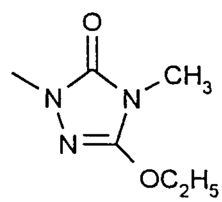 | 127 |
| 480 | NH | O | $C_3H_7$-n | (6-)$CH_3$ | 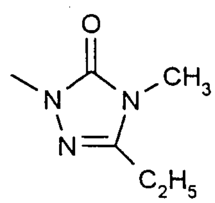 | 57 |
| 481 | NH | O | $C_2H_5$ | (6-)$CH_3$ | 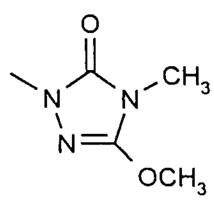 | 125 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 482 | NH | O | $C_3H_7$-n | (6-)$CH_3$ | 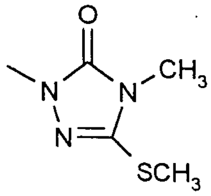 | 115 |
| 483 | NH | O | $C_3H_7$-n | (6-)$CH_3$ | 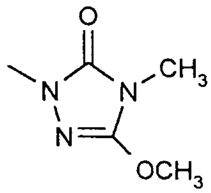 | 151 |
| 484 | NH | O | $C_2H_5$ | (5-)$CH_3$ | 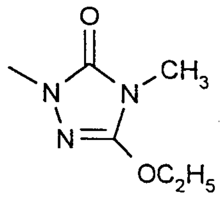 | 132 |
| 485 | NH | O | $C_2H_5$ | (5-)$CH_3$ | 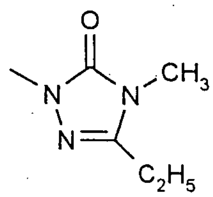 | 106 |
| 486 | NH | O | $C_2H_5$ | (5-)$CH_3$ | 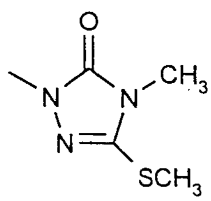 | 163 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 487 | NH | O | $C_2H_5$ | (5-)$CH_3$ | 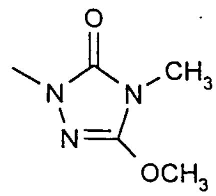 | 137 |
| 488 | - | O | $C_2H_5$ | (6-)$C_3H_7$-i | 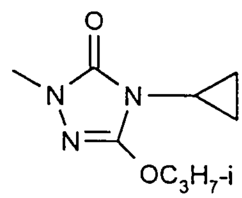 | 166 |
| 489 | - | O | $C_2H_5$ | (6-)$C_3H_7$-i | 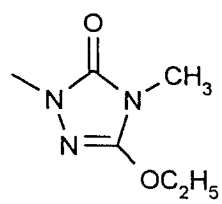 | 169 |
| 490 | - | O | $C_2H_5$ | (6-)$C_3H_7$-i | 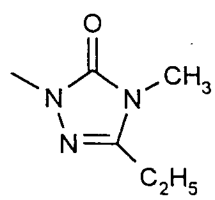 | 130 |
| 491 | NH | O | 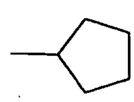 | (6-)$CH_3$ | 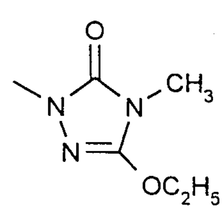 | 148 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 492 | NH | O | 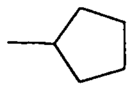 | (6-)$CH_3$ | 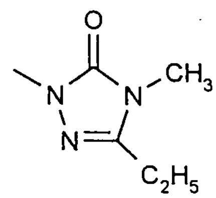 | 138 |
| 493 | NH | O | 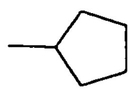 | (6-)$CH_3$ | 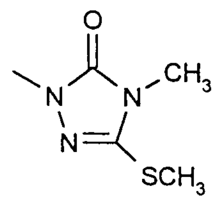 | 147 |
| 494 | - | O | 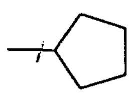 | (6-)$CH_3$ | 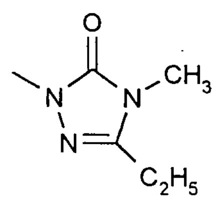 | 124 |
| 495 | - | O | 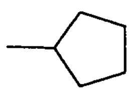 | (6-)$CH_3$ | 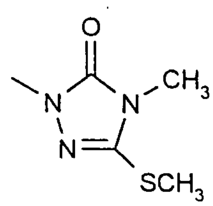 | 152 |
| 496 | - | O | 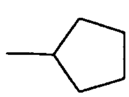 | (6-)$CH_3$ | 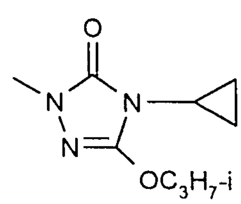 | 141 |

Tabelle 1 - Fortsetzung

| Bsp.-Nr. | A | Q | $R^1$ | (Position-) $R^2$ | $R^3$ | Schmelz-punkt (°C) |
|---|---|---|---|---|---|---|
| 497 | - | O | 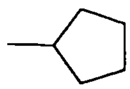 | (6-)$CH_3$ | 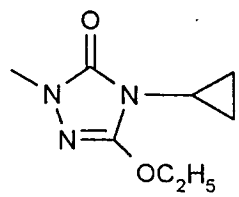 | 127 |
| 498 | - | O | 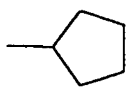 | (6-)$CH_3$ | 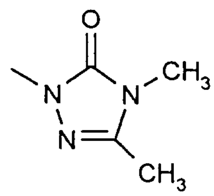 | 144 |
| 499 | - | O | 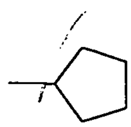 | (6-)$CH_3$ | 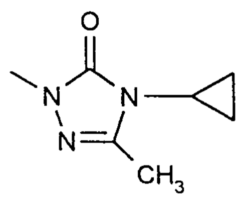 | 107 |
| 500 | - | O | $C_3H_7$-n | (6-)$CH_3$ | 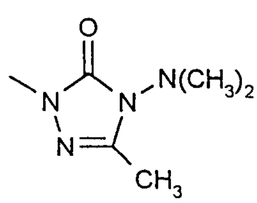 | 265 (Na-Salz) |
| 501 | - | O | $C_2H_5$ | (6-)$CH_3$ | 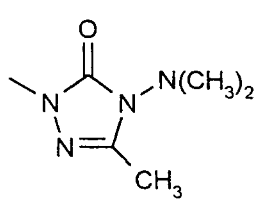 | 269 (Na-Salz) |

<u>Tabelle 1</u> - Fortsetzung

| Bsp.-Nr. | A | Q | $R^1$ | (Position-) $R^2$ | $R^3$ | Schmelzpunkt (°C) |
|---|---|---|---|---|---|---|
| 502 | - | O | $C_3H_7$-i | (6-)$CH_3$ | 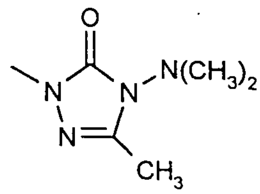 | 237 (Na-Salz) |
| 503 | - | O | 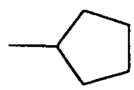 | (6-)$CH_3$ | 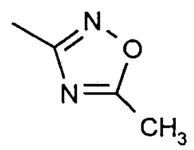 | 73 |
| 504 | - | O | $C_3H_7$-i | (6-)$CH_3$ | 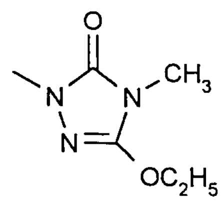 | 220 (Na-Salz) |
| 505 | - | O | $C_3H_7$-i | (6-)$CH_3$ | 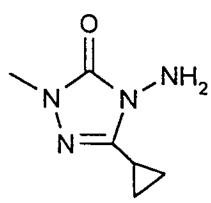 | 140 |
| 506 | - | O | $C_4H_9$-s | (6-)$CH_3$ | 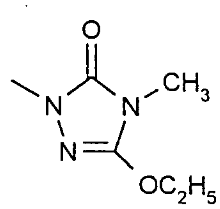 | 120 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 507 | - | O | $C_4H_9$-s | (6-)$CH_3$ | 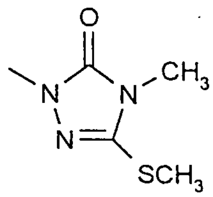 | 117 |
| 508 | - | O | $C_4H_9$-s | (6-)$CH_3$ | 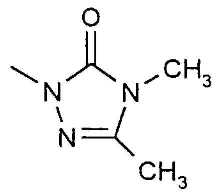 | 128 |
| 509 | - | O | $C_4H_9$-s | (6-)$CH_3$ | 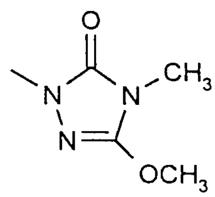 | 232 |
| 510 | - | O | $C_4H_9$-s | (6-)$CH_3$ | 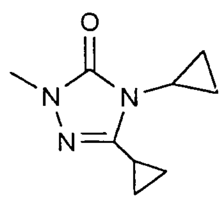 | 268 |
| 511 | - | O | $C_4H_9$-s | (6-)$CH_3$ | 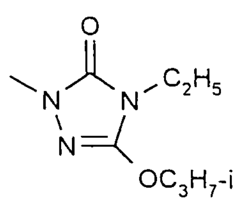 | 130 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 512 | - | O | $C_4H_9$-s | (6-)$CH_3$ | 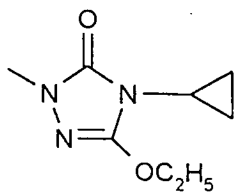 | 137 |
| 513 | - | O | $C_4H_9$-s | (6-)$CH_3$ | 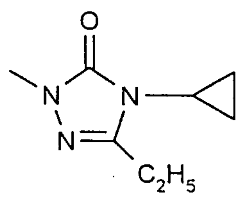 | 145 |
| 514 | - | O | $C_4H_9$-s | (6-)$CH_3$ | 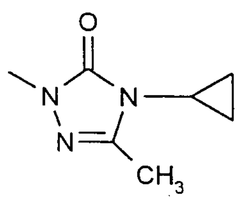 | 164 |
| 515 | - | O | $C_4H_9$-s | (6-)$CH_3$ | 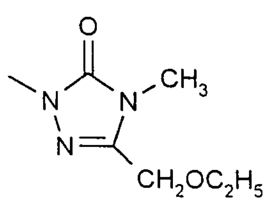 | 89 |
| 516 | - | O | $C_4H_9$-s | (6-)$CH_3$ | 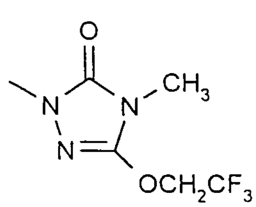 | 86 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 517 | - | O | $C_4H_9$-s | (6-)$CH_3$ | 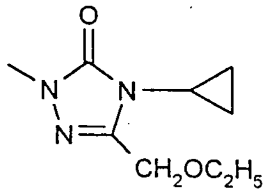 | 98 |
| 518 | - | O | $C_4H_9$-s | (6-)$CH_3$ | 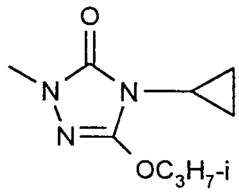 | 122 |
| 519 | - | O | $C_4H_9$-s | (6-)$CH_3$ | 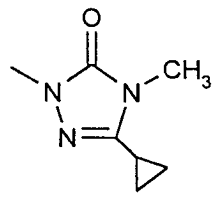 | 135 |
| 520 | - | O | $C_2H_5$ | (6-)$CH_3$ | 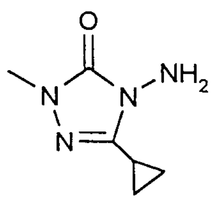 | 142 |
| 521 | - | O | $CH_3$ | (6-)$CH_3$ | 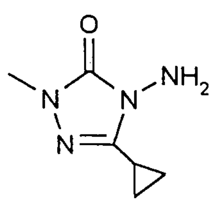 | 157 |

Tabelle 1 - Fortsetzung

| Bsp.-Nr. | A | Q | $R^1$ | (Position-) $R^2$ | $R^3$ | Schmelz-punkt (°C) |
|---|---|---|---|---|---|---|
| 522 | - | O | $C_3H_7$-n | (6-)$CH_3$ | 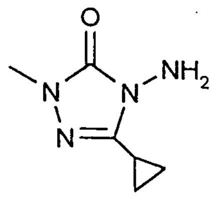 | 126 |
| 523 | - | O | $C_4H_9$-s | (6-)$CH_3$ | 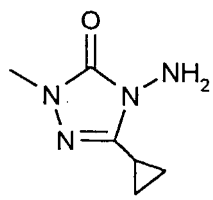 | 140 |
| 524 | - | O | $C_2H_5$ | (6-)$CH_3$ | 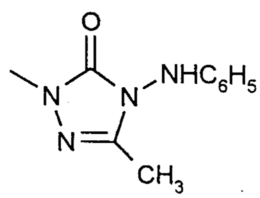 | 164 |
| 525 | - | O | $CH_3$ | (6-)$CH_3$ | 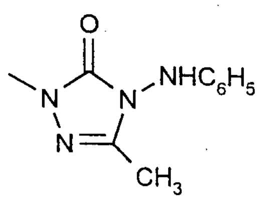 | 166 |
| 526 | - | O | $C_3H_7$-n | (6-)$CH_3$ | 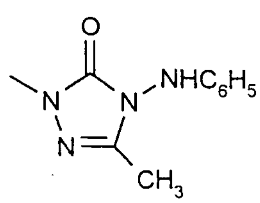 | 145 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 527 | - | O | $CH_3$ | (6-)$CH_3$ | 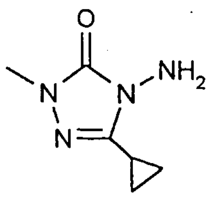 | 239 (Na-Salz) |
| 528 | - | O | $C_3H_7$-n | (6-)$CH_3$ | 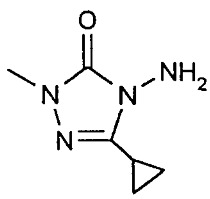 | 206 (Na-Salz) |
| 529 | - | O | $C_2H_5$ | (6-)$CH_3$ | 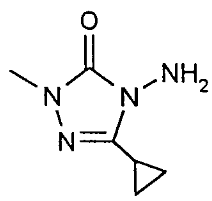 | 211 (Na-Salz) |
| 530 | - | O | $CH_3$ | (6-)$C_3H_7$-i | 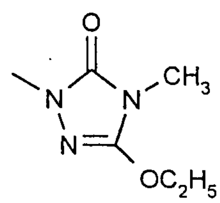 | 166 |
| 531 | - | O | $CH_3$ | (6-)$C_3H_7$-i | 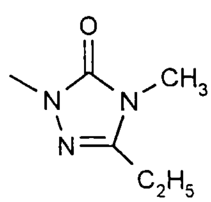 | 178 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 532 | - | O | $C_3H_7$-i | (6-)$C_3H_7$-i | 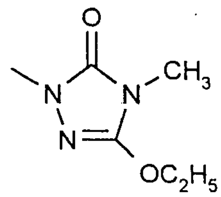 | 131 |
| 533 | - | O | $C_3H_7$-i | (6-)$C_3H_7$-i | 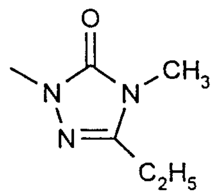 | 130 |
| 534 | - | O | $C_3H_7$-i | (6-)$C_3H_7$-i | 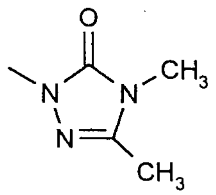 | 133 |
| 535 | - | O | $C_3H_7$-i | (6-)$C_3H_7$-i | 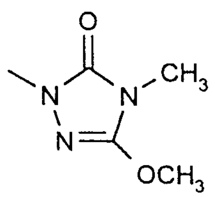 | 116 |
| 536 | - | O | $CH_3$ | (6-)$C_3H_7$-i | 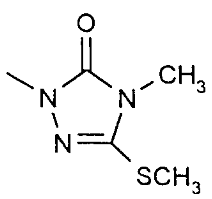 | 192 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 537 | - | O | $CH_3$ | (6-)$C_3H_7$-i | 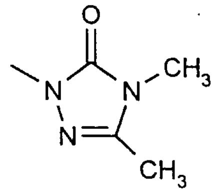 | 200 |
| 538 | - | O | $CH_3$ | (6-)$C_3H_7$-i | 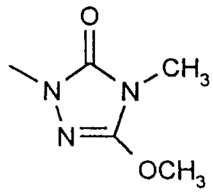 | 200 |
| 539 | - | O | $CH_3$ | (6-)$C_3H_7$-i | 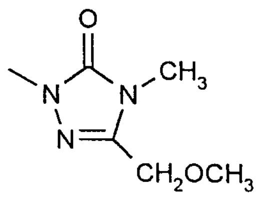 | 105 |
| 540 | - | O | $CH_3$ | (6-)$C_3H_7$-i | 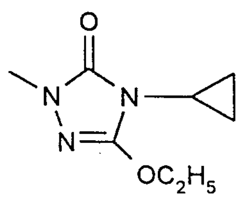 | 154 |
| 541 | - | O | $CH_3$ | (6-)$C_3H_7$-i | 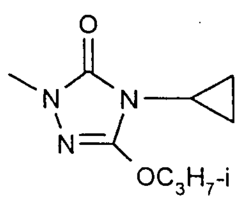 | 152 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 542 | - | O | $C_3H_7$-n | (6-)$C_3H_7$-i | 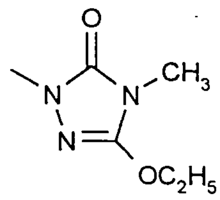 | 132 |
| 543 | - | O | $C_3H_7$-n | (6-)$C_3H_7$-i | 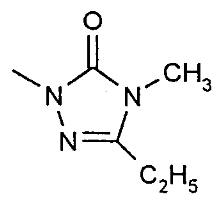 | 129 |
| 544 | - | O | $C_3H_7$-n | (6-)$C_3H_7$-i | 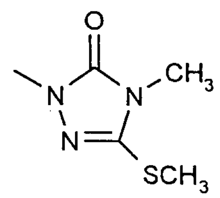 | 179 |
| 545 | - | O | $CH_3$ | (6-)$C_3H_7$-i | 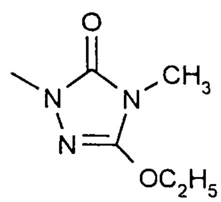 | 174 (Na-Salz) |
| 546 | - | O | $C_3H_7$-n | (6-)$C_3H_7$-i | 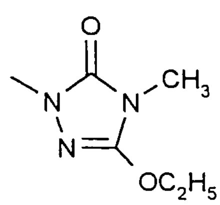 | 158 (Na-Salz) |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 547 | - | O | $C_2H_5$ | (6-)$C_3H_7$-i | 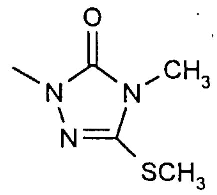 | 200 |
| 548 | - | O | $C_2H_5$ | (6-)$C_3H_7$-i | 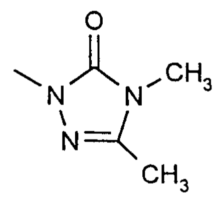 | 147 |
| 549 | - | O | $C_2H_5$ | (6-)$C_3H_7$-i | 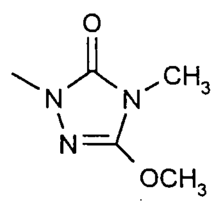 | 149 |
| 550 | - | O | $C_2H_5$ | (6-)$C_3H_7$-i | 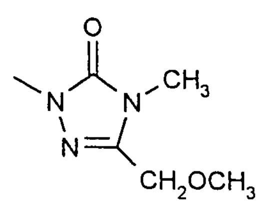 | 136 |
| 551 | - | O | $C_2H_5$ | (6-)$C_3H_7$-i | 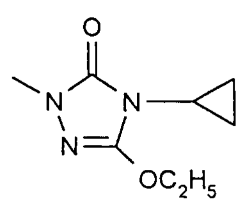 | 134 |

Tabelle 1 - Fortsetzung

| Bsp.-Nr. | A | Q | $R^1$ | (Position-) $R^2$ | $R^3$ | Schmelzpunkt (°C) |
|---|---|---|---|---|---|---|
| 552 | - | O | $C_3H_7$-i | (6-)$C_3H_7$-i | 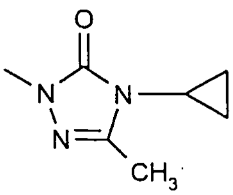 | 175 |
| 553 | - | O | $C_3H_7$-i | (6-)$C_3H_7$-i | 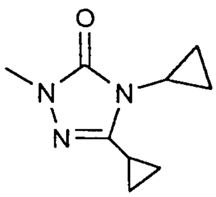 | 147 |
| 554 | - | O | $C_3H_7$-i | (6-)$C_3H_7$-i | 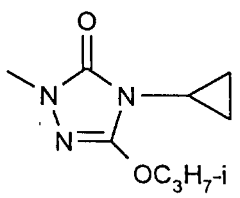 | 167 |
| 555 | - | O | $C_3H_7$-i | (6-)$C_3H_7$-i | 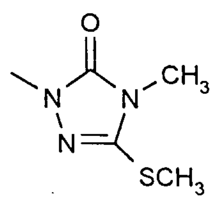 | 173 |
| 556 | - | O | $C_3H_7$-i | (6-)$CH_3$ | 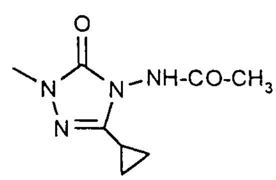 | 188 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 557 | NH | O | $CH_3$ | (6-)$CH_3$ | 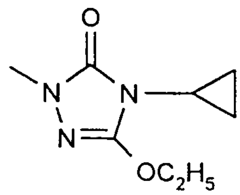 | 181 |
| 558 | NH | O | $C_3H_7$-i | (6-)$CH_3$ | 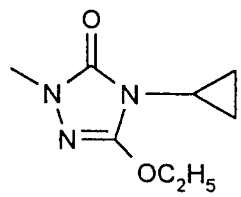 | 136 |
| 559 | - | O | $CH_3$ | (6-)$CH_3$ | 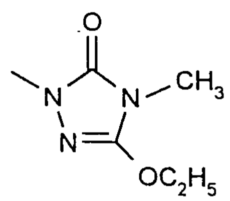 | 241 (Na-Salz) |
| 560 | NH | O | $C_3H_7$-n | (6-)$CH_3$ | 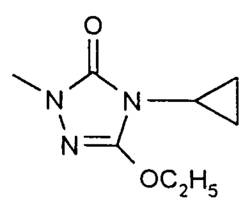 | 129 |
| 561 | - | O | $C_3H_7$-i | (6-)$OCH_3$ | 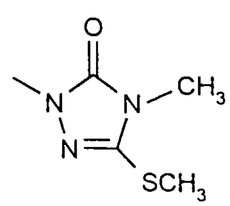 | 94 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 562 | - | O | $C_3H_7$-i | (6-)$OCH_3$ | 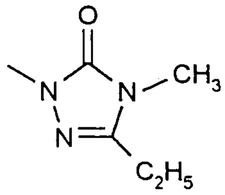 | 80 |
| 563 | - | O | $C_3H_7$-i | (6-)$OC_2H_5$ | 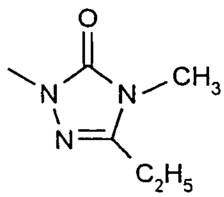 | 68 |
| 564 | - | O | $C_3H_7$-i | (6-)$OC_2H_5$ | 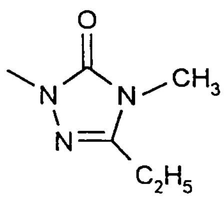 | 91 |
| 565 | - | O | $C_3H_7$-n | (6-)$OCH_3$ | 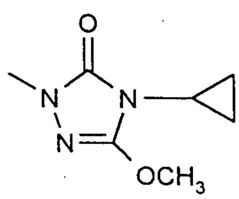 | 123 |
| 566 | - | O | $C_3H_7$-n | (6-)$OCH_3$ | 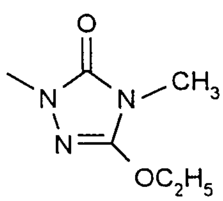 | 104 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 567 | - | O | $C_3H_7$-n | (6-)$OCH_3$ | 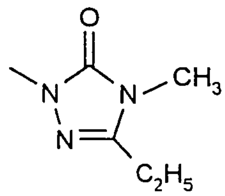 | 90 |
| 568 | - | S | $C_3H_7$-n | (6-)$OCH_3$ | 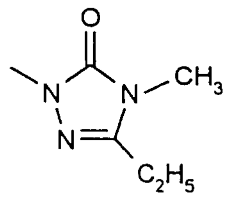 | 107 |
| 569 | - | O | $C_3H_7$-n | (6-)$OCH_3$ | 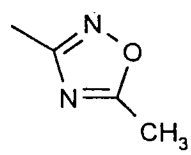 | 70 |
| 570 | - | O | $C_2H_5$ | (6-)$OC_2H_5$ | 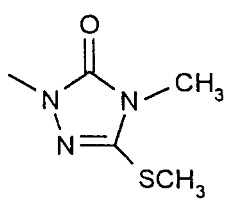 | 132 |
| 571 | - | S | $CH_3$ | (6-)$OCH_3$ | 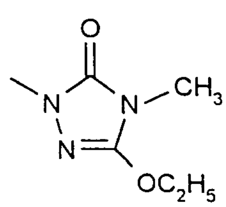 | 150 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 572 | - | O | $C_2H_5$ | (6-)$OCH_3$ | 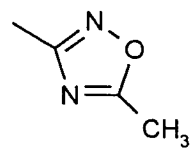 | 127 |
| 573 | - | O | $C_3H_7$-i | (6-)$OC_3H_7$-i | 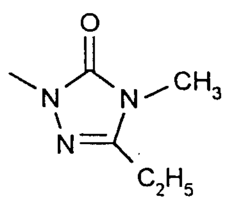 | 110 |
| 574 | - | S | $C_3H_7$-n | (6-)$OC_3H_7$-n | 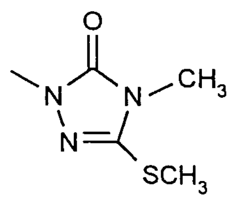 | 130 |
| 575 | - | S | $C_3H_7$-n | (6-)$OC_3H_7$-n | 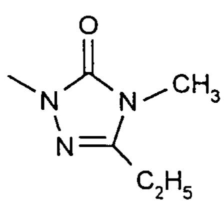 | 135 |
| 576 | - | S | $C_3H_7$-n | (6-)$OC_3H_7$-n | 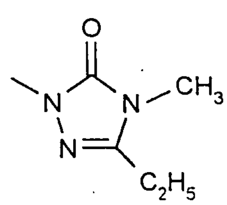 | 199 (Na-Salz) |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 577 | - | O | $C_2H_5$ | (6-)$OC_2H_5$ | 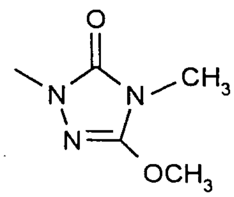 | 173 |
| 578 | - | O | $C_2H_5$ | (6-)$OCH_3$ | 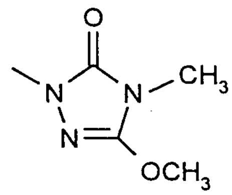 | 168 |
| 579 | - | O | $C_3H_7$-n | (6-)$OC_3H_7$-n | 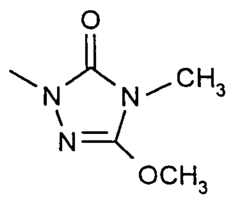 | 125 |
| 580 | - | O | $C_3H_7$-n | (6-)$OC_2H_5$ | 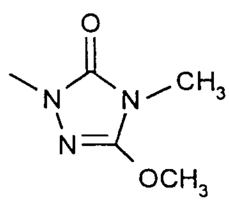 | 140 |
| 581 | - | O | $C_3H_7$-n | (6-)$OCH_3$ | 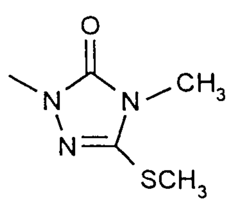 | 115 |

Tabelle 1 - Fortsetzung

| Bsp.-Nr. | A | Q | $R^1$ | (Position-) $R^2$ | $R^3$ | Schmelz-punkt |
|---|---|---|---|---|---|---|
| 582 | - | S | $C_3H_7$-n | (6-)$OCH_3$ | 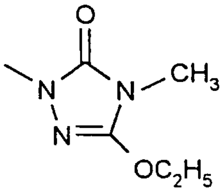 | 111 |
| 583 | - | S | $C_3H_7$-n | (6-)$OCH_3$ | 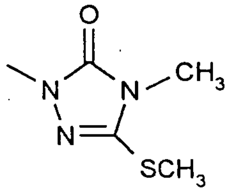 | 138 |
| 584 | - | O | $C_3H_7$-i | (6-)$OCH_3$ | 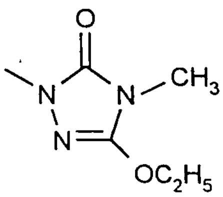 | 127 |
| 585 | - | O | $C_3H_7$-i | (6-)$OC_2H_5$ | 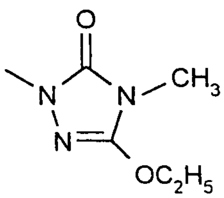 | 142 |
| 586 | - | O | $C_3H_7$-i | (6-)$OC_2H_5$ | 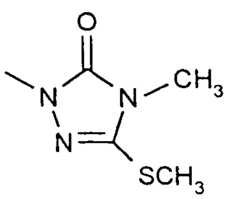 | 143 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 587 | - | O | $C_2H_5$ | (6-)$OCH_3$ | 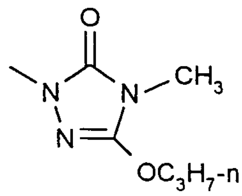 | 104 |
| 588 | - | O | $C_2H_5$ | (6-)$OCH_3$ | 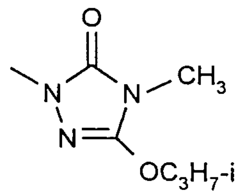 | 118 |
| 589 | - | O | $C_2H_5$ | (6-)$OCH_3$ | 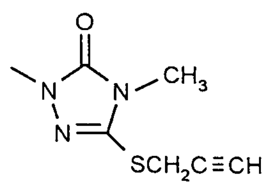 | 70 |
| 590 | - | O | $C_2H_5$ | (6-)$OCH_3$ | 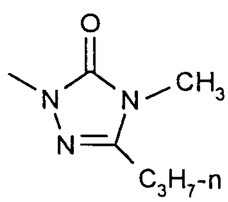 | 110 |
| 591 | - | O | $C_2H_5$ | (6-)$OCH_3$ | 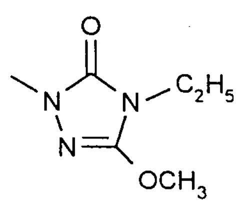 | 156 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 592 | - | O | $C_3H_7$-n | (6-)$OCH_3$ | 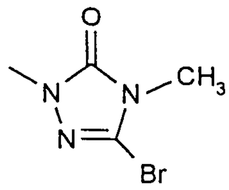 | 140 |
| 593 | - | O | $C_3H_7$-n | (6-)$OCH_3$ | 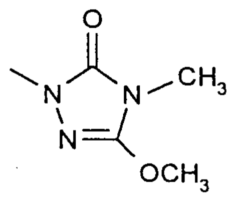 | 148 |
| 594 | - | O | $C_3H_7$-n | (6-)$OCH_3$ | 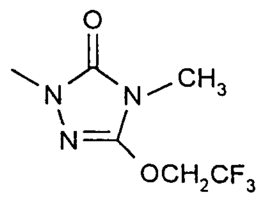 | 145 |
| 595 | - | O | $C_2H_5$ | (6-)$OCH_3$ | 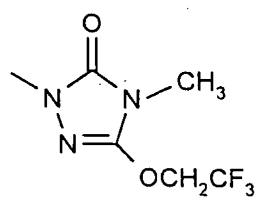 | 120 |
| 596 | - | O | $C_2H_5$ | (6-)$OCH_3$ | 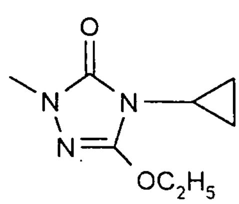 | 100 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 597 | - | O | $C_2H_5$ | (6-)$OCH_3$ | 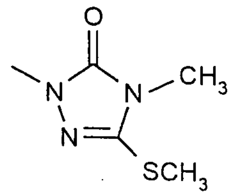 | 130 |
| 598 | - | O | $C_2H_5$ | (6-)$OCH_3$ | 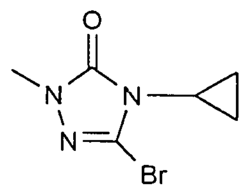 | 103 |
| 599 | - | O | $C_2H_5$ | (6-)$OCH_3$ | 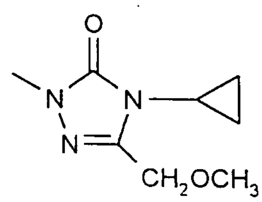 | 104 |
| 600 | - | O | $C_3H_7$-n | (6-)$OCH_3$ | 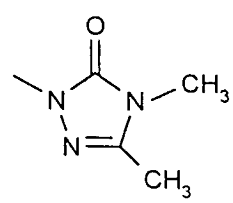 | 185 |
| 601 | - | O | $C_3H_7$-n | (6-)$OCH_3$ | 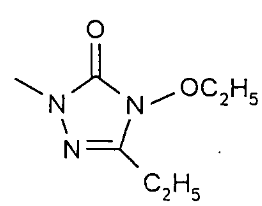 | 100 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 602 | - | O | $C_3H_7$-n | (6-)$OCH_3$ | 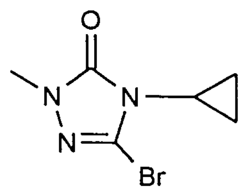 | 138 |
| 603 | - | O | $C_3H_7$-n | (6-)$OCH_3$ | 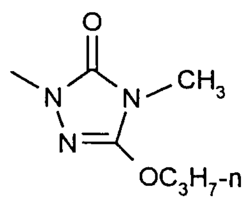 | 106 |
| 604 | - | O | $C_3H_7$-n | (6-)$OCH_3$ | 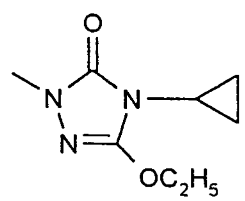 | 112 |
| 605 | - | O | $C_3H_7$-n | (6-)$OCH_3$ | 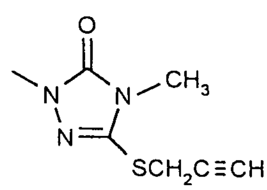 | 140 |
| 606 | - | O | $C_3H_7$-n | (6-)$OCH_3$ | 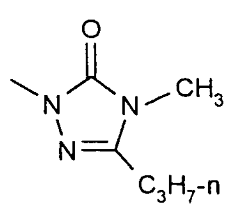 | 160 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 607 | - | O | $C_3H_7$-n | (6-)$OCH_3$ | 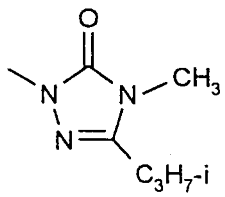 | 180 |
| 608 | - | O | $C_3H_7$-n | (6-)$OCH_3$ | 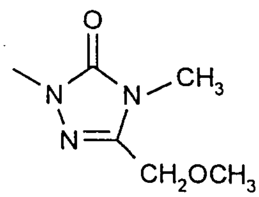 | 142 |
| 609 | - | O | $C_3H_7$-n | (6-)$OCH_3$ | 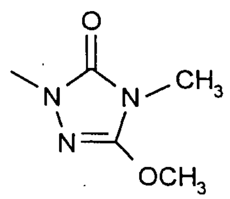 | 158 |
| 610 | - | O | $C_3H_7$-n | (6-)$OCH_3$ | 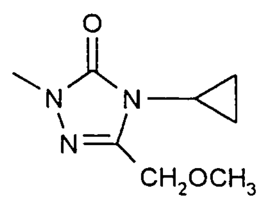 | 134 |
| 611 | - | O | $C_3H_7$-i | (6-)$OCH_3$ | 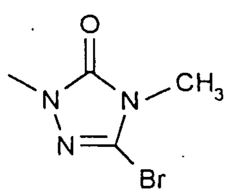 | 140 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 612 | - | O | $C_3H_7$-i | (6-)$OCH_3$ | 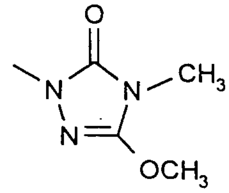 | 142 |
| 613 | - | O | $C_3H_7$-i | (6-)$OCH_3$ | 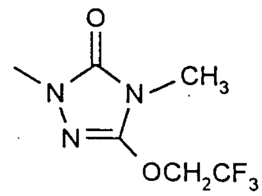 | 148 |
| 614 | - | O | $C_3H_7$-i | (6-)$OCH_3$ | 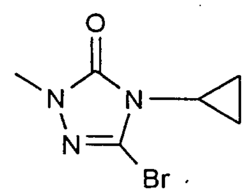 | 146 |
| 615 | - | O | $C_3H_7$-i | (6-)$OCH_3$ | 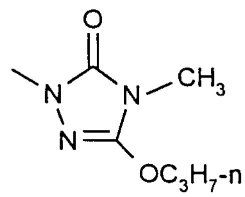 | 104 |
| 616 | - | O | $C_2H_5$ | (6-)$OC_2H_5$ | 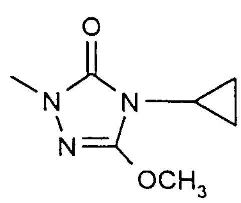 | 123 |

Tabelle 1 - Fortsetzung

| Bsp.-Nr. | A | Q | $R^1$ | (Position-) $R^2$ | $R^3$ | Schmelz-punkt |
|---|---|---|---|---|---|---|
| 617 | - | O | $C_3H_7$-n | (6-)$OC_3H_7$-n | 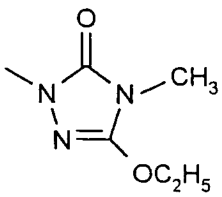 | 123 |
| 618 | - | O | $C_3H_7$-n | (6-)$OC_3H_7$-n | 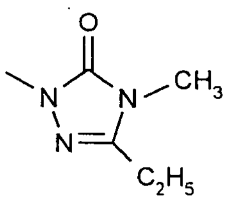 | 82 |
| 619 | - | O | $C_3H_7$-n | (6-)$OC_3H_7$-n | 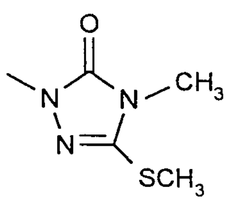 | 81 |
| 620 | - | S | $C_3H_7$-n | (6-)$OC_3H_7$-n | 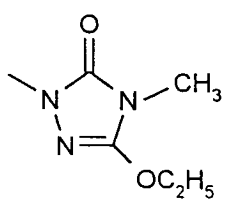 | 88 |
| 621 | - | S | $C_2H_5$ | (6-)$OCH_3$ | 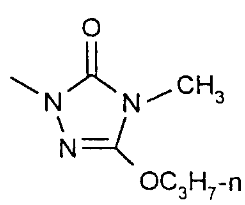 | 145 |

Tabelle 1 - Fortsetzung

| Bsp.-Nr. | A | Q | $R^1$ | (Position-) $R^2$ | $R^3$ | Schmelz-punkt |
|---|---|---|---|---|---|---|
| 622 | - | S | $C_2H_5$ | (6-)$OC_2H_5$ | 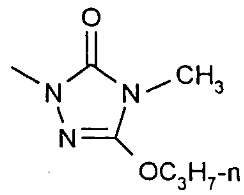 | 147 |
| 623 | - | S | $C_3H_7$-n | (6-)$OCH_3$ | 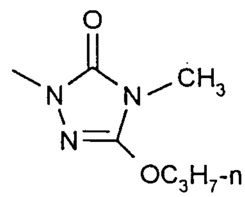 | 205 |
| 624 | - | S | $C_3H_7$-n | (6-)$OC_2H_5$ | 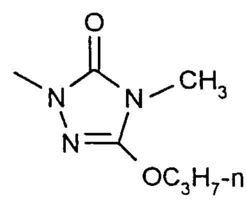 | 202 |
| 625 | - | S | $C_3H_7$-i | (6-)$OC_3H_7$-i | 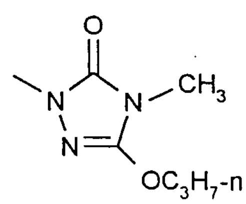 | 152 |
| 626 | - | S | $C_2H_5$ | (6-)$OC_2H_5$ | 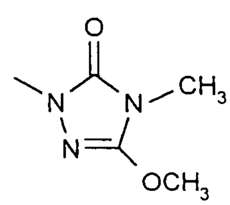 | 168 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 627 | - | S | $C_3H_7$-n | (6-)$C_3H_7$-n | 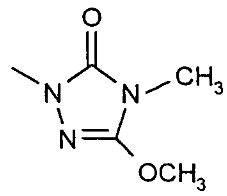 | 145 |
| 628 | - | S | $C_3H_7$-n | (6-)$OCH_3$ | 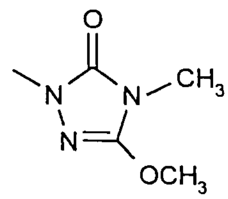 | 158 |
| 629 | - | S | $C_3H_7$-i | (6-)$OC_3H_7$-i | 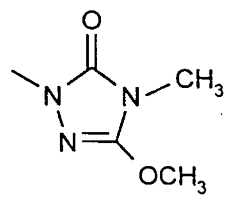 | 155 |
| 630 | - | O | $C_3H_7$-i | (6-)$OCH_3$ | 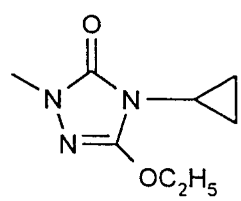 | 145 |
| 631 | - | O | $C_3H_7$-i | (6-)$OCH_3$ | 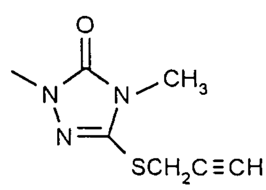 | 111 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 632 | - | O | $C_3H_7$-i | (6-)$OCH_3$ | 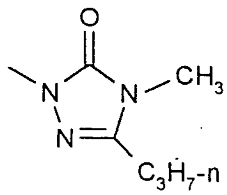 | 122 |
| 633 | - | O | $C_3H_7$-i | (6-)$OCH_3$ | 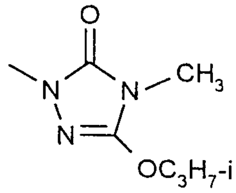 | 171 |
| 634 | - | O | $C_3H_7$-i | (6-)$OCH_3$ | 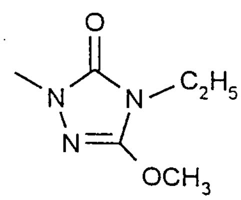 | 160 |
| 635 | - | O | $C_3H_7$-i | (6-)$OCH_3$ | 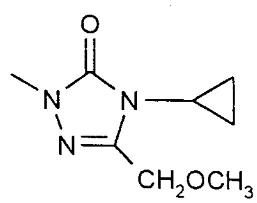 | 142 |
| 636 | - | O | $C_2H_5$ | (6-)$OC_2H_5$ | 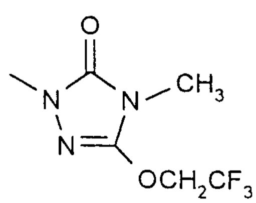 | 106 |

Tabelle 1 - Fortsetzung

| Bsp.-Nr. | A | Q | $R^1$ | (Position-) $R^2$ | $R^3$ | Schmelz-punkt |
|---|---|---|---|---|---|---|
| 637 | - | O | $C_2H_5$ | (6-)$OC_2H_5$ | 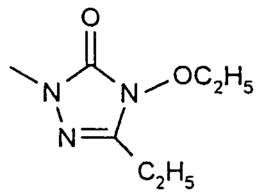 | 106 |
| 638 | - | O | $C_2H_5$ | (6-)$OC_2H_5$ | 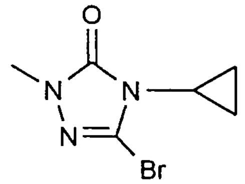 | 159 |
| 639 | - | O | $C_2H_5$ | (6-)$OC_2H_5$ | 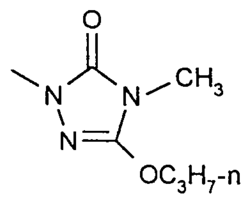 | 148 |
| 640 | - | O | $C_2H_5$ | (6-)$OC_2H_5$ | 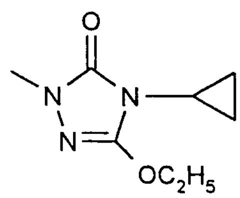 | 126 |
| 641 | - | O | $C_2H_5$ | (6-)$OC_2H_5$ | 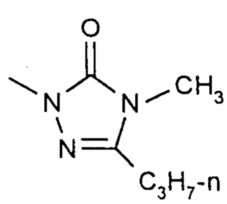 | 111 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 642 | - | O | $C_2H_5$ | (6-)$OC_2H_5$ | 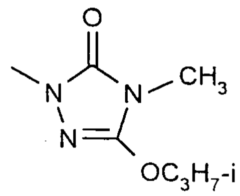 | 171 |
| 643 | - | O | $C_2H_5$ | (6-)$OC_2H_5$ | 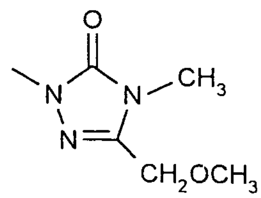 | 127 |
| 644 | - | O | $C_2H_5$ | (6-)$OC_2H_5$ | 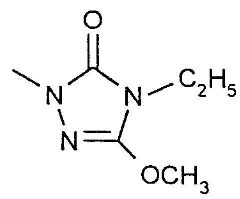 | 148 |
| 645 | - | O | $C_2H_5$ | (6-)$OC_2H_5$ | 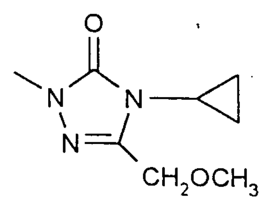 | 123 |
| 646 | - | O | $C_3H_7$-n | (6-)$OC_3H_7$-n | 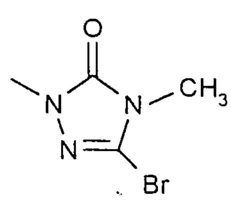 | 138 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 647 | - | O | $C_3H_7$-n | (6-)$OC_3H_7$-n | 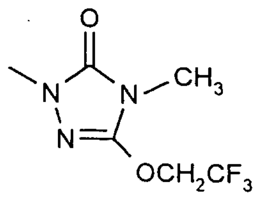 | 95 |
| 648 | - | O | $C_3H_7$-n | (6-)$OC_3H_7$-n | 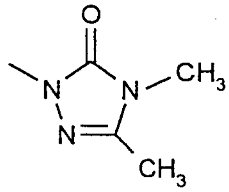 | 130 |
| 649 | - | O | $C_3H_7$-n | (6-)$OC_3H_7$-n | 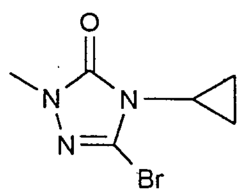 | 74 |
| 650 | - | O | $C_3H_7$-n | (6-)$OC_3H_7$-n | 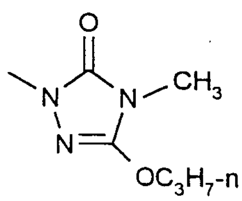 | 109 |
| 651 | - | O | $C_3H_7$-n | (6-)$OC_3H_7$-n | 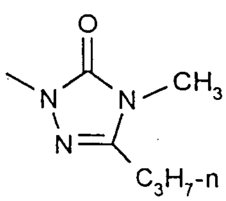 | 75 |

Tabelle 1 - Fortsetzung

| Bsp.-Nr. | A | Q | $R^1$ | (Position-) $R^2$ | $R^3$ | Schmelz-punkt |
|---|---|---|---|---|---|---|
| 652 | - | O | $C_3H_7$-n | (6-)$OC_3H_7$-n | 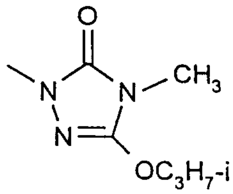 | 147 |
| 653 | - | O | $C_3H_7$-n | (6-)$OC_3H_7$-n | 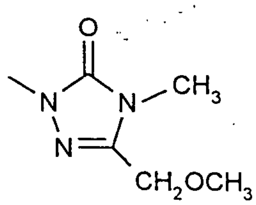 | 99 |
| 654 | - | O | $C_3H_7$-n | (6-)$OC_3H_7$-n | 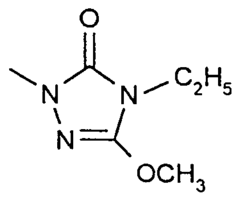 | 102 |
| 655 | - | O | $C_3H_7$-n | (6-)$OC_3H_7$-n | 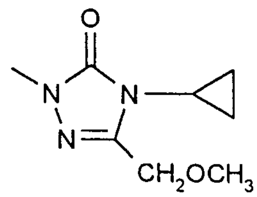 | 98 |
| 656 | - | O | $C_3H_7$-i | (6-)$OC_3H_7$-i | 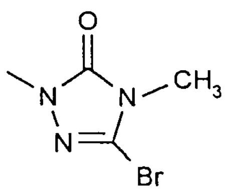 | 138 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 657 | - | O | $C_3H_7$-i | (6-)$OC_3H_7$-i | 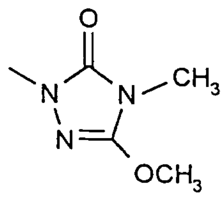 | 127 |
| 658 | - | O | $C_3H_7$-i | (6-)$OC_3H_7$-i | 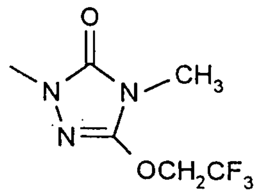 | 160 |
| 659 | - | O | $C_3H_7$-i | (6-)$OC_3H_7$-i | 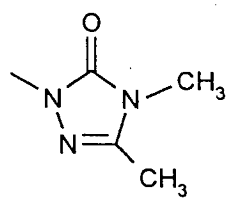 | 115 |
| 660 | - | O | $C_3H_7$-i | (6-)$OC_3H_7$-i | 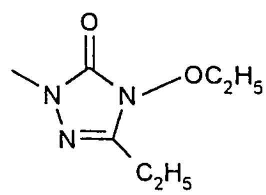 | 108 |
| 661 | - | O | $C_3H_7$-i | (6-)$OC_3H_7$-i | 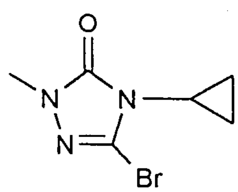 | 154 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 662 | - | O | $C_3H_7$-i | (6-)$OC_3H_7$-i | 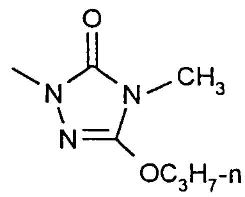 | 144 |
| 663 | - | O | $C_2H_4OC_2H_5$ | (6-)$OCH_3$ | 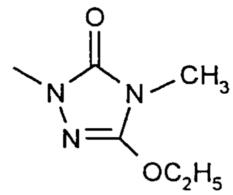 | 124 |
| 664 | - | O | $C_2H_4OC_2H_5$ | (6-)$OCH_3$ | 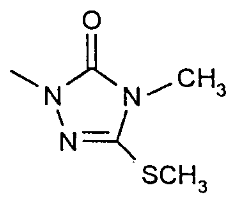 | 138 |
| 665 | - | O | $C_4H_9$-n | (6-)$OCH_3$ | 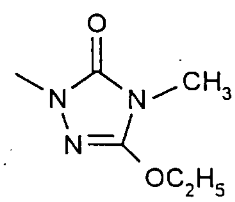 | 130 |
| 666 | - | O | $C_4H_9$-n | (6-)$OCH_3$ | 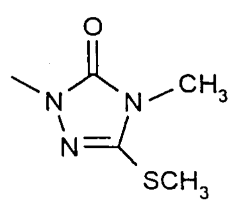 | 132 |

Tabelle 1 - Fortsetzung

| Bsp.-Nr. | A | Q | $R^1$ | (Position-) $R^2$ | $R^3$ | Schmelz-punkt |
|---|---|---|---|---|---|---|
| 667 | - | O | $C_3H_7$-i | (6-)$OC_3H_7$-i | 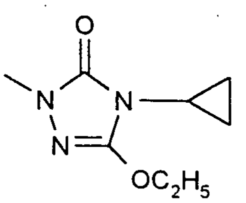 | 100 |
| 668 | - | O | $C_3H_7$-i | (6-)$OC_3H_7$-i | 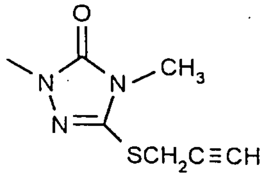 | 108 |
| 669 | - | O | $C_3H_7$-i | (6-)$OC_3H_7$-i | 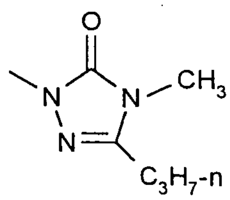 | 130 |
| 670 | - | O | $C_3H_7$-i | (6-)$OC_3H_7$-i | 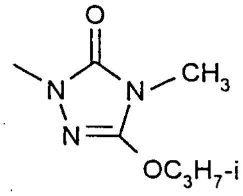 | 133 |
| 671 | - | O | $C_3H_7$-i | (6-)$OC_3H_7$-i | 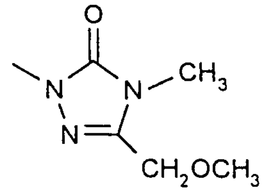 | 125 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 672 | - | O | $C_3H_7$-i | (6-)$OC_3H_7$-i | 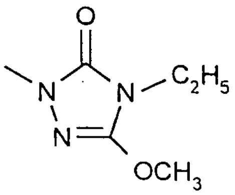 | 108 |
| 673 | - | O | $C_3H_7$-i | (6-)$OC_3H_7$-i | 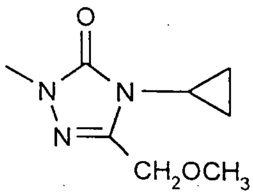 | 110 |
| 674 | - | O | $C_4H_9$-n | (6-)$OCH_3$ | 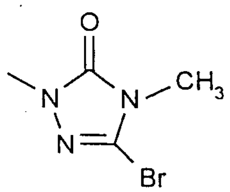 | 144 |
| 675 | - | O | $C_4H_9$-n | (6-)$OCH_3$ | 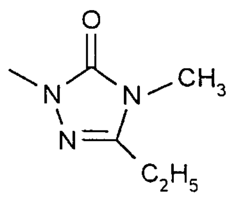 | 116 |
| 676 | - | O | $C_4H_9$-n | (6-)$OCH_3$ | 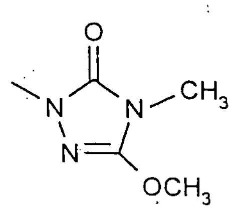 | 139 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 677 | - | O | $C_4H_9$-n | (6-)$OCH_3$ | 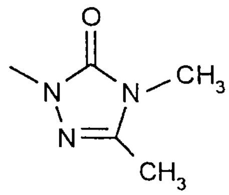 | 174 |
| 678 | - | O | $C_4H_9$-n | (6-)$OCH_3$ | 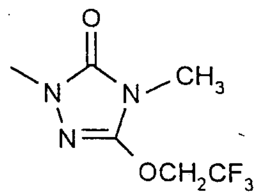 | 149 |
| 679 | - | O | $C_4H_9$-n | (6-)$OCH_3$ | 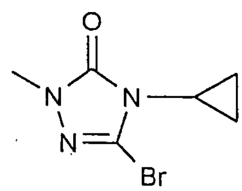 | 104 |
| 680 | - | O | $C_4H_9$-n | (6-)$OCH_3$ | 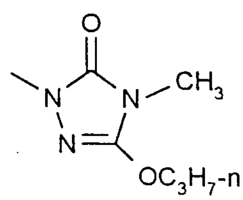 | 98 |
| 681 | - | O | $C_4H_9$-n | (6-)$OCH_3$ | 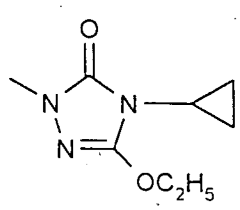 | 112 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 682 | - | O | $C_4H_9$-n | (6-)$OCH_3$ | 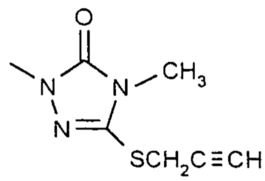 | 100 |
| 683 | - | O | $C_4H_9$-n | (6-)$OCH_3$ | 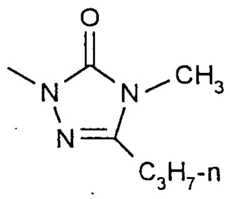 | 92 |
| 684 | - | O | $C_4H_9$-n | (6-)$OCH_3$ | 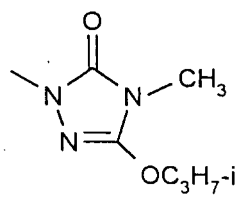 | 115 |
| 685 | - | O | $C_4H_9$-n | (6-)$OCH_3$ | 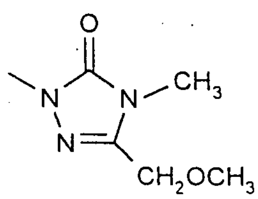 | 99 |
| 686 | - | O | $C_4H_9$-n | (6-)$OCH_3$ | 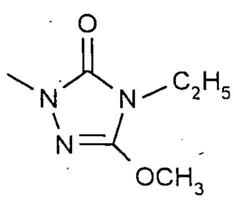 | 102 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 687 | - | O | $C_4H_9$-n | (6-)$OCH_3$ | 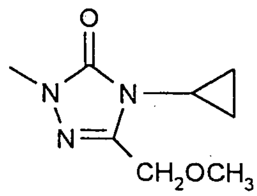 | 106 |
| 688 | - | O | $C_2H_4OC_2H_5$ | (6-)$OCH_3$ | 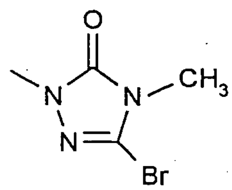 | 120 |
| 689 | - | O | $C_2H_4OC_2H_5$ | (6-)$OCH_3$ | 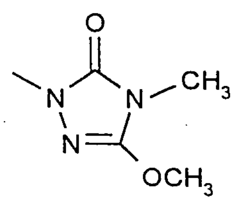 | 98 |
| 690 | - | O | $C_2H_4OC_2H_5$ | (6-)$OCH_3$ | 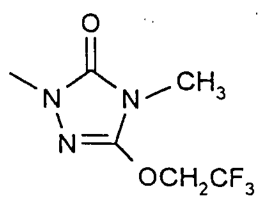 | 138 |
| 691 | - | O | $C_4H_9$-i | (6-)$OC_2H_5$ | 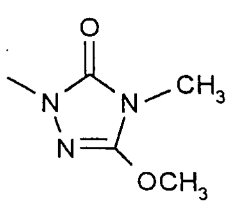 | 118 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 692 | - | O | $C_2H_4OC_2H_5$ | (6-)$OCH_3$ | 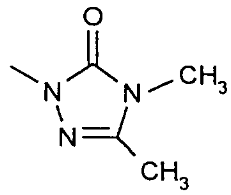 | 138 |
| 693 | - | O | $C_2H_4OC_2H_5$ | (6-)$OCH_3$ | 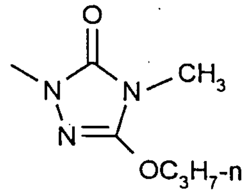 | 94 |
| 694 | - | O | $C_2H_4OC_2H_5$ | (6-)$OCH_3$ | 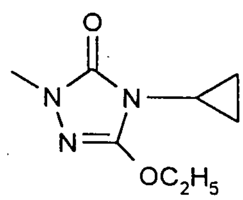 | 94 |
| 695 | - | O | $C_2H_4OC_2H_5$ | (6-)$OCH_3$ | 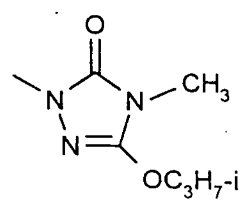 | 65 |
| 696 | - | O | $C_2H_4OC_2H_5$ | (6-)$OCH_3$ | 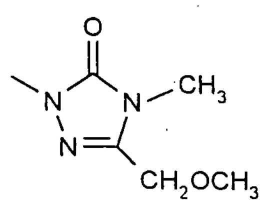 | 60 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 697 | - | O | $C_2H_4OC_2H_5$ | (6-)$OCH_3$ | 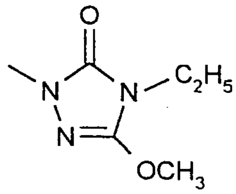 | 104 |
| 698 | - | O | $C_4H_9$-n | (6-)$OC_4H_9$-n | 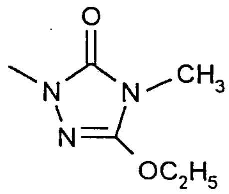 | 104 |
| 699 | - | O | $C_4H_9$-n | (6-)$OC_4H_9$-n | 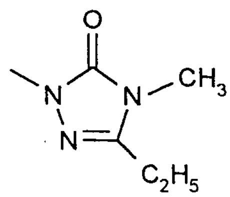 | 137 |
| 700 | - | O | $C_4H_9$-n | (6-)$OC_4H_9$-n | 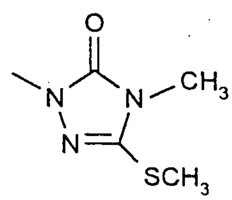 | 70 |
| 701 | - | O | $C_4H_9$-n | (6-)$OC_4H_9$-n | 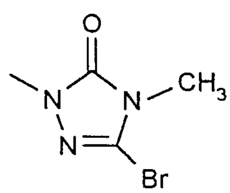 | 110 |

Tabelle 1 - Fortsetzung

| Bsp.-Nr. | A | Q | $R^1$ | (Position-) $R^2$ | $R^3$ | Schmelz-punkt |
|---|---|---|---|---|---|---|
| 702 | - | O | $C_4H_9$-n | (6-)$OC_4H_9$-n | 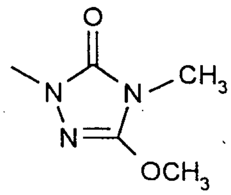 | 102 |
| 703 | - | O | $C_3H_7$-i | (6-)$OC_3H_7$-i | 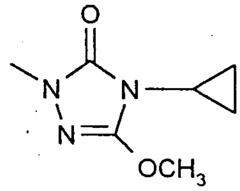 | 137 |
| 704 | - | O | $C_3H_7$-i | (6-)$OCH_3$ | 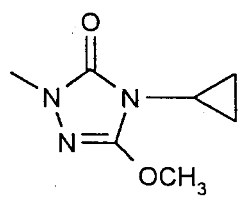 | 160 |
| 705 | - | O | $C_3H_7$-n | (6-)$OC_3H_7$-n | 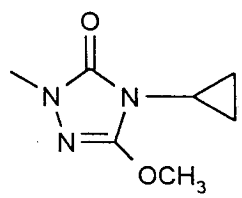 | 94 |
| 706 | - | O | $C_3H_7$-n | (6-)$OC_2H_5$ | 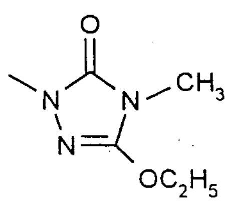 | 155 |

Tabelle 1 - Fortsetzung

| Bsp.-Nr. | A | Q | $R^1$ | (Position-) $R^2$ | $R^3$ | Schmelz-punkt |
|---|---|---|---|---|---|---|
| 707 | - | O | $C_3H_7$-n | (6-)$OC_2H_5$ | 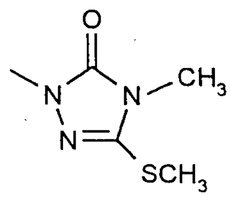 | 130 |
| 708 | - | O | $C_3H_7$-n | (6-)$OC_2H_5$ | 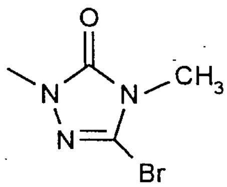 | 142 |
| 709 | - | O | $C_3H_7$-n | (6-)$OC_2H_5$ | 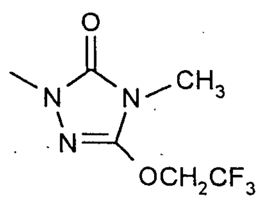 | 85 |
| 710 | - | O | $C_3H_7$-n | (6-)$OC_2H_5$ | 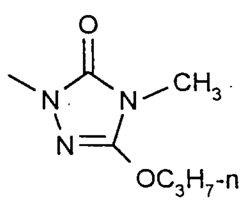 | 106 |
| 711 | - | O | $C_3H_7$-n | (6-)$OC_2H_5$ | 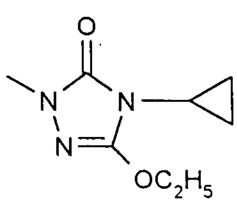 | 87 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 712 | - | S | $C_3H_7$-i | (6-)$OCH_3$ | 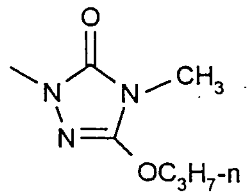 | 120 |
| 713 | - | S | $C_4H_9$-n | (6-)$OCH_3$ | 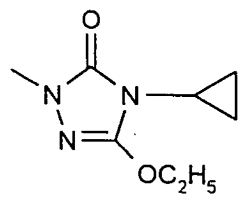 | 143 |
| 714 | - | S | $C_3H_7$-i | (6-)$OC_3H_7$-i | 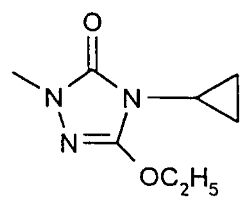 | 152 |
| 715 | - | S | $C_2H_4OC_2H_5$ | (6-)$OCH_3$ | 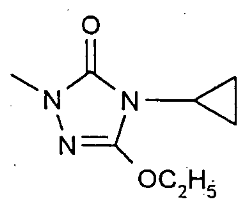 | 112 |
| 716 | - | S | $C_3H_7$-i | (6-)$OCH_3$ | 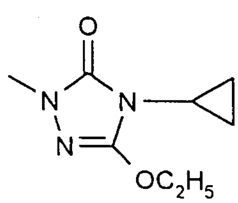 | 130 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 717 | - | S | $C_3H_7$-i | (6-)$OCH_3$ | 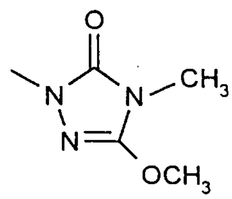 | 165 |
| 718 | - | S | $C_3H_7$-i | (6-)$OC_3H_7$-i | 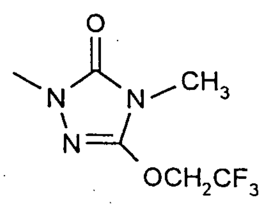 | 161 |
| 719 | - | S | $C_4H_9$-n | (6-)$OCH_3$ | 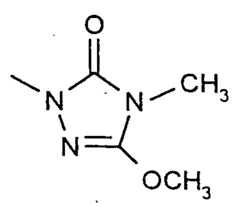 | 111 |
| 720 | - | S | $C_2H_5$ | (6-)$OCH_3$ | 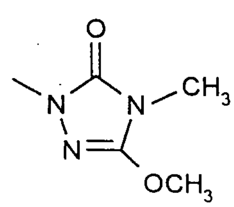 | 156 |
| 721 | - | S | $C_2H_4OC_2H_5$ | (6-)$OCH_3$ | 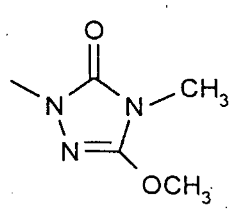 | 137 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 722 | - | S | $CH_3$ | (6-)$OCH_3$ | 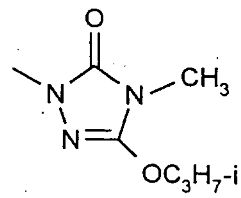 | 163 |
| 723 | - | S | $C_3H_7$-n | (6-)$OCH_3$ | 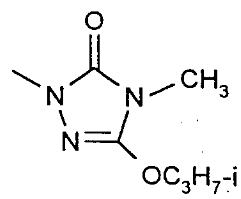 | 113 |
| 724 | - | S | $C_3H_7$-n | (6-)$OCH_3$ | 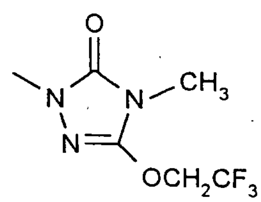 | 130 |
| 725 | - | S | $C_4H_9$-n | (6-)$OCH_3$ | 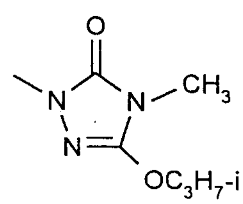 | 154 |
| 726 | - | S | $C_3H_7$-i | (6-)$OC_3H_7$-i | 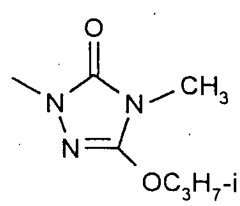 | 157 |

Tabelle 1 - Fortsetzung

| Bsp.-Nr. | A | Q | $R^1$ | (Position-) $R^2$ | $R^3$ | Schmelz-punkt |
|---|---|---|---|---|---|---|
| 727 | - | S | $C_3H_7$-n | (6-)$OC_3H_7$-n | 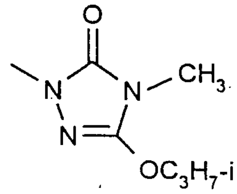 | 142 |
| 728 | - | S | $C_2H_5$ | (6-)$OC_2H_5$ | 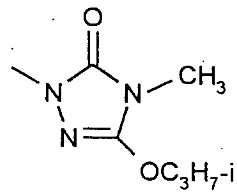 | 162 |
| 729 | - | S | $CH_3$ | (6-)$OCH_3$ | 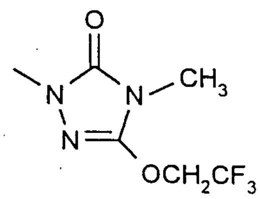 | 157 |
| 730 | - | S | $C_2H_5$ | (6-)$OCH_3$ | 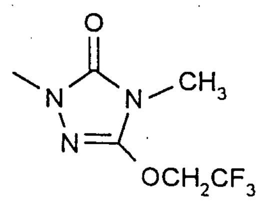 | 108 |
| 731 | - | S | $CH_3$ | (6-)$OCH_3$ | 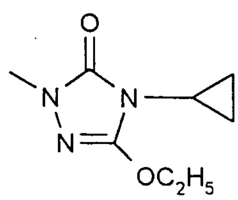 | 172 |

Tabelle 1 - Fortsetzung

| Bsp.-Nr. | A | Q | $R^1$ | (Position-) $R^2$ | $R^3$ | Schmelz-punkt |
|---|---|---|---|---|---|---|
| 732 | - | S | $CH_3$ | (6-)$OCH_3$ | 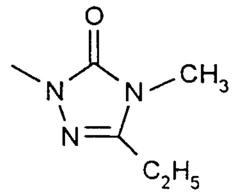 | 147 |
| 733 | - | S | $C_3H_7$-n | (6-)$OC_3H_7$-n | 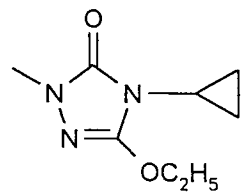 | 160 |
| 734 | - | S | $C_3H_7$-n | (6-)$OC_3H_7$-n | 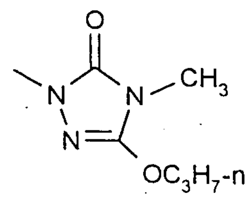 | 103 |
| 735 | - | O | $C_3H_7$-n | (6-)$OC_3H_7$-n | 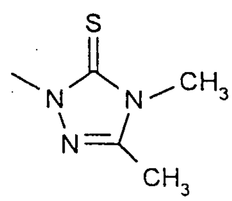 | 172 |
| 736 | - | S | $C_2H_5$ | (6-)$OC_2H_5$ | 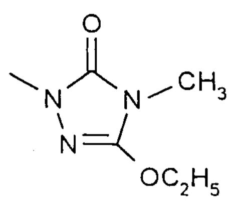 | 137 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 737 | - | S | $C_2H_5$ | (6-)$OC_2H_5$ | 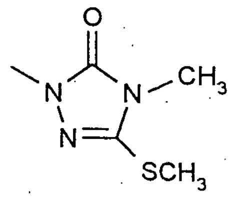 | 156 |
| 738 | - | S | $C_2H_4OC_2H_5$ | (6-)$OCH_3$ | 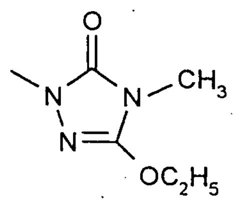 | 103 |
| 739 | - | S | $C_3H_7$-i | (6-)$OC_3H_7$-i | 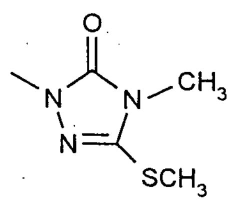 | 134 |
| 740 | - | S | $C_4H_9$-n | (6-)$OC_4H_9$-n | 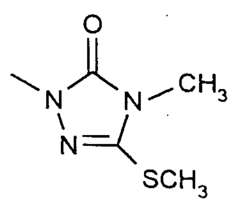 | 87 |
| 741 | - | S | $C_4H_9$-n | (6-)$OC_4H_9$-n | 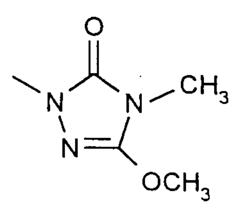 | 110 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 742 | - | S | $C_4H_9$-n | (6-)$OC_4H_9$-n | 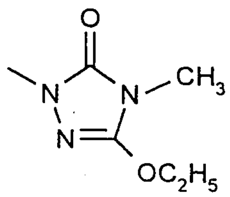 | 88 |
| 743 | - | S | $C_4H_9$-n | (6-)$OC_4H_9$-n | 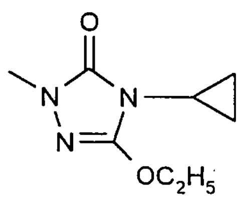 | 98 |
| 744 | - | S | $C_4H_9$-n | (6-)$OC_4H_9$-n | 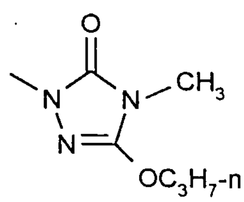 | 98 |
| 745 | - | S | $C_4H_9$-n | (6-)$OC_4H_9$-n | 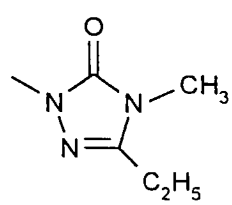 | 88 |
| 746 | - | S | $C_4H_9$-n | (6-)$OC_4H_9$-n | 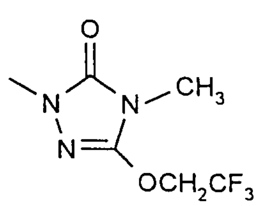 | 104 |

Tabelle 1 - Fortsetzung

| Bsp.-Nr. | A | Q | $R^1$ | (Position-) $R^2$ | $R^3$ | Schmelz-punkt |
|---|---|---|---|---|---|---|
| 747 | - | S | $C_4H_9$-n | (6-)$OC_4H_9$-n | 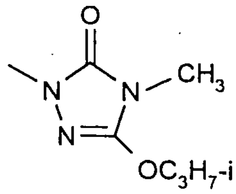 | 75 |
| 748 | - | O | $C_2H_5$ | (6-)$OC_2H_5$ | 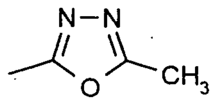 | 145 |
| 749 | - | S | $C_3H_7$-i | (6-)$OC_2H_5$ | 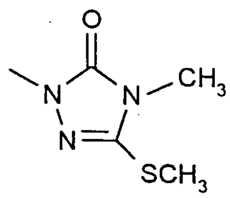 | 131 |
| 750 | - | S | $C_3H_7$-i | (6-)$OC_2H_5$ | 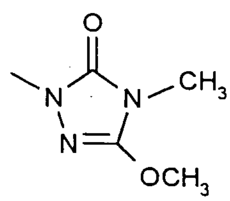 | 158 |
| 751 | - | S | $C_3H_7$-i | (6-)$OC_2H_5$ | 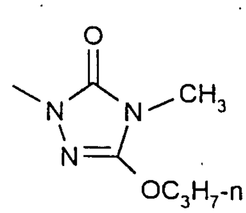 | 132 |

Tabelle 1 - Fortsetzung

| Bsp.-Nr. | A | Q | $R^1$ | (Position-) $R^2$ | $R^3$ | Schmelz-punkt |
|---|---|---|---|---|---|---|
| 752 | - | S | $C_3H_7$-i | (6-)$OC_2H_5$ | 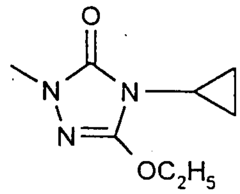 | 142 |
| 753 | - | S | $C_3H_7$-i | (6-)$OC_2H_5$ | 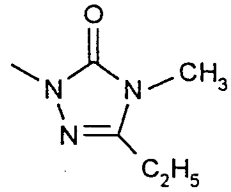 | 130 |
| 754 | - | S | $C_3H_7$-i | (6-)$OC_2H_5$ | 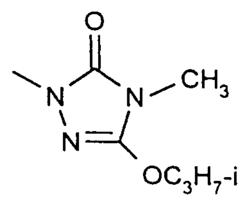 | 170 |
| 755 | - | S | $C_3H_7$-n | (6-)$OC_2H_5$ | 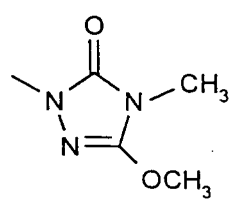 | 152 |
| 756 | - | S | $C_3H_7$-n | (6-)$OC_2H_5$ | 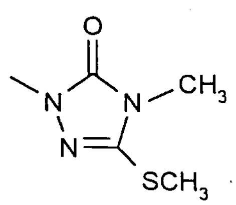 | 138 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 757 | - | S | $C_3H_7$-n | (6-)$OC_2H_5$ | 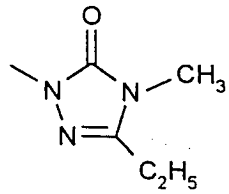 | 130 |
| 758 | - | S | $C_3H_7$-n | (6-)$OC_2H_5$ | 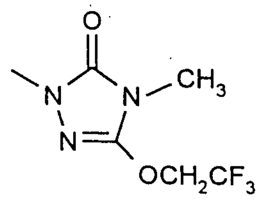 | 150 |
| 759 | - | S | $C_3H_7$-n | (6-)$OC_2H_5$ | 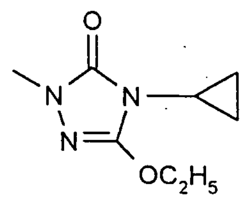 | 156 |
| 760 | - | S | $C_3H_7$-n | (6-)$OC_2H_5$ | 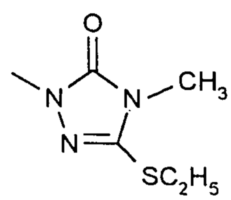 | 110 |
| 761 | - | S | $C_3H_7$-n | (6-)$OC_2H_5$ | 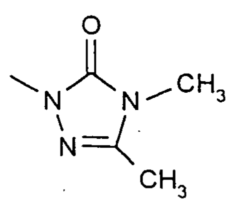 | 120 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 762 | - | S | $C_4H_9$-n | (6-)$OC_4H_9$-n | 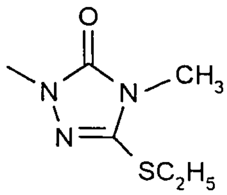 | 104 |
| 763 | - | S | $C_3H_7$-i | (6-)$OCH_3$ | 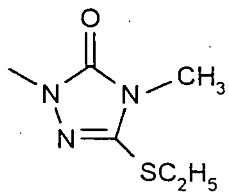 | 105 |
| 764 | - | S | $C_3H_7$-n | (6-)$OC_3H_7$-n | 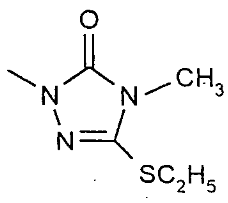 | 120 |
| 765 | - | S | $C_3H_7$-n | (6-)$OCH_3$ | 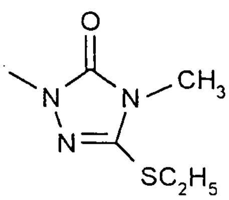 | 135 |
| 766 | - | S | $C_3H_7$-n | (6-)$OC_3H_7$-n | 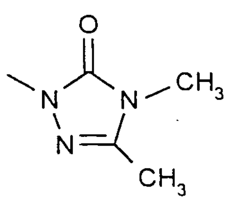 | 116 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 767 | - | S | $C_3H_7$-i | (6-)$OC_3H_7$-i | 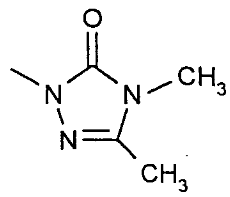 | 110 |
| 768 | - | S | $C_2H_4OC_2H_5$ | (6-)$OCH_3$ | 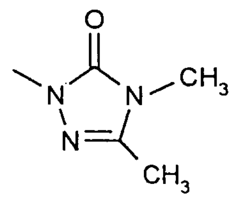 | 95 |
| 769 | - | S | $C_3H_7$-i | (6-)$OCH_3$ | 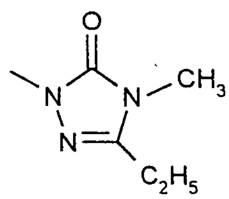 | 112 |
| 770 | - | O | $C_3H_7$-i | (6-)$OC_2H_5$ | 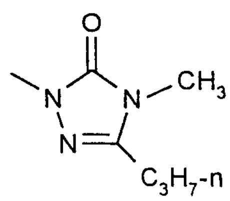 | 70 |
| 771 | - | O | $C_3H_7$-i | (6-)$OC_2H_5$ | 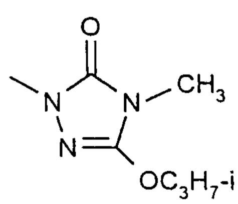 | 132 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 772 | - | O | $C_3H_7$-i | (6-)$OC_2H_5$ | 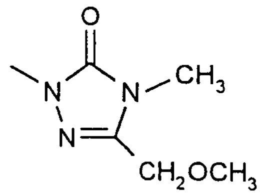 | 75 |
| 773 | - | O | $C_3H_7$-i | (6-)$OC_2H_5$ | 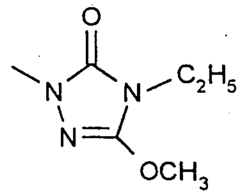 | 118 |
| 774 | - | O | $C_3H_7$-i | (6-)$OC_2H_5$ | 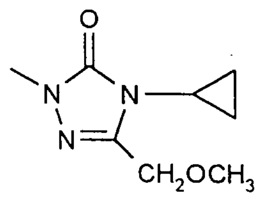 | 85 |
| 775 | - | O | $C_3H_7$-i | (6-)$OC_2H_5$ | 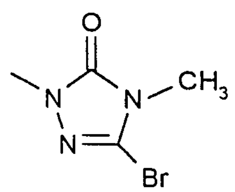 | 130 |
| 776 | - | O | $C_3H_7$-i | (6-)$OC_2H_5$ | 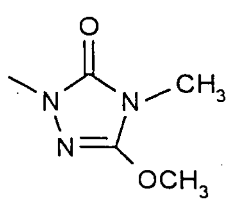 | 120 |

<u>Tabelle 1</u> - Fortsetzung

| Bsp.-Nr. | A | Q | $R^1$ | (Position-) $R^2$ | $R^3$ | Schmelzpunkt |
|---|---|---|---|---|---|---|
| 777 | - | O | $C_3H_7$-i | (6-)$OC_2H_5$ | 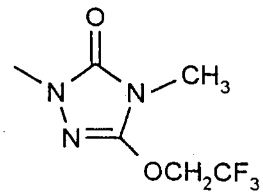 | 124 |
| 778 | - | O | $C_3H_7$-i | (6-)$OC_2H_5$ | 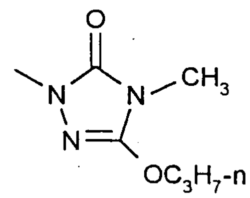 | 130 |
| 779 | - | O | $C_3H_7$-i | (6-)$OC_2H_5$ | 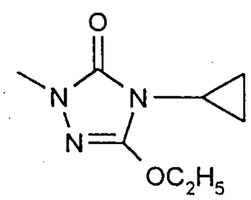 | 100 |
| 780 | - | O | $C_2H_5$ | (6-)$OCH_3$ | 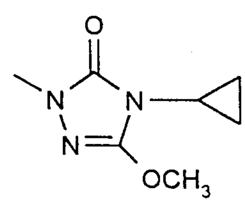 | 172 |
| 781 | - | O | $C_2H_5$ | (6-)$OCH_3$ | 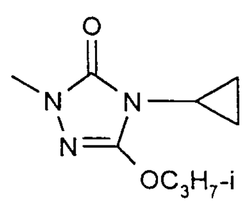 | 164 |

Tabelle 1 - Fortsetzung

| Bsp.-Nr. | A | Q | $R^1$ | (Position-) $R^2$ | $R^3$ | Schmelz-punkt |
|---|---|---|---|---|---|---|
| 782 | - | O | $C_3H_7$-i | (6-)$OC_3H_7$-i | 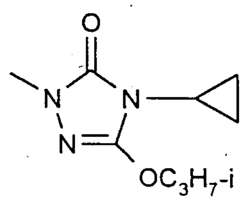 | 118 |
| 783 | - | O | $C_3H_7$-i | (6-)$OC_3H_7$-i | 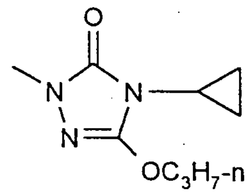 | 88 |
| 784 | - | O | $C_4H_9$-s | (6-)$OCH_3$ | 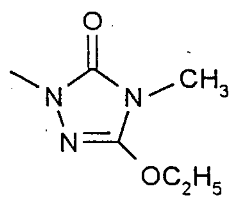 | 124 |
| 785 | - | O | $C_4H_9$-s | (6-)$OCH_3$ | 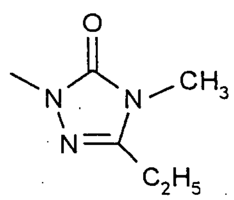 | 100 |
| 786 | - | O | $C_4H_9$-n | (6-)$OC_2H_5$ | 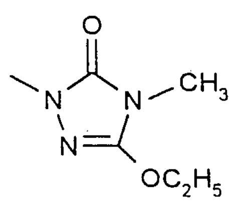 | 106 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 787 | - | O | $C_4H_9$-n | (6-)$OC_2H_5$ | 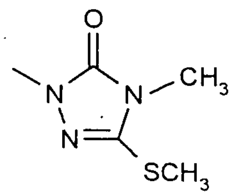 | 108 |
| 788 | - | O | $C_4H_9$-n | (6-)$OC_2H_5$ | 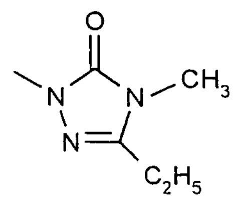 | 105 |
| 789 | - | O | $C_4H_9$-s | (6-)$OCH_3$ | 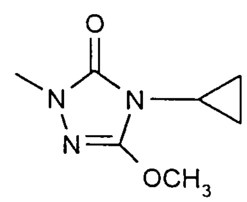 | 112 |
| 790 | - | O | $C_4H_9$-n | (6-)$OC_2H_5$ | 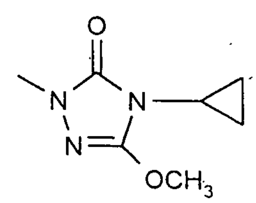 | 80 |
| 791 | - | O | $C_4H_9$-s | (6-)$OCH_3$ | 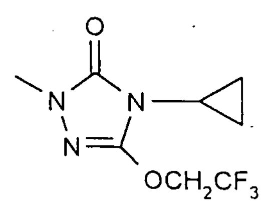 | 130 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 792 | - | O | $C_4H_9$-n | (6-)$OC_2H_5$ | 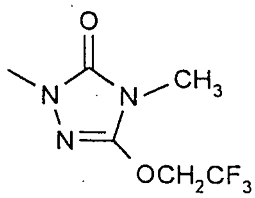 | 120 |
| 793 | - | O | $C_4H_9$-n | (6-)$OC_2H_5$ | 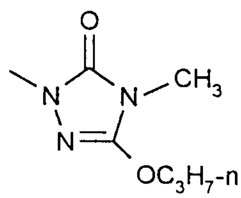 | 95 |
| 794 | - | O | $C_4H_9$-n | (6-)$OC_2H_5$ | 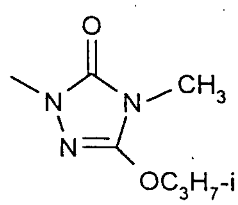 | 96 |
| 795 | - | O | $C_4H_9$-s | (6-)$OCH_3$ | 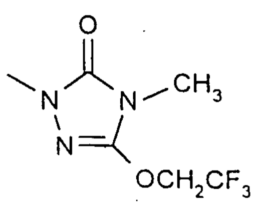 | 130 |
| 796 | - | O | $C_4H_9$-s | (6-)$OCH_3$ | 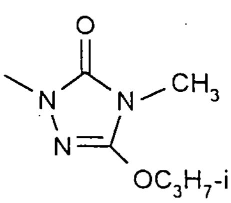 | 118 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 797 | - | O | $C_4H_9$-s | (6-)$OCH_3$ | 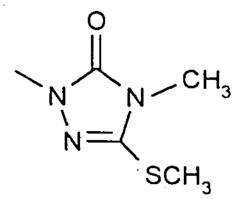 | 134 |
| 798 | - | O | $C_4H_9$-s | (6-)$OCH_3$ | 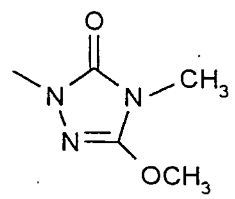 | 118 |
| 799 | - | O | $C_4H_9$-n | (6-)$OC_2H_5$ | 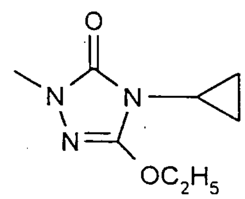 | 90 |
| 800 | - | O | $C_4H_9$-s | (6-)$OCH_3$ | 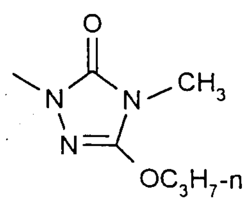 | 78 |
| 801 | - | O | $C_4H_9$-s | (6-)$OCH_3$ | 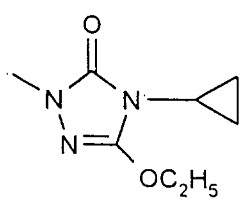 | 112 |

Tabelle 1 - Fortsetzung

| Bsp.-Nr. | A | Q | $R^1$ | (Position-) $R^2$ | $R^3$ | Schmelz-punkt |
|---|---|---|---|---|---|---|
| 802 | - | O | $C_4H_9$-s | (6-)$OCH_3$ | 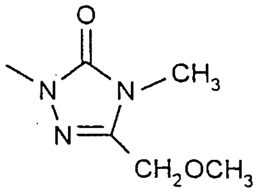 | 78 |
| 803 | - | O | $C_4H_9$-s | (6-)$OCH_3$ | 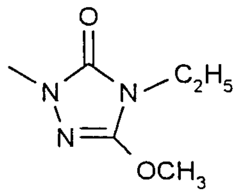 | 80 |
| 804 | - | O | $C_4H_9$-n | (6-)$OC_4H_9$-n | 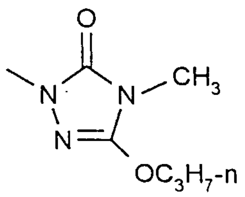 | 55 |
| 805 | - | O | $C_4H_9$-n | (6-)$OC_4H_9$-n | 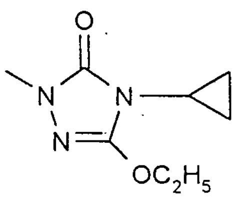 | 100 |
| 806 | - | O | $C_4H_9$-n | (6-)$OC_4H_9$-n | 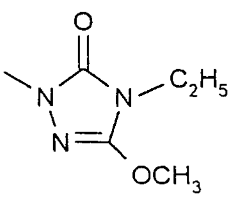 | 92 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 807 | - | O | $C_3H_7$-n | (6-)$OC_2H_5$ | 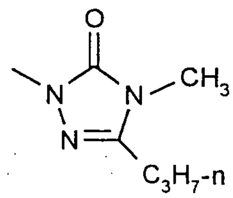 | 74 |
| 808 | - | O | $C_3H_7$-n | (6-)$OC_2H_5$ | 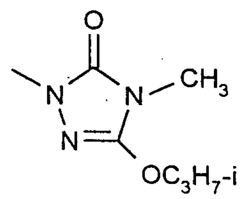 | 143 |
| 809 | - | O | $C_3H_7$-n | (6-)$OC_2H_5$ | 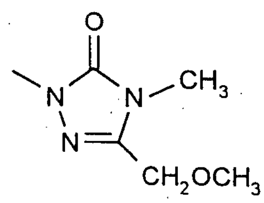 | 102 |
| 810 | - | O | $C_3H_7$-n | (6-)$OC_2H_5$ | 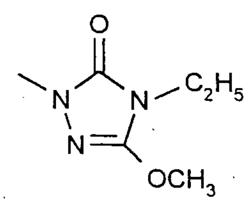 | 95 |
| 811 | - | O | $C_4H_9$-n | (6-)$OC_4H_9$-n | 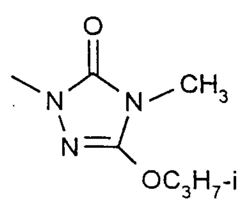 | 82 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 812 | - | O | $C_4H_9$-n | (6-)$OC_2H_5$ | 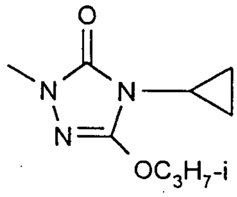 | 92 |
| 813 | - | O | $C_4H_9$-n | (6-)$OCH_3$ | 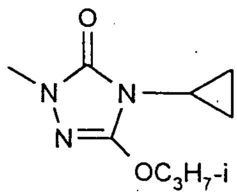 | 90 |
| 814 | - | O | $C_4H_9$-n | (6-)$OCH_3$ | 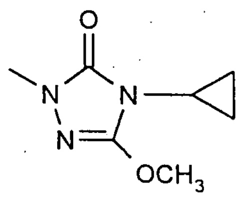 | 124 |
| 815 | - | O | $C_4H_9$-n | (6-)$OCH_3$ | 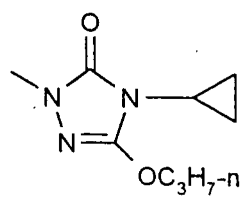 | 85 |
| 816 | - | O | $C_4H_9$-n | (6-)$OCH_3$ | 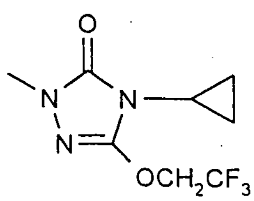 | 90 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 817 | - | O | $C_2H_4OC_2H_5$ | (6-)$OCH_3$ | 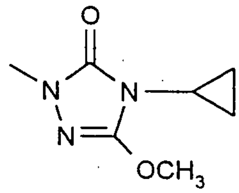 | 90 |
| 818 | - | O | 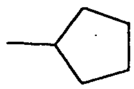 | (6-)$OCH_3$ | 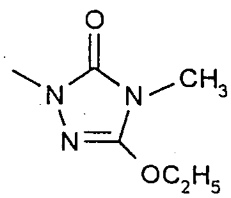 | 165 |
| 819 | - | O | 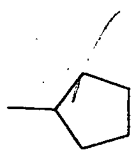 | (6-)$OCH_3$ | 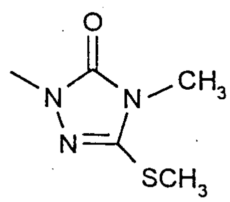 | 130 |
| 820 | - | O | 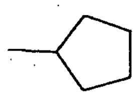 | (6-)$OCH_3$ | 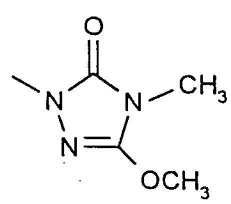 | 149 |
| 821 | - | O | $C_4H_9$-s | (6-)$OC_4H_9$-s | 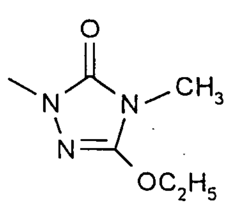 | 125 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 822 | - | O | 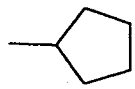 | (6-)$OCH_3$ | 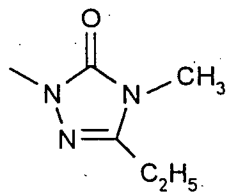 | 112 |
| 823 | - | O | 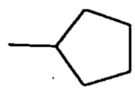 | (6-)$OCH_3$ | 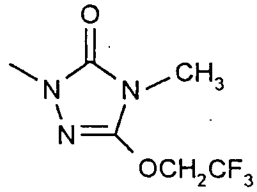 | 156 |
| 824 | - | O | 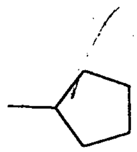 | (6-)$OCH_3$ | 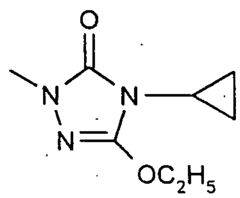 | 148 |
| 825 | - | O | 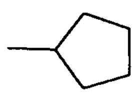 | (6-)$OCH_3$ | 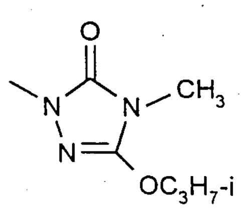 | 145 |
| 826 | - | O | 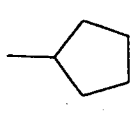 | (6-)$OCH_3$ | 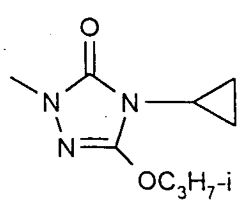 | 156 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 827 | - | O | 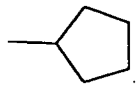 | (6-)$OCH_3$ | 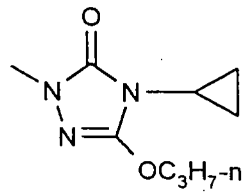 | 126 |
| 828 | - | O | 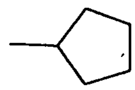 | (6-)$OCH_3$ | 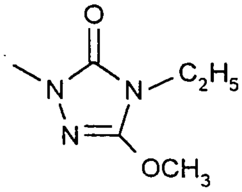 | 134 |
| 829 | - | O | 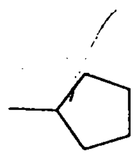 | (6-)$OCH_3$ | 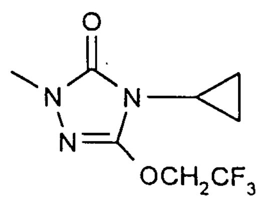 | 114 |
| 830 | - | O | 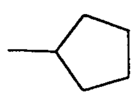 | (6-)$OCH_3$ | 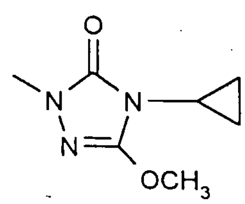 | 141 |
| 831 | - | O | $C_4H_9$-i | (6-)$OC_2H_5$ | 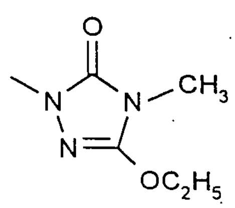 | 125 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 832 | - | O | $C_4H_9$-i | (6-)$OC_2H_5$ | 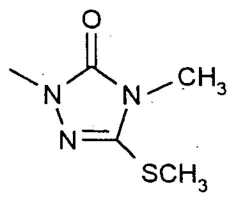 | 128 |
| 833 | - | O | $C_4H_9$-i | (6-)$OC_2H_5$ | 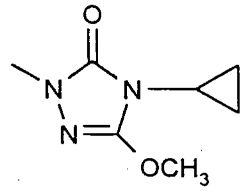 | 106 |
| 834 | - | O | $C_4H_9$-i | (6-)$OC_2H_5$ | 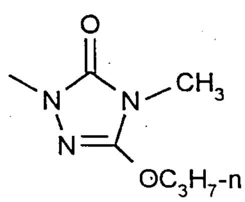 | 88 |
| 835 | - | O | $C_4H_9$-i | (6-)$OC_2H_5$ | 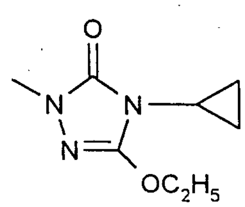 | 112 |
| 836 | - | O | $C_4H_9$-i | (6-)$OC_2H_5$ | 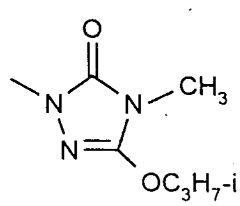 | 125 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 837 | - | O | $C_4H_9$-i | (6-)$OC_2H_5$ | 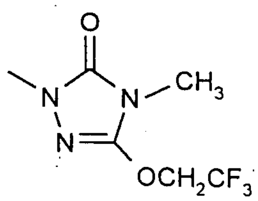 | 106 |
| 838 | - | O | H | (6-)OH | 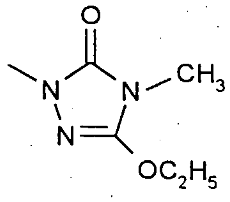 | 172 |
| 839 | - | O | $C_4H_9$-i | (6-)$OCH_3$ | 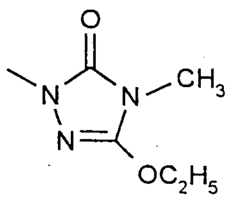 | 102 |
| 840 | - | O | $C_4H_9$-i | (6-)$OCH_3$ | 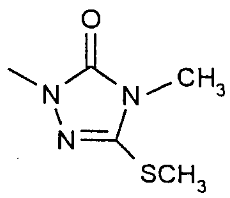 | 114 |
| 841 | - | O | $C_4H_9$-i | (6-)$OCH_3$ | 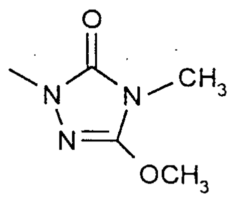 | 124 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 842 | - | O | $C_4H_9$-i | (6-)$OCH_3$ | 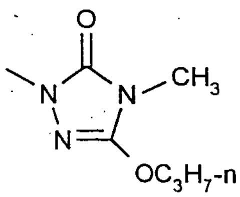 | 98 |
| 843 | - | O | $C_4H_9$-i | (6-)$OCH_3$ | 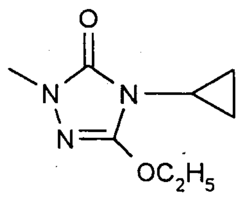 | 146 |
| 844 | - | O | $C_4H_9$-i | (6-)$OCH_3$ | 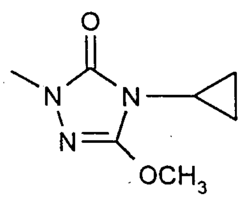 | 97 |
| 845 | - | O | $C_4H_9$-i | (6-)$OCH_3$ | 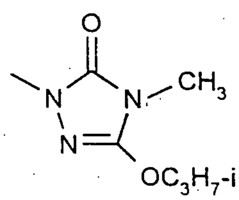 | 117 |
| 846 | - | O | $C_4H_9$-i | (6-)$OCH_3$ | 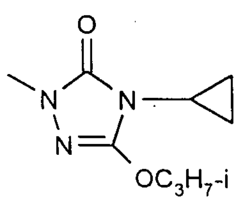 | 142 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 847 | - | O | $C_4H_9$-i | (6-)$OCH_3$ | 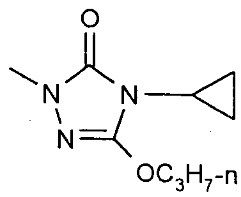 | 109 |
| 848 | - | S | $C_2H_5$ | (6-)$OCH_3$ | 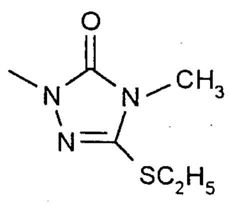 | 138 |
| 849 | - | S | $C_2H_5$ | (6-)$OCH_3$ | 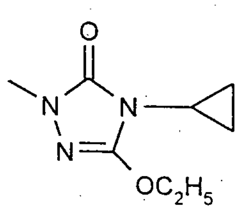 | 135 |
| 850 | - | S | $C_2H_5$ | (6-)$OCH_3$ | 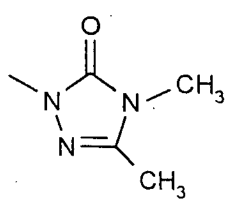 | 155 |
| 851 | - | S | $C_2H_5$ | (6-)$OCH_3$ | 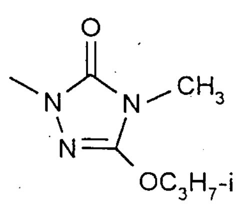 | 160 |

Tabelle 1 - Fortsetzung

| Bsp.-Nr. | A | Q | $R^1$ | (Position-) $R^2$ | $R^3$ | Schmelz-punkt |
|---|---|---|---|---|---|---|
| 852 | - | S | $C_4H_9$-s | (6-)$OCH_3$ | 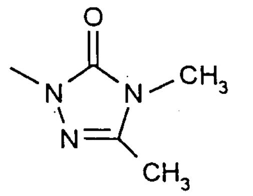 | 108 |
| 853 | - | S | $C_4H_9$-s | (6-)$OCH_3$ | 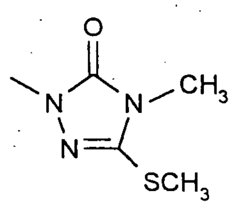 | 143 |
| 854 | - | S | $C_4H_9$-s | (6-)$OCH_3$ | 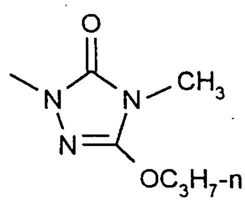 | 105 |
| 855 | - | S | $C_4H_9$-s | (6-)$OCH_3$ | 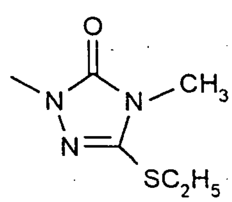 | 65 |
| 856 | - | S | $C_4H_9$-s | (6-)$OCH_3$ | 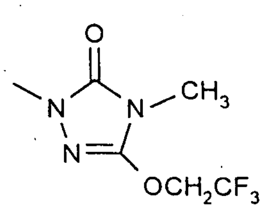 | 114 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 857 | - | O | $C_2H_5$ | (3-)$CH_3$ | 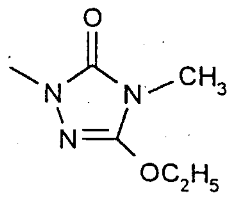 | 179 |
| 858 | - | S | $C_4H_9$-s | (6-)$OCH_3$ | 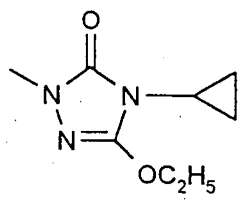 | 146 |
| 859 | - | S | $C_3H_7$-i | (6-)$OCH_3$ | 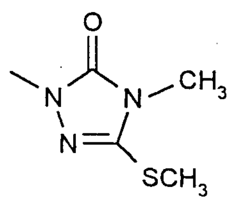 | 146 |
| 860 | - | S | $C_4H_9$-n | (6-)$OCH_3$ | 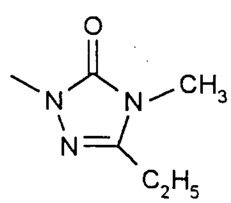 | 132 |
| 861 | - | S | $C_4H_9$-n | (6-)$OCH_3$ | 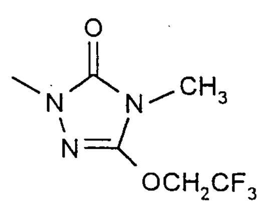 | 112 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 862 | - | S | $C_4H_9$-n | (6-)$OCH_3$ | 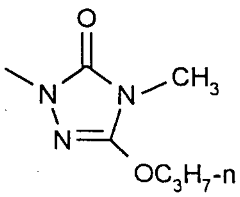 | 100 |
| 863 | - | S | $C_4H_9$-n | (6-)$OCH_3$ | 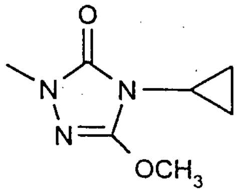 | 138 |
| 864 | - | S | $C_3H_7$-i | (6-)$OC_2H_5$ | 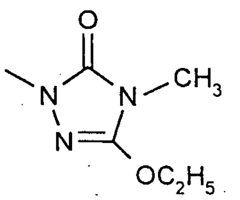 | 155 |
| 865 | - | S | $C_3H_7$-i | (6-)$OC_2H_5$ | 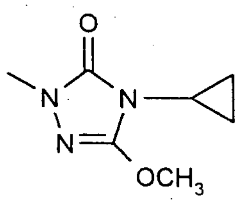 | 140 |
| 866 | - | S | $C_4H_9$-n | (6-)$OCH_3$ | 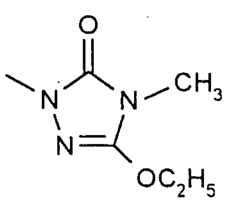 | 140 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 867 | - | O | $C_4H_9$-i | (6-)$OCH_3$ | 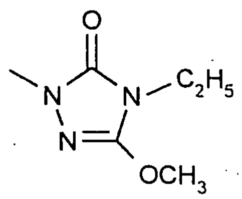 | 131 |
| 868 | - | O | $C_4H_9$-i | (6-)$OCH_3$ | 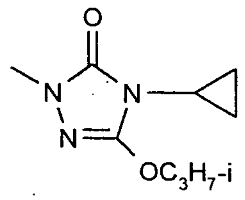 | 135 |
| 869 | - | O | $C_4H_9$-i | (6-)$OCH_3$ | 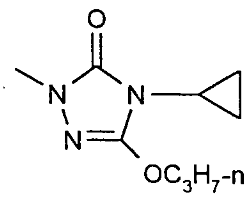 | 137 |
| 870 | - | O | $C_4H_9$-n | (6-)$OC_4H_9$-n | 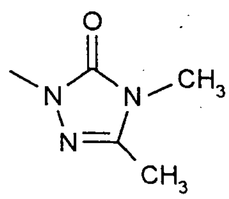 | 123 |
| 871 | - | O | $C_4H_9$-n | (6-)$OC_2H_5$ | 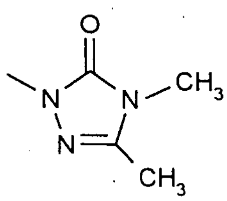 | 118 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 872 | - | O | 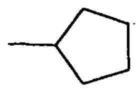 | (6-)$OCH_3$ | 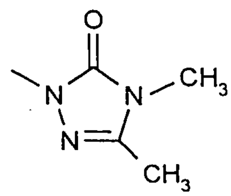 | 164 |
| 873 | - | O | $C_3H_7$-i | (6-)$OCH_3$ | 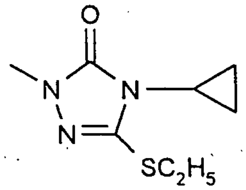 | 150 |
| 874 | - | O | $C_3H_7$-i | (6-)$OC_3H_7$-i | 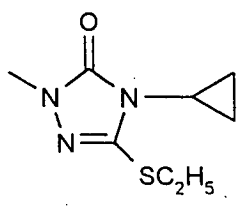 | 148 |
| 875 | - | O | $C_3H_7$-n | (6-)$OCH_3$ | 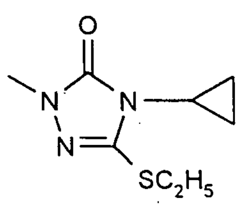 | 147 |
| 876 | - | O | $C_2H_5$ | (6-)$OC_2H_5$ | 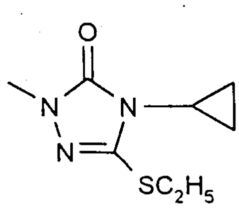 | 108 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 877 | - | O | $C_3H_7$-i | (6-)$OC_3H_7$-i | 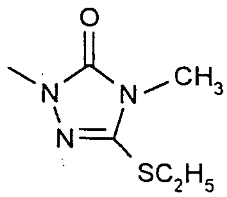 | 108 |
| 878 | - | O | $C_3H_7$-i | (6-)$OC_3H_7$-i | 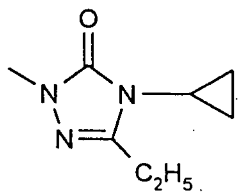 | 148 |
| 879 | - | O | $C_2H_5$ | (6-)$OC_2H_5$ | 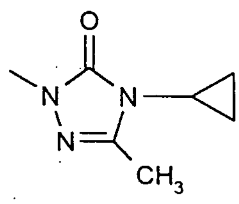 | 176 |
| 880 | - | O | $C_3H_7$-n | (6-)$OCH_3$ | 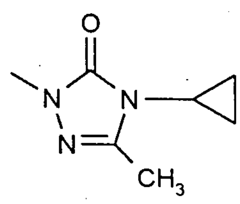 | 144 |
| 881 | - | O | $C_3H_7$-i | (6-)$OC_3H_7$-i | 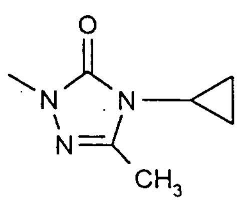 | 167 |

Tabelle 1 - Fortsetzung

| Bsp.-Nr. | A | Q | $R^1$ | (Position-) $R^2$ | $R^3$ | Schmelz-punkt |
|---|---|---|---|---|---|---|
| 882 | - | O | $C_2H_5$ | (6-)$OC_2H_5$ | 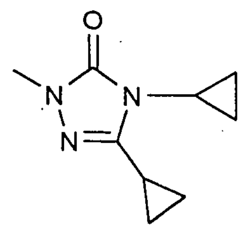 | 135 |
| 883 | - | O | $C_3H_7$-n | (6-)$OCH_3$ | 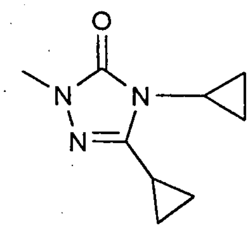 | 100 |
| 884 | - | O | $C_2H_5$ | (6-)$OC_2H_5$ | 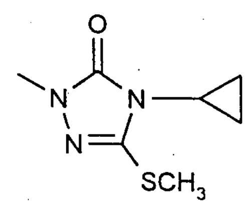 | 158 |
| 885 | - | O | $C_3H_7$-n | (6-)$OCH_3$ | 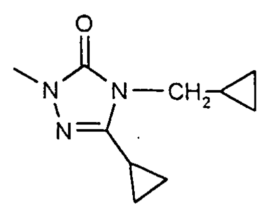 | 108 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 886 | - | O | $C_3H_7$-i | (6-)$OC_3H_7$-i | 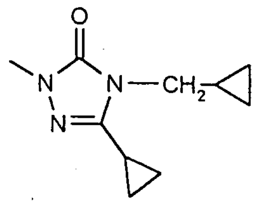 | 164 |
| 887 | - | O | $C_3H_7$-i | (6-)$OC_3H_7$-i | 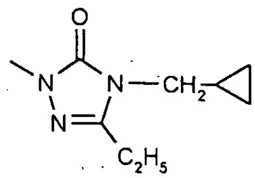 | 157 |
| 888 | - | O | $C_3H_7$-n | (6-)$OCH_3$ | 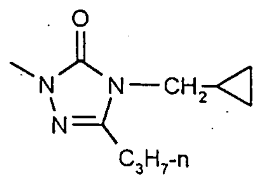 | 113 |
| 889 | - | O | $C_3H_7$-i | (6-)$OC_3H_7$-i | 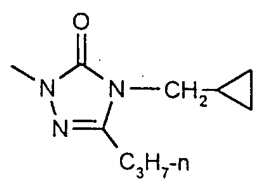 | 132 |
| 890 | - | O | $C_3H_7$-n | (6-)$OCH_3$ | 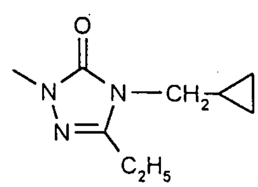 | 92 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 891 | - | O | $C_3H_7$-i | (6-)$OC_3H_7$-i | 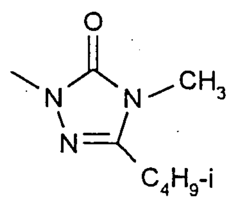 | 141 |
| 892 | - | O | $C_3H_7$-i | (6-)$OC_3H_7$-i | 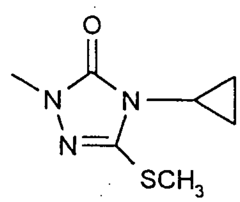 | 159 |
| 893 | - | O | $C_2H_5$ | (6-)$OC_2H_5$ | 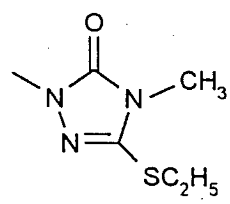 | 139 |
| 894 | - | O | $C_2H_5$ | (6-)$OC_2H_5$ | 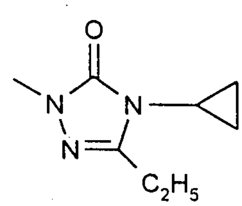 | 150 |
| 895 | - | O | $C_3H_7$-n | (6-)$OCH_3$ | 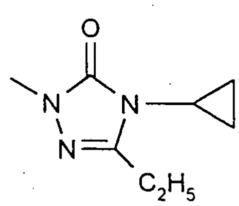 | 126 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 896 | - | O | $C_3H_7$-i | (6-)$CH_3$ | 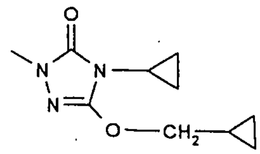 | 148 |
| 897 | - | O | $C_3H_7$-i | (6-)$CH_3$ | 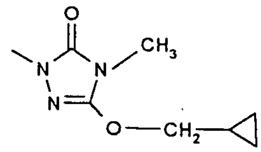 | 157 |
| 898 | - | O | $C_3H_7$-i | (6-)$CH_3$ | 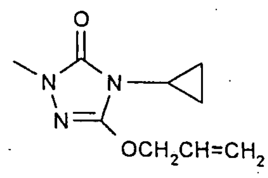 | 125 |
| 899 | NH | O | $CH_3$ | (6-)$OCH_3$ | 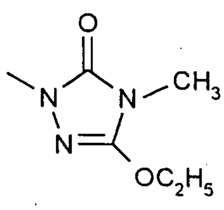 | 182 |
| 900 | NH | O | $CH_3$ | (6-)$OCH_3$ | 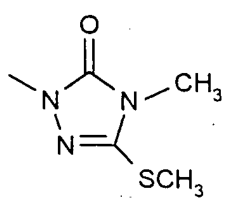 | 175 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 901 | - | O | $CF_3$ | (6-)$CH_3$ | 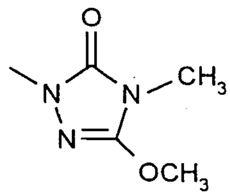 | 198 (Na-Salz) |
| 902 | - | O | $CF_3$ | (6-)$CH_3$ | 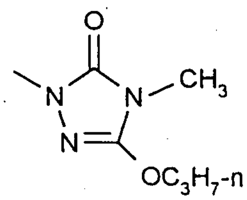 | 129 |
| 903 | - | O | $CF_3$ | (6-)$CH_3$ | 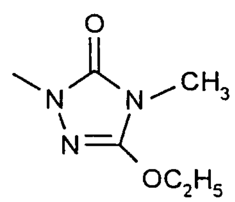 | 149 |
| 904 | - | O | $CF_3$ | (6-)$CH_3$ | 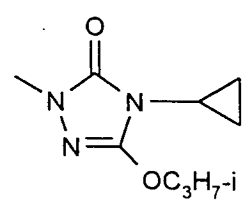 | 163 |
| 905 | - | O | $CF_3$ | (6-)$C_2H_5$ | 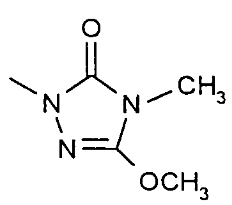 | 121 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 906 | - | O | $C_3H_7$-i | (6-)$CH_3$ | 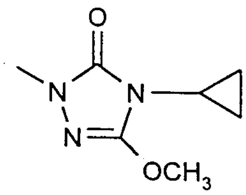 | 170 |
| 907 | - | O | $C_3H_7$-i | (6-)$CH_3$ | 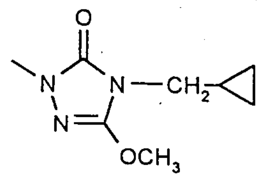 | 125 |
| 908 | - | O | $C_3H_7$-i | (6-)$CH_3$ | 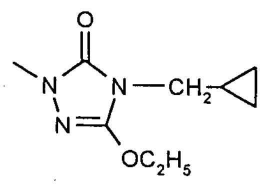 | 129 |
| 909 | - | O | $C_3H_7$-i | (6-)$CH_3$ | 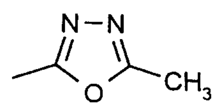 | 156 |
| 910 | - | O | $CH_3$ | (6-)$OCH_3$ | 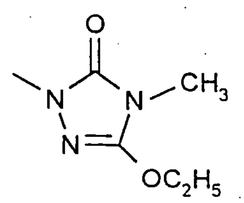 | 157 |
| 911 | - | O | $CH_3$ | (6-)$OCH_3$ | 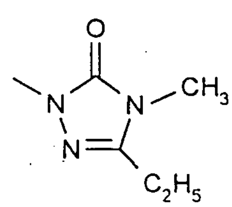 | 177 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 912 | - | O | $CH_3$ | (6-)$OCH_3$ | 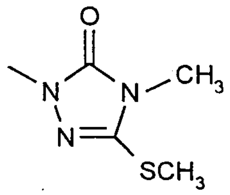 | 172 |
| 913 | - | O | $C_3H_7$-i | (6-)$CH_3$ | 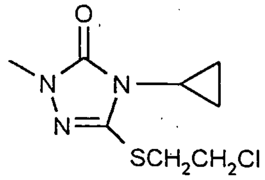 | 132 |
| 914 | - | O | $C_3H_7$-i | (6-)$CH_3$ | 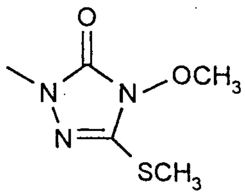 | 153 |
| 915 | - | O | $C_3H_7$-i | (6-)$CH_3$ | 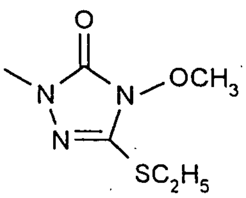 | 150 |
| 916 | - | O | $C_3H_7$-i | (6-)$CH_3$ | 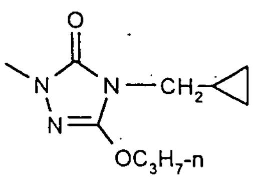 | 110 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 917 | - | O | $C_3H_7$-i | (6-)$CH_3$ | 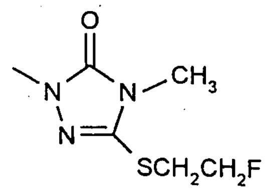 | 131 |
| 918 | - | O | $C_3H_7$-i | (6-)$CH_3$ | 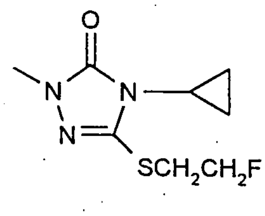 | 133 |
| 919 | - | O | $CH_3$ | (6-)$OCH_3$ | 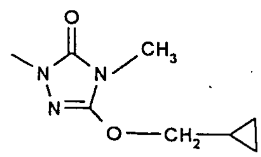 | 153 |
| 920 | - | O | $C_3H_7$-i | (6-)$CH_3$ | 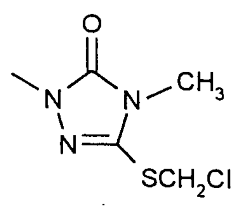 | 122 |
| 921 | - | O | $C_3H_7$-i | (6-)$CH_3$ | 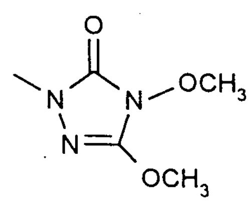 | 147 |

Tabelle 1 - Fortsetzung

| Bsp.-Nr. | A | Q | $R^1$ | (Position-) $R^2$ | $R^3$ | Schmelz-punkt |
|---|---|---|---|---|---|---|
| 922 | - | O | $CH_3$ | (6-)$OCH_3$ | 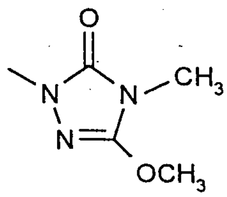 | 160 |
| 923 | - | O | $CH_3$ | (6-)$OCH_3$ | 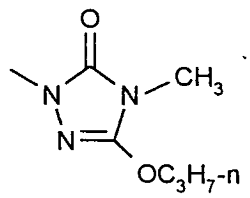 | 182 |
| 924 | - | O | $CH_3$ | (6-)$OCH_3$ | 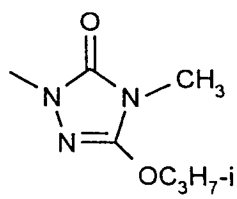 | 142 |
| 925 | - | O | $CH_3$ | (6-)$OCH_3$ | 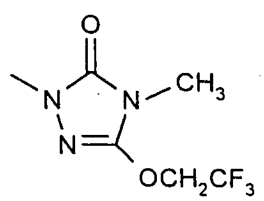 | 178 |
| 926 | - | O | $C_3H_7$-i | (6-)$CH_3$ | 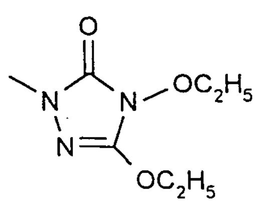 | 151 |

Tabelle 1 - Fortsetzung

| Bsp.-Nr. | A | Q | $R^1$ | (Position-) $R^2$ | $R^3$ | Schmelz-punkt |
|---|---|---|---|---|---|---|
| 927 | - | O | $CH_3$ | (6-)$OCH_3$ | 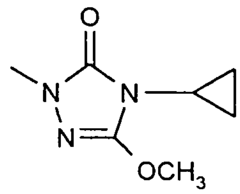 | 178 |
| 928 | - | O | $CH_3$ | (6-)$OCH_3$ | 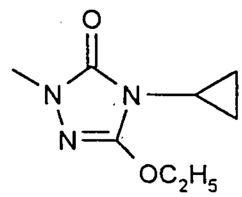 | 130 |
| 929 | - | O | $CH_3$ | (6-)$OCH_3$ | 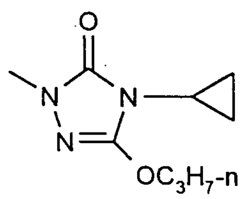 | 124 |
| 930 | - | O | $CH_3$ | (6-)$OCH_3$ | 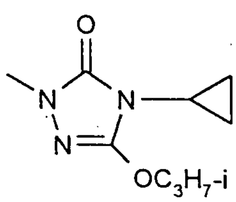 | 153 |
| 931 | - | O | $CH_3$ | (6-)$OCH_3$ | 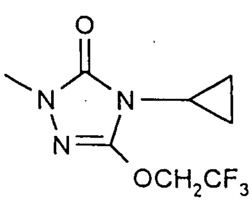 | 157 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 932 | - | O | $CH_3$ | (6-)$OCH_3$ | 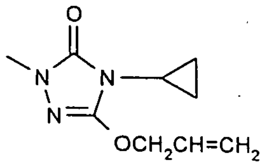 | 114 |
| 933 | - | O | $CH_3$ | (6-)$OCH_3$ | 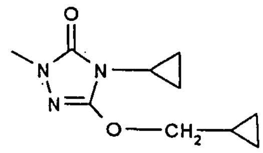 | 130 |
| 934 | - | O | $C_3H_7$-i | (6-)$CH_3$ | 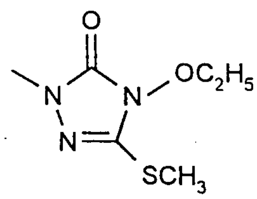 | 151 |
| 935 | - | O | $C_3H_7$-i | (6-)$CH_3$ | 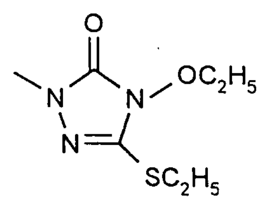 | 153 |
| 936 | - | O | $CH_3$ | (6-)$OCH_3$ | 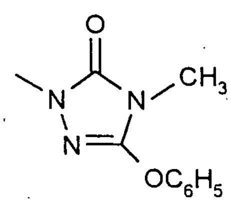 | 167 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 937 | - | O | $CF_2Cl$ | (6-)$CH_3$ | 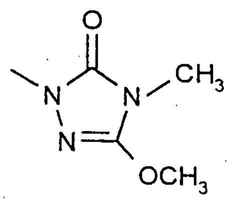 | 180 (Na-Salz) |
| 938 | - | O | $CH_3$ | (6-)$OCH_3$ | 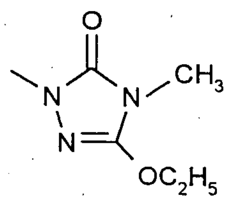 | 157 (Na-Salz) |
| 939 | NH | O | $CH_2CH_2F$ | (6-)$CH_3$ | 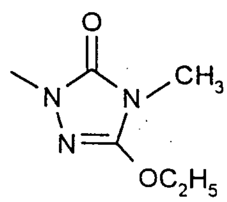 | 163 |
| 940 | NH | O | $CH_2CHF_2$ | (6-)$CH_3$ | 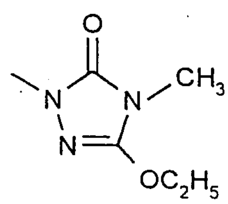 | 160 |
| 941 | NH | O | $CF_2CHFCl$ | (6-)$CH_3$ | 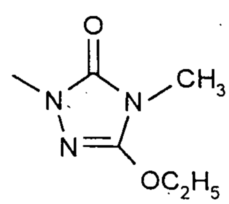 | 88 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 942 | - | O | $CH_2CH_2F$ | (6-)$CH_3$ | 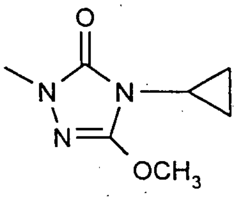 | 178 |
| 943 | - | O | $CH_2CH_2F$ | (6-)$CH_3$ | 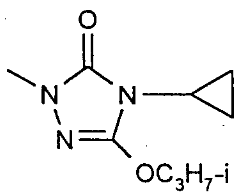 | 135 |
| 944 | - | O | $CH_2CH_2F$ | (6-)$CH_3$ | 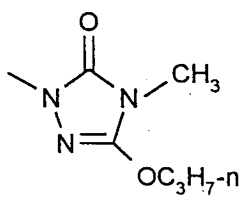 | 127 |
| 945 | - | O | $CH_2CH_2F$ | (6-)$CH_3$ | 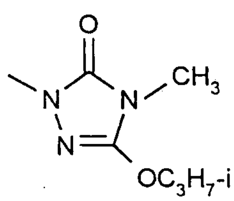 | 139 |
| 946 | - | O | $CH_2CH_2F$ | (6-)$CH_3$ | 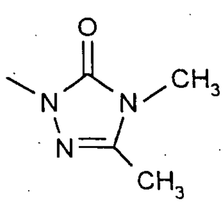 | 280 (Na-Salz) |

Tabelle 1 - Fortsetzung

| Bsp.-Nr. | A | Q | $R^1$ | (Position-) $R^2$ | $R^3$ | Schmelz-punkt |
|---|---|---|---|---|---|---|
| 947 | - | O | $CH_2CH_2F$ | (6-)$CH_3$ | 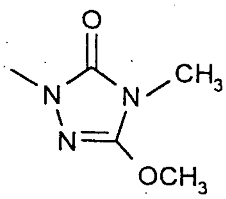 | 171 |
| 948 | - | O | $CH_2CH_2F$ | (6-)$CH_3$ | 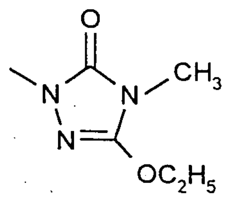 | 144 |
| 949 | - | O | $CH_2CHF_2$ | (6-)$CH_3$ | 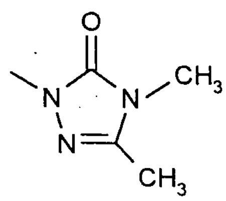 | 273 (Na-Salz) |
| 950 | - | O | $CH_2CHF_2$ | (6-)$CH_3$ | 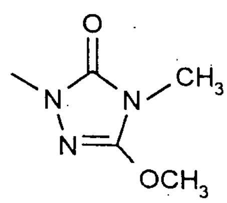 | 181 |
| 951 | - | O | $CH_2CHF_2$ | (6-)$CH_3$ | 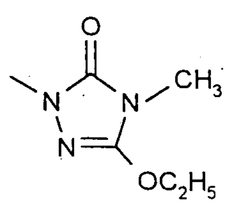 | 142 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 952 | - | O | $CH_2CHF_2$ | (6-)$CH_3$ | 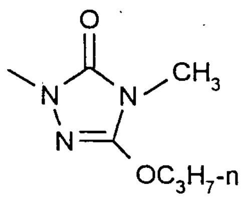 | 114 |
| 953 | - | O | $CH_2CHF_2$ | (6-)$CH_3$ | 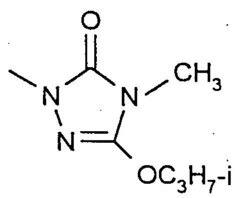 | 108 |
| 954 | - | O | $CH_2CHF_2$ | (6-)$CH_3$ | 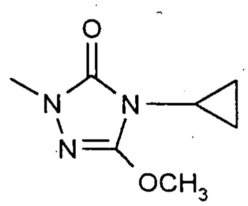 | 185 |
| 955 | - | O | $CH_2CHF_2$ | (6-)$CH_3$ | 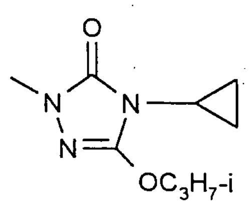 | 150 |
| 956 | - | O | $CF_3$ | (6-)$C_2H_5$ | 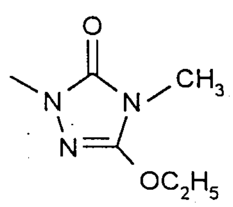 | 143 (Na-Salz) |

Tabelle 1 - Fortsetzung

| Bsp.-Nr. | A | Q | $R^1$ | (Position-) $R^2$ | $R^3$ | Schmelz-punkt |
|---|---|---|---|---|---|---|
| 957 | - | O | $CF_3$ | (6-)$CH_3$ | 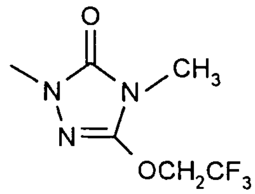 | 155 |
| 958 | - | O | $CF_3$ | (6-)$CH_3$ | 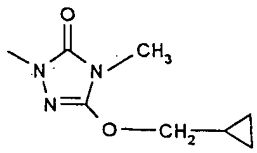 | 112 |
| 959 | - | O | $CF_3$ | (6-)$CH_3$ | 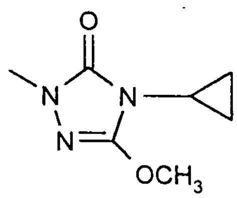 | 166 |
| 960 | - | O | $CF_3$ | (6-)$CH_3$ | 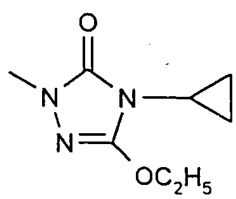 | 137 |
| 961 | - | O | $CF_3$ | (6-)$CH_3$ | 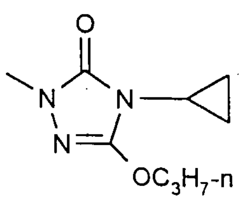 | 132 |

Tabelle 1 - Fortsetzung

| Bsp.-Nr. | A | Q | $R^1$ | (Position-) $R^2$ | $R^3$ | Schmelzpunkt |
|---|---|---|---|---|---|---|
| 962 | - | O | $CF_3$ | (6-)$CH_3$ | 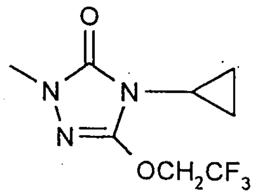 | 172 |
| 963 | - | O | $CF_3$ | (6-)$CH_3$ | 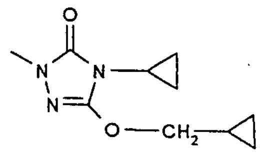 | 139 |
| 964 | - | O | $CF_3$ | (6-)$CH_3$ | 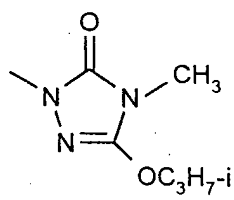 | 130 |
| 965 | - | O | $C_2H_5$ | (3-)$CH_3$ | 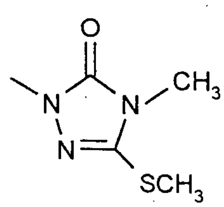 | 184 |

Die in Tabelle 1 als Beispiel 9 aufgeführte Verbindung kann beispielsweise wie folgt hergestellt werden:

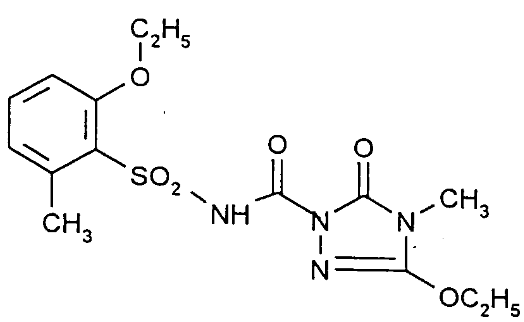

(9)

(Verfahren (b))

1,4 g (0,01 Mol) 5-Ethoxy-4-methyl-2,4-dihydro-3H-1,2,4-triazol-3-on und 2,4 g (0,01 Mol) 2-Ethoxy-6-methyl-phenylsulfonylisocyanat werden in 50 ml Acetonitril 15 Stunden bei 20°C gerührt. Man destilliert das Lösungsmittel ab, verrührt den Rückstand mit Diethylether und saugt den Niederschlag ab.

Man erhält 3,3 g (85% der Theorie) 5-Ethoxy-4-methyl-2-(2-ethoxy-6-methyl-phenylsulfonylaminocarbonyl)-2,4-dihydro-3H-1,2,4-triazol-3-on vom Schmelzpunkt 160°C.

Ausgangsstoffe der Formel (II) bzw. (IIa):

Beispiel (II-1)

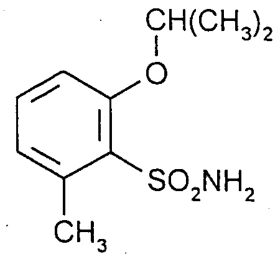

64,6 g (0,26 Mol) 2-Isopropoxy-6-methyl-benzolsulfochlorid werden in 350 ml 25%iger wässriger Ammoniaklösung 12 Stunden bei 20°C gerührt. Das kristallin angefallene Produkt wird anschließend durch Absaugen isoliert.

Man erhält 54 g (90% der Theorie) 2-Isopropoxy-6-methyl-benzolsulfonamid vom Schmelzpunkt 78°C.

Beispiel (II-2)

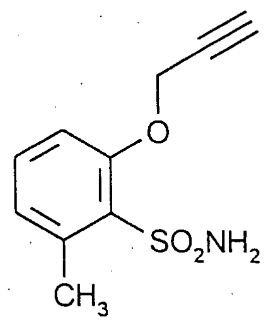

Eine Mischung aus 1,9 g (10 mMol) 2-Hydroxy-6-methyl-benzolsulfonamid, 1,2 g (10 mMol) Propargylbromid (in Form einer 80%igen Lösung in Toluol) und 1,4 g (10 mMol) Kaliumcarbonat wird 2 Stunden unter Rückfluß erhitzt. Dann wird filtriert, das Filtrat im Wasserstrahlvakuum eingeengt, der Rückstand mit Petrolether digeriert und das hierbei kristallin angefallene Produkt durch Absaugen isoliert.

Man erhält 2,1 g (93% der Theorie) 6-Methyl-2-propargyloxy-benzolsulfonamid vom Schmelzpunkt 129°C.

Beispiel (II-3)

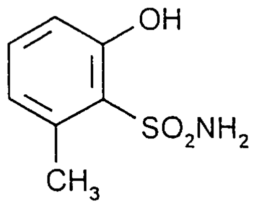

188 ml einer 1-molaren Lösung von Bor(III)-bromid in Methylenchlorid werden bei 20°C unter Rühren zu einer Lösung von 32,3 g (0,15 Mol) 2-Ethoxy-6-methylbenzolsulfonamid in 300 ml Methylenchlorid tropfenweise gegeben und die Reaktionsmischung wird 30 Minuten bei 20°C gerührt. Dann werden bei 0°C bis 5°C (Eiskühlung) 300 ml Methanol zugetropft. Nach Erwärmen auf 20°C wird im Wasserstrahlvakuum eingeengt und der Rückstand mit Essigsäureethylester verrührt. Die erhaltene Lösung wird mit Wasser gewaschen, mit Natriumsulfat getrocknet und filtriert. Das Filtrat wird im Wasserstrahlvakuum eingeengt, der Rückstand durch Verrühren mit Petrolether zur Kristallistion gebracht und das kristalline Produkt durch Absaugen isoliert.

Man erhält 20,3 g (72% der Theorie) 2-Hydroxy-6-methyl-benzolsulfonamid vom Schmelzpunkt 126°C.

Analog zu den Beispielen (II-1) bis (II-3) sowie entsprechend der allgemeinen Beschreibung des erfindungsgemäßen Herstellungsverfahrens können beispielsweise auch die in der nachstehenden Tabelle 2 aufgeführten Verbindungen der Formel (II) bzw. (IIa) hergestellt werden.

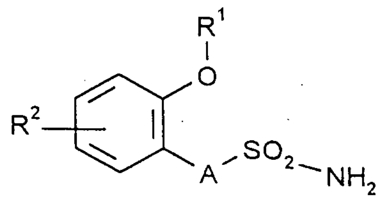

(II)

Tabelle 2:

| Beispiele für die Verbindungen der Formel (II) | | | | |
|---|---|---|---|---|
| Bsp.-Nr. | A | $R^1$ | (Position-) $R^2$ | Schmelzpunkt (°C) |
| II-4 | - | $C_2H_5$ | (6-) $CH_3$ | 104 |
| II-5 | - | n-$C_3H_7$ | (6-) $CH_3$ | 63 |
| II-6 | - | $-CH_2CH_2Cl$ | (6-) $CH_3$ | 102 |
| II-7 | - | $CH_3$ | (6-)$CH_3$ | 132 |
| II-8 | - | $-CH_2C_6H_5$ | (6-)$CH_3$ | 131 |
| II-9 | - | $-CH_2COOCH_3$ | (6-)$CH_3$ | 90 |
| II-10 | - | $CH_3$ | (6-)$C_3H_7$-n | 108 |
| II-11 | - | $C_2H_5$ | (6-)$C_3H_7$-n | 80 |
| II-12 | - | $C_2H_5$ | (5-)$CH_3$ | 131 |
| II-13 | - | $CH_3$ | (6-)Cl | 166 |
| II-14 | - | $C_2H_5$ | (6-)Cl | 121 |
| II-15 | - | H | (6-)Cl | 118 |
| II-16 | - | i-$C_3H_7$ | (6-)Cl | 85 |
| II-17 | - | -CH2CH=CH2 | (6-)Cl | 106 |
| II-18 | - | $-CH_2C\equiv CH$ | (6-)Cl | 181 |
| II-19 | - | $CF_3$ | (5-)Cl | |

Tabelle 2: (fortgesetzt)

| Beispiele für die Verbindungen der Formel (II) | | | | |
|---|---|---|---|---|
| Bsp.-Nr. | A | $R^1$ | (Position-) $R^2$ | Schmelzpunkt (°C) |
| II-20 | - | $CHF_2$ | (5-)$CH_3$ | 127 |
| II-21 | - | $CHF_2$ | (6-)$CH_3$ | 89 |
| II-22 | - | $CH_3$ | (5-)$C(CH_3)_3$ | 160 |
| II-23 | - | $CH_3$ | (5-)Cl | |
| II-24 | - | $CHF_2$ | (4-)$CH_3$ | 153 |
| II-25 | - | $-CF_2CHFCl$ | (6-)$CH_3$ | 85 |
| II-26 | - | $C_2H_5$ | (6-)$CH_2Cl$ | |

Ausgangsstoffe der Formel (IV):

Beispiel (IV-1)

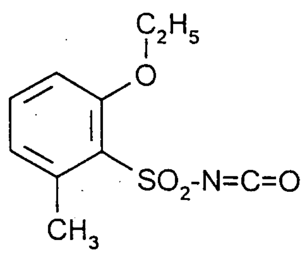

21,5 g (0,1 Mol) 2-Ethoxy-6-methyl-benzolsulfonsäureamid und 10 g (0,1 Mol) n-Butylisocyanat werden in 100 ml Chlorbenzol zum Sieden erhitzt. Bei Rückflußtemperatur wird 4 Stunden Phosgen eingeleitet. Die klare Lösung wird unter vermindertem Druck eingeengt und der Rückstand feindestilliert. Bei einem Druck von 0,8 mbar und einer Kopftemperatur von 135 - 140°C geht 2-Ethoxy-6-methyl-phenylsulfonylisocyanat über, das in der Vorlage erstarrt.

Man erhält 7,9 g 2-Ethoxy-6-methyl-phenylsulfonylisocyanat als farbloses Produkt vom Schmelzpunkt 40°C.

Ausgangsstoffe der Formel (VI) bzw. (VIa):

Beispiel (VI-1)

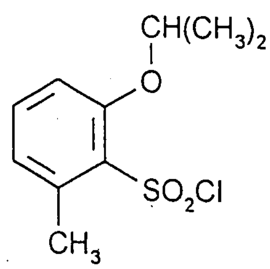

47,8 g (0,29 Mol) 2-Isopropoxy-6-methyl-anilin werden in einer Mischung aus 87 ml 1N-Salzsäure und 145 ml konz. Salzsäure gelöst und die Lösung wird auf -5°C abgekühlt. Bei -5°C bis 0°C wird dann unter Rühren eine Lösung von 22 g (0,32 Mol) Natriumnitrit in 87 ml Wasser zugetropft und die Mischung wird noch eine Stunde bei ca. 0°C gerührt. Nach Beseitigung des Nitrit-Überschusses mit Amidosulfonsäure wird die erhaltene Diazoniumsalz-Lösung bei -5°C bis 0°C zu einer gesättigten Lösung von Schwefeldioxid in 175 ml 1,2-Dichlor-ethan tropfenweise gegeben. Nach ca. 30 Minuten gibt man 1,7 g Kupfer(I)-chlorid und 1,7 g Dodecyl-trimethylammoniumbromid dazu, lässt das Reaktionsgemisch innerhalb von ca. 60 Minuten auf Raumtemperatur kommen, erwärmt es innerhalb einer weiteren Stunde auf ca. 40°C und rührt es ca. 12 Stunden bei dieser Temperatur. Bei ca. 20°C werden dann 14,2 g einer 35%igen Hydrogenperoxid-Lösung dazu gegeben und die Mischung wird ca. 30 Minuten gerührt. Anschließend wird mit 300 ml Methylenchlorid verrührt, die organische Phase abgetrennt, mit Wasser gewaschen, mit Natriumsulfat getrocknet und filtriert. Vom Filtrat wird das Lösungsmittel im Wasserstrahlvakuum sorgfältig abdestilliert.

Man erhält 65,9 g (90% der Theorie) 2-Isopropoxy-6-methyl-benzolsulfochlorid als braunstichigen öligen Rückstand.

$^1H$-NMR ($CDCl_3$, TMS, $\delta$ ppm): 1,47 (d, J=6,1 Hz, $2xCH_3$); 2,68 (s, $CH_3$); 4,79 (sept., J=6,1 Hz, 1H); 6,83 (d, J=7,5 Hz, 1H); 6,95 (d, J=8,4 Hz, 1H); 7,45 (pseudo t, J=8,3 Hz, 1H).

Analog Beispiel (VI-1) können beispielsweise auch die in der nachstehenden Tabelle 3 aufgeführten Verbindungen der Formel (VI) bzw. (VIa) hergestellt werden.

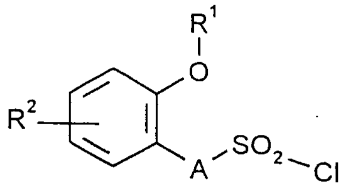

(VI)

Tabelle 3:

| Beispiele für die Verbindungen der Formel (VI) | | | | |
|---|---|---|---|---|
| Bsp.-Nr. | A | $R^1$ | (Position-) R2 | Physikal. Daten |
| VI-2 | - | $CH_3$ | (6-) $CH_3$ | Fp.: 52°C |
| VI-3 | - | $C_2H_5$ | (6-) $CH_3$ | $^1H$-NMR ($CDCl_3$, TMS, $\delta$, ppm): 1,55 (t, J=6,97 Hz, $CH_3$), 2,69 (s, $CH_3$), 4,24 (q, J=6,97 Hz, $CH_2$), 6,87 (d, J=7,68 Hz, 1H), 6,95 (d, J=8,34 Hz, 1H), 7,46 (pseudo t, J=8,1 Hz, 1H) |
| VI-4 | - | n-$C_3H_7$ | (6-) $CH_3$ | $^1H$-NMR ($CDCl_3$, TMS, $\delta$, ppm): 1,33 (t, J=7,38 Hz, $CH_3$), 1,95 (m, $CH_2$), 2,69 (s, $CH_3$), 4,12 (t, J=6,3 Hz, $CH_2$), 6,86 (d, J=7,69 Hz, 1H), 6,94 (d, J=8,37 Hz, 1H), 7,46 (pseudo t, J=7,8 Hz, 1H) |
| VI-5 | - | $-CH_2CH_2Cl$ | (6-) $CH_3$ | $^1H$-NMR ($CDCl_3$, TMS, $\delta$, ppm): 2,71 (s, $CH_3$), 3,94 (t, J=6,1 Hz, $CH_2$), 4,41 (t, J=6,1 Hz, $CH_2$), 6,96 (t, J=7,1 Hz, 2H), 7,5 (t, J=7,8 Hz, 1H) |
| VI-6 | - | $-CH_2CH_2OC_2H_5$ | (6-) $CH_3$ | $^1H$-NMR ($CDCl_3$, TMS, $\delta$, ppm): 1,23 (t, J=7 Hz, $CH_3$), 2,69 (s, $CH_3$), 3,65 (q, J=7 Hz, $CH_2$), 3,91 (t, J=5,16 Hz, $CH_2$), 4,30 (t, J=5,16 Hz, $CH_2$), 6,89 (d, J=7,7 Hz, 1H), 7,0 (d, J=8,3 Hz, 1H), 7,47 (pseudo t, J=8,1 Hz, 1H) |
| VI-7 | - | $C_2H_5$ | (5-) $CH_3$ | $^1H$-NMR ($CDCl_3$, TMS, $\delta$, ppm): 1,53 (t, J=7Hz, $CH_3$), 2,36 (s, $CH_3$), 4,25 (q, J=7 Hz, $CH_2$), 7,0 (d, J=8,53 Hz, 1H), 7,45 (d, J1=8,53 Hz, J2=2,15 Hz, 1H), 7,75 (d, J=2,15 Hz, 1H) |
| VI-8 | - | n-$C_3H_7$ | (5-) $CH_3$ | $^1H$-NMR ($CDCl_3$, TMS, $\delta$, ppm): 1,08 (t, J=7,38 Hz, $CH_3$), 1,85 (m, $CH_2$), 2,23 (s, $CH_3$), 3,99 (t, J=6,5 Hz), 6,74 (d, J=8,2 Hz, 1H), 6,92 (m, 1H), 7,34 (d, J=1,65 Hz, 1H) |
| VI-9 | - | i-$C_3H_7$ | (5-) $CH_3$ | $^1H$-NMR ($CDCl_3$, TMS, $\delta$, ppm): 1,45 (d, J=6,06, $2xCH_3$), 2,35 (s, $CH_3$), 4,77 (sept., J=6,06 Hz, 1H), 6,99 (d, J=8,57 Hz, 1H), 7,43 (dd, J1=8,56 Hz, 1H, J2=2,1 Hz, 1H), 7,74 (d, J=2,1 Hz, 1H) |
| VI-10 | - | $C_2H_5$ | (6-)$CH_2Cl$ | (Öl) |
| VI-11 | - | $-CF_2CHFCl$ | (6-)$CH_3$ | $^1H$-NMR ($CDCl_3$, TMS, $\delta$, ppm): 2,78 (s, $CH_3$), 6,46 (td, CHFCl), 7,2-7,6 (Ar-H) |

Tabelle 3: (fortgesetzt)

| Beispiele für die Verbindungen der Formel (VI) | | | | |
|---|---|---|---|---|
| Bsp.-Nr. | A | $R^1$ | (Position-) R2 | Physikal. Daten |
| VI-12 | - | $CHF_2$ | (6-)$CH_3$ | $^1$H-NMR ($CDCl_3$, TMS, $\delta$, ppm): 2,76 (s, $CH_3$), 6,61 (t, $CHF_2$), 7,27-7,59 (Ar-H) |
| VI-13 | - | $CH_3$ | (5-)$C(CH_3)_3$ | Fp.: 62°C |
| VI-14 | - | $CHF_2$ | (4-)$CH_3$ | $^1$H-NMR ($CDCl_3$, TMS, $\delta$, ppm): 2,50 (s, $CH_3$), 6,68 (t, $CHF_2$), 7,05-7,92 (Ar-H) |
| VI-15 | - | $CHF_2$ | (5-)$CH_3$ | $^1$H-NMR ($CDCl_3$, TMS, $\delta$, ppm): 2,45 (s, $CH_3$), 6,64 (t, $CHF_2$), 7,35-7,86 (AR-H) |
| VI-16 | - | $-CH_2CH_2Cl$ | (6-)$CH_3$ | |
| VI-17 | - | $-CH_2CH=CH_2$ | (6-)$CH_3$ | |
| VI-18 | - | $-CH_2C\equiv CH$ | (6-)$CH_3$ | |
| VI-19 | - | $-CH_2C_6H_5$ | (6-)$CH_3$ | |

Ausgangsstoffe der Formel (X):

Beispiel (X-1)

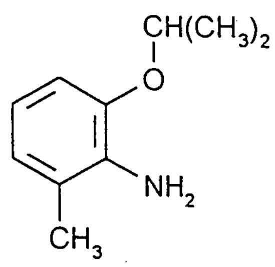

Stufe 1: Herstellung von 2-Isopropoxy-6-methyl-nitrobenzol

Eine Mischung aus 153 g (1,0 Mol) 3-Methyl-2-nitro-phenol, 172,5 g (1,25 Mol) Kaliumcarbonat, 170 g (1,0 Mol) 2-Iod-propan und 400 ml Aceton wird 12 Stunden unter Rückfluß erhitzt. Anschließend wird im Wasserstrahlvakuum eingeengt, der Rückstand mit 400 ml Methylenchlorid verrührt, filtriert und mit Methylenchlorid nachgewaschen. Vom Filtrat wird das Lösungsmittel im Wasserstrahlvakuum sorgfältig abdestilliert.
Man erhält 183,4 g 2-Isopropoxy-6-methyl-nitrobenzol als gelben öligen Rückstand.
$^1$H-NMR ($CDCl_3$, TMS, $\delta$, ppm): 1,33 (d, J=6,1 Hz, 2x$CH_3$), 2,28 (s, $CH_3$), 4,6 (sept., J=6,1 Hz, 1H), 6,8 (d, J=7,7 Hz, 1H), 6,87 (d, J=8,4 Hz, 1H), 7,26 (pseudo t, J=8,1 Hz, 1H).

Stufe 2: Herstellung von 2-Isopropoxy-6-methyl-anilin

183,3 g (0,94 Mol) 2-Isopropoxy-6-methyl-nitrobenzol werden in 1 Liter Essigsäureethylester in Gegenwart von 9,5 g Raney-Nickel unter einem WasserstoffDruck von 40 bis 60 bar 5 Stunden lang hydriert. Dann wird filtriert und vom Filtrat das Lösungsmittel im Wasserstrahlvakuum sorgfältig abdestilliert.
Man erhält 139,4 g (90% der Theorie) 2-Isopropoxy-6-methyl-anilin als orangefarbenen öligen Rückstand.
$^1$H-NMR ($CDCl_3$, TMS, $\delta$, ppm): 1,36 (d, J=6,1 Hz, 2x$CH_3$), 2,16 (s, $CH_3$), 3,72 (s, $NH_2$), 4,51 (sept., J=6,1 Hz, 1H), 6,65-6,70 (m, 3H).

Anwendungsbeispiele:

In den Anwendungsbeispielen werden die nachstehend genannten Verbindungen als Vergleichssubstanzen verwendet:

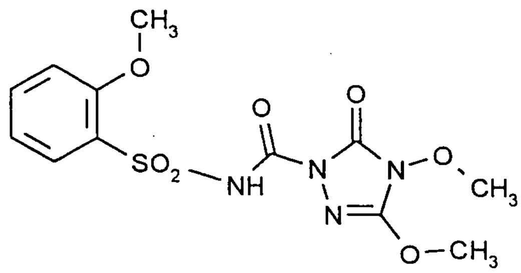

(A)

4,5-Dimethoxy-2-(2-methoxy-phenylsulfonylaminocarbonyl)-2,4-dihydro-3H-1,2,4-triazol-3-on (bekannt aus EP 534266);

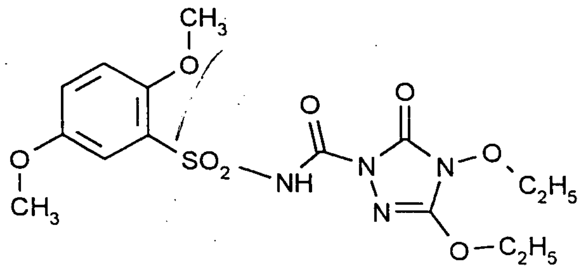

(B)

4,5-Diethoxy-2-(2,5-dimethoxy-phenylsulfonylaminocarbonyl)-2,4-dihydro-3H-1,2,4-triazol-3-on (bekannt aus EP 534266).

Beispiel A

Pre-emergence-Test

| | |
|---|---|
| Lösungsmittel: | 5 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät, Nach ca. 24 Stunden wird der Boden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit.

Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)

100 % = totale Vernichtung

In diesem Test zeigen beispielsweise die Verbindungen gemäß den Herstellungsbeispielen 1, 7-21, 23-41, 46-49, 51, 54, 55, 57, 60, 62, 65, 68, 72-74, 76, 78, 79, 88, 89, 199, 207, 209, 222 und 901 sehr starke Wirkung gegen Unkräuter (vgl. Tabelle A).

Tabelle A:

| Pre-emergence-Test/Gewächshaus | | | | | | | | |
|---|---|---|---|---|---|---|---|---|
| Wirkstoff gemäß Herstellungs-Bsp.-Nr. | Aufwandmenge (g ai./ha) | Agropyron | Lolium | Abutilon | Datura | Ipomoea | Sinapis | Viola |
| (A) | 500 | 0 | 0 | 0 | 0 | 0 | 40 | 0 |
| (B) | 250 | 0 | 60 | 0 | 30 | 0 | 60 | 40 |
| 1 | 250 | 95 | 95 | 95 | 80 | 95 | 95 | 95 |
| 7 | 250 | 95 | 95 | 100 | 80 | 95 | 95 | 90 |
| 8 | 250 | 95 | 95 | 90 | 90 | 95 | 95 | 90 |
| 9 | 250 | 95 | 95 | 95 | 95 | 95 | 95 | 95 |
| 10 | 250 | 95 | 95 | 95 | 70 | 95 | 95 | 95 |
| 11 | 250 | 90 | 90 | 90 | 70 | 80 | 95 | 90 |
| 12 | 250 | 95 | 95 | 95 | 95 | 95 | 95 | 95 |
| 13 | 250 | 95 | 95 | 95 | 80 | 80 | 95 | 90 |
| 14 | 250 | 95 | 95 | 95 | - | 90 | 95 | 95 |
| 15 | 250 | 95 | 95 | 95 | 95 | 95 | 95 | 95 |
| 16 | 125 | 95 | 95 | 95 | 80 | 95 | 95 | 95 |
| 17 | 250 | 95 | 95 | 95 | 80 | 90 | 95 | 95 |
| 18 | 125 | 95 | 95 | 70 | 70 | 90 | 90 | 70 |
| 19 | 125 | 95 | 95 | 95 | 90 | 95 | 95 | 90 |
| 20 | 125 | 95 | 95 | 95 | 70 | 90 | 95 | 95 |
| 21 | 125 | 95 | 95 | 95 | - | 80 | 95 | 95 |
| 23 | 125 | 95 | 95 | 95 | 95 | 80 | 95 | 95 |
| 24 | 125 | 95 | 95 | 100 | 95 | 70 | 95 | 95 |
| 25 | 60 | 95 | 95 | 95 | 80 | 90 | 95 | 80 |
| 26 | 125 | 80 | 90 | 80 | 60 | 90 | 95 | 80 |
| 27 | 60 | 80 | 90 | 95 | - | 80 | 90 | 70 |
| 28 | 60 | 95 | 95 | 90 | 80 | 90 | 90 | 80 |
| 29 | 125 | 95 | 95 | 100 | - | 70 | 95 | 80 |
| 30 | 60 | 95 | 95 | 95 | 90 | - | 95 | 100 |
| 31 | 60 | 95 | 95 | 95 | 95 | 95 | 95 | 95 |
| 32 | 125 | 95 | 95 | 95 | 80 | 90 | 90 | 90 |
| 33 | 60 | 90 | 95 | 95 | 80 | - | 95 | 95 |
| 34 | 60 | 95 | 95 | 100 | - | - | 95 | 90 |
| 35 | 60 | 90 | 95 | 100 | 80 | 70 | 90 | 90 |
| 36 | 125 | 95 | 95 | 80 | 80 | 80 | 80 | 80 |
| 38 | 125 | 95 | 90 | 80 | 80 | 80 | 90 | 90 |
| 39 | 125 | 95 | 95 | 95 | 70 | 90 | 90 | 80 |
| 40 | 125 | 95 | 95 | 95 | 90 | 95 | 90 | 95 |
| 41 | 125 | 90 | 95 | 95 | - | 90 | 95 | 80 |
| 46 | 60 | 90 | 95 | 90 | 80 | - | 95 | 95 |
| 47 | 60 | 95 | 95 | 95 | 95 | 95 | 95 | 95 |
| 48 | 60 | 95 | 95 | 95 | 80 | 95 | 95 | 95 |
| 49 | 60 | 90 | 95 | 95 | - | - | 95 | 95 |
| 51 | 60 | 80 | 90 | 90 | - | - | 95 | 95 |
| 54 | 60 | 80 | 90 | 95 | - | - | 100 | 95 |
| 55 | 60 | 95 | 95 | 95 | 90 | 80 | 95 | 95 |
| 57 | 60 | 95 | 95 | 95 | 70 | 95 | 95 | 95 |
| 60 | 60 | 95 | 100 | 100 | 90 | 95 | 95 | 95 |
| 62 | 125 | 95 | 95 | 95 | 80 | 95 | 95 | 100 |

Tabelle A: (fortgesetzt)

| Pre-emergence-Test/Gewächshaus | | | | | | | | |
|---|---|---|---|---|---|---|---|---|
| Wirkstoff gemäß Herstellungs-Bsp.-Nr. | Aufwandmenge (g ai./ha) | Agropyron | Lolium | Abutilon | Datura | Ipomoea | Sinapis | Viola |
| 65 | 125 | 95 | 100 | 95 | 95 | 95 | 95 | 95 |
| 68 | 125 | 95 | 95 | 90 | - | 90 | 95 | - |
| 72 | 125 | 95 | 95 | 95 | 95 | 95 | 95 | 95 |
| 73 | 125 | 95 | 95 | 95 | 90 | 95 | 95 | 95 |
| 74 | 125 | 95 | 95 | 95 | 100 | 95 | 95 | 95 |
| 76 | 60 | 80 | 80 | 95 | 80 | 95 | 95 | 95 |
| 78 | 125 | 80 | 95 | 90 | 95 | 95 | 95 | 100 |
| 79 | 125 | 95 | - | 95 | 80 | 95 | 95 | 100 |
| 88 | 125 | 95 | 90 | 95 | 70 | 95 | 95 | 95 |
| 89 | 125 | 95 | 95 | 95 | 70 | 95 | 95 | 95 |
| 222 | 60 | 95 | 95 | 100 | 80 | - | 95 | 95 |
| 199 | 60 | 95 | 100 | 100 | 95 | 90 | 95 | 95 |
| 207 | 60 | 95 | 100 | - | 95 | 95 | 95 | 95 |
| 209 | 60 | 95 | 95 | - | 95 | 95 | 95 | 100 |
| 901 | 250 | - | 100 | 100 | 100 | 95 | - | 100 |

Beispiel B

Post-emergence-Test

Lösungsmittel: 5 Gewichtsteile Aceton
Emulgator: 1 Gewichtsteil Alkylarylpolyglykolether

Zur Hertellung einer zweckmäßigen Wirkstoffzubereitung vermischt man 1 Gewichtsteil Wirkstoff.mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Die Konzentration der Spritzbrühe wird so gewählt, daß in 2000 1 Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)

100 % = totale Vernichtung

In diesem Test zeigen beispielsweise die Verbindungen gemäß den Herstellungsbeispielen 1, 7-10, 12, 13, 15, 16, 17, 25, 30, 31, 38, 40, 41, 46 und 47 sehr starke Wirkung gegen Unkräuter (vgl. Tabelle B).

Tabelle B:

| Post emergence-Test/Gewächshaus | | | | | | | |
|---|---|---|---|---|---|---|---|
| Wirkstoff gemäß Herstellungs-Bsp.-Nr. | Aufwandmenge (g ai./ha) | Alopecurus | Lolium | Sorghum | Abutilon | Matricaria | Stellaria |
| (A) | 250 | 20 | 30 | 40 | 0 | 0 | 0 |
| 1 | 60 | 90 | 95 | 100 | 100 | 100 | 100 |
| 7 | 125 | 95 | 80 | 100 | 100 | 95 | 100 |
| 8 | 250 | 95 | 95 | 95 | 100 | 100 | 100 |
| 9 | 125 | 100 | 90 | 100 | 100 | 100 | 100 |
| 10 | 60 | 90 | 90 | 100 | 100 | 100 | 100 |
| 12 | 250 | 95 | 95 | 100 | 100 | 100 | 100 |
| 13 | 125 | 100 | 95 | 100 | 95 | 100 | 100 |
| 15 | 125 | 100 | 95 | 100 | 100 | 100 | 100 |
| 16 | 125 | 95 | 95 | 95 | 95 | 100 | 100 |
| 17 | 250 | 95 | 90 | 95 | 100 | 100 | 100 |
| 25 | 60 | - | 95 | 95 | 95 | 100 | 100 |
| 30 | 60 | 90 | 90 | 100 | 100 | 95 | 95 |
| 31 | 15 | 90 | 80 | 100 | 95 | 95 | 95 |
| 38 | 60 | 90 | - | 90 | 95 | 100 | 95 |
| 40 | 60 | 95 | 95 | 100 | 100 | 100 | 100 |
| 41 | 60 | 95 | 95 | 100 | 100 | 95 | 100 |
| 46 | 60 | 95 | 95 | 95 | 95 | 90 | 95 |
| 47 | 15 | 90 | 95 | 90 | 100 | 95 | 100 |

**Patentansprüche**

1. Verbindungen der allgemeinen Formel (XIII)

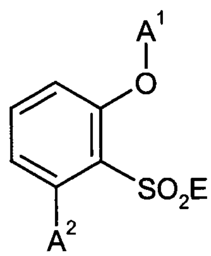

(XIII)

worin

E für $-NH_2$, -N=C=Q oder -Cl steht, wobei

Q für O oder S steht, und ferner

$A^1$ für n- oder i-Propyl, n-, i-, s- oder t-Butyl, Fluorethyl, Chlorethyl, Difluorethyl, Chlortrifluorethyl oder Ethoxyethyl steht, und

$A^2$ für Methyl, Ethyl, n- oder i-Propyl, n-, i- oder s-Butyl steht.

2. Verbindungen gemäß der allgemeinen Formel (XIII) in Anspruch 1, bei denen $A^1$, $A^2$ und E die in der folgenden Tabelle angegebene Bedeutung haben:

| $A^1$ | $A^2$ | E |
|---|---|---|
| $C_2H_5$ | Cl | $NH_2$ |
| $CH_2$-$CH_2$-Cl | $CH_3$ | $NH_2$ |
| $CH_2$-$CH_2$-Cl | $CH_3$ | Cl |
| i-$C_3H_7$ | $CH_3$ | $NH_2$ |
| i-$C_3H_7$ | $CH_3$ | Cl |
| i-$C_3H_7$ | Cl | $NH_2$ |
| n-$C_3H_7$ | $CH_3$ | $NH_2$ |
| n-$C_3H_7$ | $CH_3$ | Cl |
| -$CF_2$-CHFCl | $CH_3$ | $NH_2$ |
| -$CF_2$-CHFCl | $CH_3$ | Cl |
| $CH_3$ | n-$C_3H_7$ | $NH_2$ |
| $C_2H_5$ | n-$C_3H_7$ | $NH_2$ |

Europäisches Patentamt

# EUROPÄISCHER RECHERCHENBERICHT

Nummer der Anmeldung
EP 03 01 1479

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| D,X | EP 0 534 266 A (BAYER AG) 31. März 1993 (1993-03-31) * das ganze Dokument, insbesondere Beispiele 79, 98, sowie Anspruch 4, Verbindungen III, V * | 1,2 | C07C309/87 C07C311/29 C07C311/65 C07C331/32 //C07D249/12, C07D271/06, C07D271/10, C07D263/34, A01N47/38, A01N43/82, A01N43/74 |
| X | DE 42 34 801 A (BAYER AG) 21. April 1994 (1994-04-21) * das ganze Dokument, insbesondere Beispiele 24, 29, 34, 36, 113, 150, 151, 153, 157 und 159, sowie Seite 3, Verbindungen V, VI * | 1,2 | |
| X | EP 0 482 349 A (BASF AKTIENGESELLSCHAFT) 29. April 1992 (1992-04-29) * das ganze Dokument, insbesondere Seite 24, Gruppen A1-48, A1-49 und A1-50; Seite 30, Beispiel 145; Seite 33, Gruppen E5, E31, E48, E49 und E50; sowie Ansprüche 4-7, Verbindungen II, V und VII * | 1,2 | RECHERCHIERTE SACHGEBIETE (Int.Cl.7) |
| D,X | EP 0 425 948 A (BAYER AG) 8. Mai 1991 (1991-05-08) * das ganze Dokument, insbesondere Seite 14, Zeile 40; sowie Anspruch 4, Verbindungen III, V * | 1,2 | C07C |
| P,X | WO 96 11188 A (BAYER AKTIENGESELLSCHAFT) 18. April 1996 (1996-04-18) * das ganze Dokument, insbesondere Beispiele 42, 59, 71; sowie Anspruch 4, Verbindungen III, V und VII * | 1,2 | |
| P,X | WO 95 27703 A (BAYER AKTIENGESELLSCHAFT) 19. Oktober 1995 (1995-10-19) * das ganze Dokument, insbesondere Beispiele 56-99, 217, 218, 220, 221, 223, 224, 226, 228, 230 und 232 * | 1,2 | |
| | -/-- | | |

Der vorliegende Recherchenbericht wurde für alle Patentansprüche erstellt

| Recherchenort | Abschlußdatum der Recherche | Prüfer |
|---|---|---|
| DEN HAAG | 23. Juli 2003 | Allard, M |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

Europäisches Patentamt

# EUROPÄISCHER RECHERCHENBERICHT

Nummer der Anmeldung
EP 03 01 1479

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| P,X | EP 0 708 087 A (ROHM AND HAAS COMPANY) 24. April 1996 (1996-04-24) * Seite 10, Beispiele 45, 52 und 53 * --- | 1,2 | |
| E | WO 97 32861 A (BAYER AKTIENGESELLSCHAFT) 12. September 1997 (1997-09-12) * das ganze Dokument, insvesondere Seite 13, Gruppen 8 und 9, Seite 33, Beispiel V-1, und Anspruch 4, Verbindungen III und V * ----- | 1,2 | |

RECHERCHIERTE SACHGEBIETE (Int.Cl.7)

Der vorliegende Recherchenbericht wurde für alle Patentansprüche erstellt

| Recherchenort | Abschlußdatum der Recherche | Prüfer |
|---|---|---|
| DEN HAAG | 23. Juli 2003 | Allard, M |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT ÜBER DIE EUROPÄISCHE PATENTANMELDUNG NR.** EP 03 01 1479

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

23-07-2003

| Im Recherchenbericht angeführtes Patentdokument | Datum der Veröffentlichung | Mitglied(er) der Patentfamilie | Datum der Veröffentlichung |
|---|---|---|---|
| EP 534266 A | 31-03-1993 | DE 4131842 A1 | 01-04-1993 |
| | | BR 9203729 A | 20-04-1993 |
| | | CA 2078811 A1 | 26-03-1993 |
| | | DE 59208541 D1 | 03-07-1997 |
| | | EP 0534266 A1 | 31-03-1993 |
| | | ES 2102433 T3 | 01-08-1997 |
| | | JP 3164913 B2 | 14-05-2001 |
| | | JP 5213907 A | 24-08-1993 |
| | | MX 9205316 A1 | 01-04-1993 |
| | | US 5488028 A | 30-01-1996 |
| | | US 5554761 A | 10-09-1996 |
| | | US 5631380 A | 20-05-1997 |
| | | US 5300480 A | 05-04-1994 |
| DE 4234801 A | 21-04-1994 | DE 4234801 A1 | 21-04-1994 |
| | | AU 678775 B2 | 12-06-1997 |
| | | AU 5110793 A | 09-05-1994 |
| | | BR 9307260 A | 11-05-1999 |
| | | CA 2146877 A1 | 28-04-1994 |
| | | DE 59306215 D1 | 22-05-1997 |
| | | DK 664797 T3 | 13-10-1997 |
| | | WO 9408979 A1 | 28-04-1994 |
| | | EP 0664797 A1 | 02-08-1995 |
| | | ES 2101346 T3 | 01-07-1997 |
| | | HU 71832 A2 | 28-02-1996 |
| | | JP 8502268 T | 12-03-1996 |
| | | RU 2125559 C1 | 27-01-1999 |
| | | US 5552369 A | 03-09-1996 |
| | | US 5654438 A | 05-08-1997 |
| EP 482349 A | 29-04-1992 | DE 4029753 A1 | 26-03-1992 |
| | | AT 202772 T | 15-07-2001 |
| | | CA 2051537 A1 | 21-03-1992 |
| | | DE 59109212 D1 | 09-08-2001 |
| | | EP 0482349 A2 | 29-04-1992 |
| | | JP 4247072 A | 03-09-1992 |
| | | US 5252540 A | 12-10-1993 |
| EP 425948 A | 08-05-1991 | DE 3936622 A1 | 08-05-1991 |
| | | AU 623284 B2 | 07-05-1992 |
| | | AU 6360290 A | 09-05-1991 |
| | | BR 9005570 A | 17-09-1991 |
| | | CA 2029105 A1 | 04-05-1991 |
| | | DE 59009966 D1 | 25-01-1996 |
| | | DE 59010850 D1 | 29-10-1998 |
| | | DK 425948 T3 | 22-04-1996 |

EPO FORM P0461

Für nähere Einzelheiten zu diesem Anhang : siehe Amtsblatt des Europäischen Patentamts, Nr.12/82

## ANHANG ZUM EUROPÄISCHEN RECHERCHENBERICHT ÜBER DIE EUROPÄISCHE PATENTANMELDUNG NR. EP 03 01 1479

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

23-07-2003

| Im Recherchenbericht angeführtes Patentdokument | Datum der Veröffentlichung | Mitglied(er) der Patentfamilie | Datum der Veröffentlichung |
|---|---|---|---|
| EP 425948 A | | DK 661262 T3 | 14-06-1999 |
| | | EP 0425948 A2 | 08-05-1991 |
| | | EP 0661262 A1 | 05-07-1995 |
| | | ES 2081334 T3 | 01-03-1996 |
| | | ES 2121240 T3 | 16-11-1998 |
| | | JP 2965661 B2 | 18-10-1999 |
| | | JP 3153674 A | 01-07-1991 |
| | | US 5380864 A | 10-01-1995 |
| | | US 5380863 A | 10-01-1995 |
| | | US 5599944 A | 04-02-1997 |
| | | US 5085684 A | 04-02-1992 |
| | | US 5750718 A | 12-05-1998 |
| | | US 5149356 A | 22-09-1992 |
| | | US 5238910 A | 24-08-1993 |
| | | US 5276162 A | 04-01-1994 |
| | | ZA 9008799 A | 28-08-1991 |
| WO 9611188 A | 18-04-1996 | DE 4435547 A1 | 11-04-1996 |
| | | AU 3652695 A | 02-05-1996 |
| | | CN 1168668 A | 24-12-1997 |
| | | WO 9611188 A1 | 18-04-1996 |
| | | EP 0784616 A1 | 23-07-1997 |
| | | JP 10508298 T | 18-08-1998 |
| WO 9527703 A | 19-10-1995 | DE 4411913 A1 | 12-10-1995 |
| | | AU 2136595 A | 30-10-1995 |
| | | BR 9507294 A | 07-10-1997 |
| | | CA 2187215 A1 | 19-10-1995 |
| | | CN 1145068 A | 12-03-1997 |
| | | WO 9527703 A1 | 19-10-1995 |
| | | EP 0754179 A1 | 22-01-1997 |
| | | HU 76496 A2 | 29-09-1997 |
| | | JP 9511510 T | 18-11-1997 |
| | | US 5861358 A | 19-01-1999 |
| EP 708087 A | 24-04-1996 | US 5670691 A | 23-09-1997 |
| | | AT 182580 T | 15-08-1999 |
| | | AU 693254 B2 | 25-06-1998 |
| | | AU 3427895 A | 02-05-1996 |
| | | BR 9504435 A | 20-05-1997 |
| | | CA 2160755 A1 | 19-04-1996 |
| | | CN 1339432 A | 13-03-2002 |
| | | CN 1174189 A ,B | 25-02-1998 |
| | | DE 69511035 D1 | 02-09-1999 |
| | | DE 69511035 T2 | 16-03-2000 |
| | | EP 0708087 A1 | 24-04-1996 |

EPO FORM P0461

Für nähere Einzelheiten zu diesem Anhang : siehe Amtsblatt des Europäischen Patentamts, Nr.12/82

## ANHANG ZUM EUROPÄISCHEN RECHERCHENBERICHT ÜBER DIE EUROPÄISCHE PATENTANMELDUNG NR. EP 03 01 1479

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

23-07-2003

| Im Recherchenbericht angeführtes Patentdokument | Datum der Veröffentlichung | Mitglied(er) der Patentfamilie | Datum der Veröffentlichung |
|---|---|---|---|
| EP 708087 A | | HU 74970 A2 | 28-03-1997 |
| | | IL 115499 A | 22-12-1999 |
| | | JP 8231494 A | 10-09-1996 |
| | | TR 960327 A2 | 21-06-1996 |
| | | ZA 9508385 A | 24-04-1996 |
| | | TW 413672 B | 01-12-2000 |
| WO 9732861 A | 12-09-1997 | DE 19608445 A1 | 11-09-1997 |
| | | AU 712363 B2 | 04-11-1999 |
| | | AU 2091797 A | 22-09-1997 |
| | | BR 9707917 A | 27-07-1999 |
| | | CA 2248068 A1 | 12-09-1997 |
| | | CN 1212686 A | 31-03-1999 |
| | | WO 9732861 A1 | 12-09-1997 |
| | | EP 0885200 A1 | 23-12-1998 |
| | | US 6451737 B1 | 17-09-2002 |

EPO FORM P0461

Für nähere Einzelheiten zu diesem Anhang : siehe Amtsblatt des Europäischen Patentamts, Nr.12/82